# EUROPEAN PATENT SPECIFICATION

(11) **EP 1 635 859 B1**
(45) Date of publication and mention of the grant of the patent: **28.04.2010**
(21) Application number: 04730236.9
(22) Date of filing: 29.04.2004
(51) Int. Cl.: A61K 38/17, C07K 14/47, G01N 33/50, A61P 29/00

(54) **THERAPEUTIC USE OF PAM**
THERAPEUTISCHE VERWENDUNG VON PAM
UTILISATION THERAPEUTIQUE DE PAM

(30) Priority: 30.05.2003 EP 03012388
(43) Date of publication of application: 22.03.2006
(73) Proprietor: Sanofi-Aventis Deutschland GmbH, 65929 Frankfurt am Main (DE)
(72) Inventor: MICHAELIS, Martin, 60325 Frankfurt (DE); GEISSLINGER, Gerd, D-65812 Bad Soden (DE); SCHOLICH, Klaus, 63303 Dreieich (DE); TEGEDER, Irmgard, 60598 Frankfurt (DE)
(86) International application number: PCT/EP2004/004513
(87) International publication number: WO 2004/105785

(56) References cited:
- EHNERT CORINA ET AL: "Protein associated with Myc (PAM) is involved in spinal nociceptive processing." JOURNAL OF NEUROCHEMISTRY. ENGLAND FEB 2004, vol. 88, no. 4, February 2004 (2004-02), pages 948-957, XP001179599 ISSN: 0022-3042
- SCHOLICH K ET AL: "Protein associated with Myc (PAM) is a potent inhibitor of adenylyl cyclases." THE JOURNAL OF BIOLOGICAL CHEMISTRY. UNITED STATES 14 DEC 2001, vol. 276, no. 50, 14 December 2001 (2001-12-14), pages 47583-47589, XP002271814 ISSN: 0021-9258
- WITTPOTH C ET AL: "Adenylyl cyclase regulates signal onset via the inhibitory GTP-binding protein, Gi." THE JOURNAL OF BIOLOGICAL CHEMISTRY. UNITED STATES 25 AUG 2000, vol. 275, no. 34, 25 August 2000 (2000-08-25), pages 25915-25919, XP002271815 ISSN: 0021-9258
- WEI F ET AL: "Reduced inflammatory pain and preserved acute pain in mice lacking calmodulin stimulated adenylyl cyclases" SOCIETY FOR NEUROSCIENCE ABSTRACTS, vol. 27, no. 1, 2001, page 144, XP009026716 31st Annual Meeting of the Society for Neuroscience;San Diego, California, USA; November 10-15, 2001 ISSN: 0190-5295
- ALEY K O ET AL: "NITRIC OXIDE SIGNALING IN PAIN AND NOCICEPTOR SENSITIZATION IN THE RAT" JOURNAL OF NEUROSCIENCE, NEW YORK, NY, US, vol. 18, no. 17, 1 September 1998 (1998-09-01), pages 7008-7014, XP001004456 ISSN: 0270-6474
- GALEOTTI N ET AL: "Hypofunctionality of Gi proteins as aetiopathogenic mechanism for migraine and cluster headache." CEPHALALGIA: AN INTERNATIONAL JOURNAL OF HEADACHE. NORWAY FEB 2001, vol. 21, no. 1, February 2001 (2001-02), pages 38-45, XP002271817 ISSN: 0333-1024
- INGRAM S L ET AL: "Opioid inhibition of Ih via adenylyl cyclase." NEURON. UNITED STATES JUL 1994, vol. 13, no. 1, July 1994 (1994-07), pages 179-186, XP009026710 ISSN: 0896-6273
- YANG HUAITAO ET AL: "Developmental expression of PAM (protein associated with MYC) in the rodent brain." BRAIN RESEARCH. DEVELOPMENTAL BRAIN RESEARCH. NETHERLANDS 30 MAY 2002, vol. 136, no. 1, 30 May 2002 (2002-05-30), pages 35-42, XP002271819 ISSN: 0165-3806
- GUO Q ET AL: "Identification of a large Myc-binding protein that contains RCC1-like repeats." PROCEEDINGS OF THE NATIONAL ACADEMY OF SCIENCES OF THE UNITED STATES OF AMERICA. UNITED STATES 4 AUG 1998, vol. 95, no. 16, 4 August 1998 (1998-08-04), pages 9172-9177, XP002271820 ISSN: 0027-8424
- PATEL TARUN B ET AL: "Functional analyses of type V adenylyl cyclase." METHODS IN ENZYMOLOGY. UNITED STATES 2002, vol. 345, 2002, pages 160-187, XP001179828 ISSN: 0076-6879

### SEQUENCE LISTING

<110> Aventis Pharma Deutschland GmbH
<120> use of S1P
<130> DEAV 2003/0049
<140> 03012389.7
   <141> 2003-05-30
<160> 30
<170> PatentIn version 3.2
<210> 1
   <211> 14807
   <212> DNA
   <213> Homo sapiens
<400> 1
   tcttggagcg ttctcagttt ctcaacagat cttcacttgc taggcagcca gaagccggcg     60
   gcagtggcgg caccgcctcc tcctcacatt cccggggtgg cggggttaga tgagcggccc    120
   cagtcgcggc gccgggggcg ctgttcatgc cggttcccga cggctccgtg gctgctgcgg    180
   ggctggggct ggggctaccc gccgcggact ccccgggtca ctaccagctg ctgctgtcag    240
   gccgggccct ggccgaccgc taccggagga tttataccgc tgcgctcaat gacagggacc    300
   aggggggcgg cagcgctgga cacccagcct ccaggaataa gaaaatttta aataagaaga    360
   aattgaaaag aaaacagaag agcaaatcaa aagtgaagac aagaagcaag tctgaaaact    420
   tagagaatac agtaatcata ccagatatca aactacatag caatccttct gctttcaata    480
   tttactgtaa tgtacgccat tgcgttctgg aatggcagaa aaaggaaata tcattggcag    540
   ccgcatctaa gaactctgtg cagagtggag aatcagatag tgatgaagaa gaggaatcca    600
   aagagccccc tatcaagctt ccaaagatta ttgaggttgg cctttgtgaa gtttttgaat    660
   tgatcaaaga gacacgattt tctcatccat ccctgtgtct caggagtctc caagccctgc    720
   tcaacgtgct gcagggccag cagccagaag tgctccagtc tgagccacct gaggtcctag    780
   agtctctctt ccagcttctt ttggaaatca ccgttcgaag tactgggatg aatgacagca    840
   caggacagtc cttaacagca ctttcctgtg cttgcctctt tagtctggtg gcttcttggg    900
   gagaaacagg aaggacactt caggccatct ctgctatcct caccaacaat ggaagccatg    960
   cttgccaaac tattcaggtg ccaacaattc taaattcgct acagagaagt gtacaagcag   1020
   ttttggtggg aaaaattcaa attcaggact ggtttagtaa tggcattaag aaagcagctt   1080
   taatgcacaa gtggccatta aaagaaatat ctgttgatga agatgaccaa tgtctacttc   1140
   agaatgatgg attttttctt tatctattat gcaaggatgg attatataaa ataggctctg   1200
   gatacagtgg aacagttagg ggccatatat acaattctac atcccgtatt agaaacagaa   1260
   aagaaaaaaa gtcttggtta gggtatgctc agggttattt attatataga gatgtgaata   1320
   accacagcat gacagccata aggataagcc ctgaaacact ggagcaagat ggtactgtga   1380
   tgttaccaga ttgccacact gaaggtcaaa atattttatt cactgatgga gaatatatta   1440
   atcagatagc tgcttcaaga gatgatggct ttgttgtcag aatatttgcc acaagcactg   1500
   aacctgttct acagcaagaa ttgcaactta aactggctag aaaatgctta catgcctgtc   1560
   gtatctcatt attcgatctg gaaaaggact tgcatattat aagtacagga tttgatgagg   1620
   agtcagcaat tcttggtgca ggacgagagt ttgcgctaat gaaaacagca aatggaaaga   1680
   tatattacac tggcaaatac cagagtcttg gaatcaaaca aggtggtcct tcagcaggaa   1740
   aatgggttga gctaccaatt acaaaatctc caaagatagt acacttctca gttggacacg   1800
   atggctctca cgccctttta gttgcagaag atgggagcat attctttaca ggatctgcta   1860
   gtaaaggaga agatggagaa tcaattaaga gcagacggca atccaaacct tataaaccta   1920
   aaaagataat taagatggaa ggaaagattg tggtatatac agcctgcaat aatggaagta   1980
   gttctgttat ttctaaagat ggagaactct acatgtttgg aaaagatgcc atttactctg   2040
   atagttcaag tttggtaact gatttgaagg gccattttgt aactcaggta gctatgggca   2100
   aagctcacac ttgtgtttta atgaagaatg gagaggtgtg gacatttggt gtaaataata   2160
   aaggacagtg tggacgagat actggtgcca tgaaccaagg tgggaaaggg tttggagttg   2220
   aaaatatggc aacagcaatg gatgaagacc tggaagaaga actagatgaa aaagatgaga   2280
   agtctatgat gtgccctcca ggcatgcaca aatggaagct ggagcagtgc atggtttgca   2340
   ctgtctgtgg agactgtaca ggttatggag ccagctgtgt cagtagtgga cggccagaca   2400
   gagtccccgg agggatctgt ggttgtggtt ccggagaatc tggttgtgct gtgtgtggat   2460
   gttgcaaggc ctgtgcaaga gagttagatg gtcaagaggc aagacaaaga ggaattcttg   2520
   atgcagtgaa agaaatgata cctttagatc ttcttttagc tgtcccagtg cccggggtta   2580
   acattgaaga acaccttcag ttacgacaag aagaaaaacg gcaacgtgta atcagaaggc   2640
   acagattaga ggaaggaaga ggcccccttg tatttgctgg tcctattttt atgaaccatc   2700
   gagaacaggc tctagccaga ctcagatccc atccagcaca cgtaaagcat aaacgggaca   2760
   agcacaaaga tggaagtgga gaaagaggcg aaaaggatgc aagcaaaatc acaacatacc   2820
   ctccaggctc tgtgcgattt gactgtgagc tccgggcagt ccaagtcagc tgtggatttc   2880
   accattcagt ggttttaatg gaaaatggag atgtctatac atttggttat gggcagcatg   2940
   ggcagctagg acatggagat gtcaactcca ggggatgtcc cactcttgtt caagcattgc   3000
   caggccctag cacacaagtc actgcaggca gcaaccatac ggcagtactt ttaatggatg   3060
   gacaggtctt cacatttgga agtttttcta aaggacaact gggcagacca attttggatg   3120
   tgccatattg gaatgcaaag ccagctccca tgcctaacat tggatcaaaa tatggaagaa   3180
   aagctacttg gataggtgca agtggggacc aaactttttt acgaattgat gaagcactta   3240
   ttaattctca tgtacttgct acatcagaaa tttttgccag taaacacata ataggcttgg   3300
   tacctgcttc tatatcagaa cctcctccat ttaaatgcct tctgataaat aaagtggatg   3360
   ggagttgtaa aacttttaat gactcagaac aagaggatct gcaaggattt ggtgtgtgtc   3420
   ttgatcctgt atatgatgta atttggaggt ttcgaccaaa tactagagag ctgtggtgtt   3480
   acaatgcggt ggttgctgat gccaggcttc cctctgcagc agacatgcag tccagatgta 3540
   gtatcctaag tcctgaactt gccttaccaa caggatcaag ggccctcact acccgatctc 3600
   atgcagcttt gcacatttta ggttgtcttg ataccttggc agctatgcag gacttaaaaa 3660
   tgggtgttgc aagtacagag gaagagactc aagcagtaat gaaggtttat tctaaagaag 3720
   attatagtgt ggtaaacagg tttgaaagtc atggaggagg ctggggttat tctgcccatt 3780
   cagtagaagc tatacgtttc agtgccgaca ctgatatttt acttggtggt cttggtctgt 3840
   ttggaggtag aggagaatat actgctaaaa ttaagctgtt tgaattgggt cctgatggag 3900
   gagatcatga aactgatggt gaccttcttg cagagactga tgtattggct tatgactgtg 3960
   ctgctagaga aaaatatgca atgatgtttg atgagcctgt tctcctgcaa gctgggtggt 4020
   ggtatgtggc atgggcccga gtgtcaggac ccagcagtga ctgtggatct catggacagg 4080
   catctattac cacagatgat ggggttgttt tccagttcaa gagttcaaag aaatcaaata 4140
   atggtacaga tgttaatgcg ggtcagatac ctcagttatt atacagactt ccaaccagtg 4200
   atggcagtgc ttcaaaaggc aaacagcaaa ccagtgaacc tgtacacatt ttaaagaggt 4260
   cttttgcaag aactgtctca gtggaatgtt ttgagtcatt gttgagtatt cttcactgga 4320
   gctggaccac cttagtctta ggagttgaag aacttagagg attaaaagga ttccagttca 4380
   cagctacact cctagattta gagagactgc gctttgtggg tacctgttgt ctgaggttat 4440
   tgcgtgtcta tacctgtgaa atttacccag tgtcagctac aggaaaagca gttgtagaag 4500
   aaactagcaa attagcagag tgtattggaa aaaccagaac tttgttaaga aaaattttat 4560
   cagaaccact tgatcactgc atggtgaaat tggataatga tcctcaagga tatctcagtc 4620
   aacccttgag tcttctagaa gctgtccttc aggaatgtca taatactttc actgcctgct 4680
   ttcattcttt ctacccaact cctgccttac agtgggcttg cctttgtgat ctgctgaatt 4740
   gtttggatca ggatatccaa gaagcaaact tcaagacatc aagtagccga ctccttgcag 4800
   ctgttatgtc agctctgtgt cacacgtctg ttaagctgac ttccatcttc ccgattgcgt 4860
   atgatggaga agtattacta cgatcaattg ttaaacaagt tagtacagag aacgactcaa 4920
   cactagttca tcgttttccc cttttggtgg cacatatgga aaaactcagc cagagtgaag 4980
   agaatatctc agggatgaca agcttccgtg aagttctgga gaaaatgctg gtcattgttg 5040
   tgctaccagt caggaacagc ctgaggagag aaaatgaact cttctcctcc cacctcgtct 5100
   ctaacacctg tggattactg gccagcattg tcagtgaact gacagcgtca gccctgggat 5160
   ctgaggttga tggacttaat tctcttcact ctgtaaaagc tagtgctaac cgatttacaa 5220
   aaacaagtca gggcagaagt tggaacactg ggaacgggtc ccctgatgca atctgttttt 5280
   cagtagacaa acctggaata gttgtggttg gtttctctgt ctatggagga ggtggaattc 5340
   atgaatatga attagaggtg ttggttgatg atagtgaaca tgcaggagat tcaactcatt 5400
   cccacagatg gacatctctg gaattagtga aaggaacgta cacaacggat gactcaccca 5460
   gtgatatagc tgagatcaga cttgacaaag tggttccttt aaaggaaaat gttaaatatg 5520
   ctgtgcgctt gaggaactat ggaagccgta cagccaatgg agatggagga atgaccacag 5580
   ttcagtgccc tgatggtgtg acattcacat tcagcacgtg cagcttgagc agtaacggca 5640
   caaaccaaac cagaggacag atcccacaga tactctacta taggagtgaa tttgatggag 5700
   atttacaatc ccaacttctg agtaaagcca atgaagaaga taaaaactgt agcagagcat 5760
   tgtctgttgt aagcactgtc gttcgagcct ctaaggacct cctgcacaga gctcttgctg 5820
   tggatgctga tgacattcca gaactgctga gttcttccag tctgttttcc atgctgctcc 5880
   cccttattat agcctacata ggaccagtag ctgctgctat tcccaaggtg gctgtagaag 5940
   tctttggcct tgtccaacaa ttgcttccgt cagttgccat tttgaatcag aagtatgcac 6000
   cgcctgcctt caaccctaat cagtcgacag atagcaccac aggaaaccag cctgaacagg 6060
   gcctctctgc ttgtacaacc tccagtcact atgctgtcat agagagtgag cacccgtata 6120
   aacctgcctg tgtgatgcat tacaaggtga cattcccaga atgtgtgagg tggatgacaa 6180
   tcgaatttga ccctcagtgt ggtactgcac agtcagaaga tgtccttcgt ttgttgattc 6240
   ctgtcagaac tgttcagaat tcaggatatg gaccaaaatt gacatctgtt catgaaaatc 6300
   ttaattcatg gatagaatta aagaaatttt caggatcctc tgggtggcct actatggttt 6360
   tggtgttgcc aggaaatgag gccctttttt cattggagac tgcatcagat tatgtgaaag 6420
   atgacaaagc ttctttctat ggttttatgt gttttgcaat tggatatgaa tttagccctg 6480
   gacctgatga gggagtcatc caattggaaa aagaattagc caatcttggt ggggtttgtg 6540
   cagcagctct gatgaagaag gacctagcac ttcctattgg taatgaatta gaagaagacc 6600
   ttgaaattct tgaggaggct gcattgcagg tgtgcaaaac ccattctgga attcttggaa 6660
   agggtctagc tctttctcat tcaccaacta tattagaagc acttgaggga aatttaccac 6720
   tccaaatcca aagcaatgaa cagtcttttc tggatgattt tattgcctgt gtcccaggat 6780
   caagtggtgg aaggcttgca aggtggcttc agccagattc atatgcggat cctcagaaaa 6840
   catctttgat cctgaataag gatgatattc gttgtggttg gcctaccacc ataactgttc 6900
   aaacaaaaga ccagtatggg gatgtggtac atgttcccaa tatgaaggtg gaagtgaaag 6960
   ctgtccctgt ttctcagaaa aaaatgtctt tacaacaaga tcaagcaaag aaacctcaaa 7020
   ggattcctgg cagtcctgca gtaacagctg catcttctaa tactgacatg acttatggag 7080
   ggctggcatc accaaagcta gatgtttcat atgaaccaat gatagtgaag gaagctcgat 7140
   atattgccat aacaatgatg aaggtttatg aaaattattc atttgaagaa ctacgttttg 7200
   catcaccaac tcctaagaga cccagtgaga atatgctgat ccgtgtcaat aatgatggga 7260
   cttattgtgc aaattggact ccaggggcta ttggactcta cactcttcat gttaccattg 7320
   atggcattga aatcgatgct ggtctggaag taaaagtaaa agacccacca aaagggatga 7380
   taccaccagg aactcagttg gtcaaaccaa agtctgaacc tcagcctaat aaggttcgaa 7440
   aatttgtggc caaggacagt gcggggcttc gcatccgtag ccacccttcc cttcagagtg 7500
   agcagatagg catagtgaaa gtcaatggaa ctatcacttt tattgatgag atccataatg 7560
   atgatggtgt gtggctgagg ctgaatgatg agacaataaa gaagtatgtc cctaacatga 7620
   atggttacac tgaagcctgg tgcctctctt ttaatcaaca tcttggcaag agtcttctgg 7680
   tccctgttga cgaatctaaa actaatactg atgacttttt caaagacata aactcctgct 7740
   gcccacagga agcaacaatg caagaacaag atatgccatt cttgcgagga gggccaggca 7800
   tgtacaaggt agtgaagacg ggaccttcag gtcacaacat cagaagctgc cctaacctta 7860
   gaggtatccc aattggaatg ttagttctgg gaaacaaagt caaagcagtg ggagaggtaa 7920
   ccaattctga agggacatgg gtgcaactgg atcagaacag catggtagag ttctgtgaga 7980
   gtgatgaagg agaggcatgg tccttagcta gagacagagg cggaaaccag tacctccgac 8040
   atgaagatga acaagctctt ctggatcaga attctcaaac tcctcctcca agccctttct 8100
   cagtgcaagc ttttaataaa ggggcaagtt gcagtgccca aggatttgat tatggactcg 8160
   gaaatagcaa aggtgatcga ggaaacatct caacatcttc taaaccagcc tctacatcag 8220
   gaaaatcaga gctgtcctct aaacacagca gatcgcttaa acctgatgga cgtatgagcc 8280
   ggactactgc tgatcagaag aagccaaggg gcacagaaag tttatctgct agtgaatccc 8340
   tcatcttaaa atctgatgct gcaaagttga ggtcagattc ccacagtagg tcattatccc 8400
   ccaaccataa caccttgcag acattgaaat ctgatgggag gatgccttct agctccagag 8460
   ctgaatcccc aggaccaggt tctcggttgt catctcctaa gccaaagact ctcccagcca 8520
   ataggtctag cccatcgggt gctagttctc cacgctcctc ctcaccacat gataaaaatc 8580
   tacctcaaaa aagtactgct cctgttaaga caaagcttga tcctcctcgg gaacgttcta 8640
   aatcagactc ttacacactt gatccagata ccctccgcaa gaagaaaatg cccctcacag 8700
   aacctttgag aggacggtca acgtcaccaa aaccaaaatc agtaccaaag gattctacag 8760
   attcccctgg atctgaaaat agagctccct ctccccatgt ggtacaggaa aacctccaca 8820
   gtgaggtggt cgaagtctgc acctcaagta ctttaaaaac aaatagtcta acagacagca 8880
   cctgcgatga cagcagtgaa tttaagagtg tggatgaagg ttcaaataaa gttcatttta 8940
   gcattggaaa agcaccactg aaagatgaac aggaaatgag agcatctccc aaaataagtc 9000
   gaaaatgtgc taatagacac accaggccca aaaaagaaaa atcgagtttt cttttcaaag 9060
   gagatggatc caagccttta gagccagcca agcaagccat gtctccttct gtggccgaat 9120
   gtgccagagc tgtgtttgct tccttcctct ggcatgaagg catagtacat gatgcaatgg 9180
   cttgttcttc tttcctaaag tttcatcctg aactttccaa agaacatgct cctataagga 9240
   gtagtttaaa tagccaacaa cctacagaag aaaaagaaac caagttaaaa aatagacatt 9300
   cattagaaat atcatctgca ctgaatatgt ttaatattgc accccatgga ccagatatat 9360
   ctaagatggg tagcatcaac aaaaacaagg tattgtctat gcttaaggaa ccacctctgc 9420
   atgaaaaatg tgaggatggg aaaaccgaga ccacttttga aatgtccatg cataacacaa 9480
   tgaagtctaa gtctcctctt cccttaactt tacaacattt agtggctttt tgggaagaca 9540
   tctctttggc tactatcaaa gctgcttccc agaatatgat ttttccaagt cctggttcct 9600
   gtgcagttct taaaaagaaa gagtgtgaga aaggaaggaa taagaagtcc aaaaaggaaa   9660
   aaaagaaaaa agaaaaggca gaagttaggc ccaggggtaa tttgtttgga gagatggccc   9720
   agctggcagt aggaggacca gagaaagata ccatctgtga actgtgtggg gagtcacatc   9780
   catacccggt gacctatcac atgagacaag ctcacccagg ttgtggccga tatgctggtg   9840
   gacaaggtta caatagcatt gggcattttt gtggaggatg ggctggtaac tgtggtgatg   9900
   gtggcatagg aggaagcact tggtatctgg tatgtgatcg ctgtagagaa aaatacctcc   9960
   gcgaaaaaca ggctgctgca agggagaagg tcaaacaatc taggagaaaa ccaatgcaag  10020
   tcaagacccc tcgtgccttg cccaccatgg aagctcacca ggtgattaaa gccaatgcac  10080
   tcttcctgct gtccctgagc agtgcagcag aaccgagcat tctgtgttac catcctgcaa  10140
   agccattcca atctcagttg cccagtgtaa aagaaggcat ttctgaggat cttcctgtga  10200
   aaatgccttg tctgtacctg cagacattag ctaggcatca tcatgaaaat tttgtgggct  10260
   atcaagatga caatctattc caggatgaaa tgagatatct acgttcaaca tctgtacctg  10320
   ccccgtatat atcagtaact cctgatgcaa gtcctaatgt atttgaagag ccagagagca  10380
   atatgaagtc tatgccacca agtttagaaa ccagtcccat aactgatact gatcttgcaa  10440
   agagaactgt cttccaaaga tcatactcag ttgttgcttc cgaatatgat aaacaacact  10500
   ccattttacc tgcacgagtt aaagctattc ctagaagaag agttaacagt ggagacactg  10560
   aagttggttc ttcccttttg agacatccgt ctcctgagct ttctcggcta atctcagccc  10620
   acagctctct ttctaaagga gaacgaaatt tccagtggcc agttttagct tttgttatac  10680
   aacatcatga tctagaaggt cttgaaatag caatgaaaca ggccctaagg aaatctgctt  10740
   gtcgagtttt tgctatggag gctttcaact ggcttctgtg taatgtcatc caaaccactt  10800
   ctctccatga tattctgtgg cattttgtgg catcactgac tcctgcacca gtggaaccag  10860
   aggaagaaga ggatgaagaa aataaaacaa gcaaagaaaa ttcagaacaa gagaaagata  10920
   caagagtatg tgaacatcca ctctcagaca tagtgattgc cggggaacgt gctcatcctt  10980
   taccacacac ctttcaccgc ttgctgcaga ccatctcaga ccttatgatg tctctcccca  11040
   gcggcagttc attacagcaa atggccctga ggtgctggag tctcaaattc aagcaatctg  11100
   atcaccagtt ccttcatcag agcaacgtct ttcatcacat taacaatatt ttgtcaaagt  11160
   cagatgatgg cgatagtgaa gagagtttta gcatcagtat acagtctggc tttgaagcta  11220
   tgagtcagga attatgcata gtaatgtgct taaaggactt aaccagcatt gttgacataa  11280
   aaacttcaag ccgacctgcc atgattggca gtttgacaga cggctccaca gaaacctttt  11340
   gggaatcagg agatgaagat aaaaacaaaa ctaagaacat caccatcaac tgtgtaaaag  11400
   gaatcaatgc ccgctatgtg tctgttcacg tggacaattc ccgagatctt gggaataaag  11460
   ttacctcaat gaccttctta actggcaaag cagtagaaga tttgtgcaga ataaagcagg  11520
   ttgatctgga ttccaggcac attggctggg taacaagtga acttccagga ggggataatc  11580
   acatcataaa aattgaatta aaaggcccag aaaatacact gagagttcga caagtcaaag  11640
   tcctgggctg gaaagatggt gaaagcacaa aaatagctgg ccagatttca gccagtgtgg 11700
   cccagcagag gaactgtgaa gctgagactc tgcgagtatt cagactgatt acgtctcaag 11760
   tatttggaaa gctcatctct ggagatgctg aacctacacc agaacaagag gaaaaagcac 11820
   tattgtcatc acctgaagga gaagaaaaag tatacaatgc aacatcagat gctgacctga 11880
   aagaacatat ggttggaatc atattcagca ggagtaagct gactaactta caaaaacagg 11940
   tgtgtgctca tattgtccaa gctattcgca tggaagctac cagagtccgt gaagaatggg 12000
   aacatgctat atcaagcaaa gaaaatgcca attctcagcc aaatgatgaa gatgcctcct 12060
   ctgatgccta ctgctttgag ctgctctcta tggttttagc actgagtggc tctaacgttg 12120
   gccggcaata tctggctcaa cagctaaccc tgcttcagga tctcttctcg ctgcttcaca 12180
   cagcctctcc tagagtccag agacaggtaa cctctttact aagaagagtt ttgcctgaag 12240
   taacccctag tcgtctggcc agcatcatag gagtgaaatc cctcccccca gcagatatca 12300
   gtgatatcat tcactcaaca gagaaaggag actggaataa gctgggtatc ttggacatgt 12360
   ttctaggatg cattgccaaa gcactcactg tacagctaaa agccaaagga accaccatca 12420
   ctggaacagc tggtaccact gtgggcaaag gagttacaac agttactctt ccgatgattt 12480
   tcaattccag ttatctccga cgaggtgaaa gtcattggtg gatgaagggc tcaaccccta 12540
   cccagatctc agagatcatc attaaactta tcaaggatat ggcagcaggt catctgtcag 12600
   aagcttggtc ccgagtgaca aaaaatgcta ttgcagaaac catcattgcc ttgaccaaga 12660
   tggaagaaga atttaggtct ccagtgagat gtattgcaac aactagactc tggcttgctc 12720
   tcgcatccct atgtgttctt gatcaggacc acgtagatcg tctctcctcg ggagatggga 12780
   tgggaaagga tggacaacaa aaacaaatgc ctatgtgtga taaccatgat gatggtgaaa 12840
   ctgcagcaat cattttatgc aatgtctgtg gaaatttatg tacagactgt gacagattcc 12900
   ttcaccttca tcgaagaacc aaaactcatc aaagacaggt cttcaaagaa gaagaagaag 12960
   ctataaaggt tgaccttcat gaaggttgtg gtagaaccaa attgttctgg ttgatggcac 13020
   tggcagattc taaaacaatg aaggcaatgg tggaattccg agaacacaca ggcaaaccca 13080
   ccacgagtag ctcagaagca tgtcgcttct gtggttccag gagtggaaca gagttatctg 13140
   ctgttggcag tgtttgttct gatgcagatt gccaggaata cgctaagata gcctgtagta 13200
   agacgcatcc ttgtggccat ccatgcgggg gtgttaaaaa cgaagagcac tgtctgccct 13260
   gtctacacgg ctgtgacaaa agtgccacaa gcctgaagca agacgccgat gacatgtgca 13320
   tgatatgttt caccgaagcg ctctcggcag caccagccat tcagctggat tgtagtcaca 13380
   tattccactt acagtgctgt cggcgagtat tagaaaatcg atggcttggc ccaaggataa 13440
   catttggatt tatatcttgt cccatttgca agaacaaaat taatcacata gtactaaaag 13500
   acctacttga tccaataaaa gaactctatg aggatgtcag aagaaaagcc ttaatgagat 13560
   tggaatatga aggtctgcat aagagtgaag ctatcacaac tcctggtgtg aggttttata 13620
   atgacccagc tggctatgca atgaatagat atgcatatta tgtgtgctac aaatgcagaa 13680
   aggcatattt tggtggtgaa gctcgctgcg atgctgaggc tggacgggga gatgattatg   13740
   atcccagaga gctcatttgt ggtgcctgtt ctgatgtttc cagggctcag atgtgtccca   13800
   aacatggcac agactttttg gaatataaat gtcgctactg ctgttcagtg gctgtttttt   13860
   tctgttttgg aacaacacat ttttgtaatg cttgtcatga tgattttcaa agaatgacta   13920
   gcattcctaa ggaagaacta ccacactgtc ctgcaggtcc caaaggcaag cagttagaag   13980
   gaactgaatg tccactccat gttgttcatc cacccactgg ggaagagttt gctctgggat   14040
   gtggagtgtg cagaaatgcc cacacttttt agaacacgca gatcctttgt ctacagagag   14100
   aaaaattgcc ttcatccccc aagaggatgc ggtgaagttt aaactctgct caccataagg   14160
   acgggaccat ttttacatcc atgaaaatga accattcaca gtgcaagaag gataccaaat   14220
   accatgtaca taattcttgc tatgaaaagt ttccccatta ttttggttta tcttcttttg   14280
   aacaaatgac atcaaacttg tgaggtgttt gcatgtggcc attaccgtca ttggcctgtg   14340
   aagcattgga catttataga taattgatat aaaagaatcg ccatgcccat ggactaagaa   14400
   cgatgctggc tttcaagcaa aaaagaaaaa taatcattgt ttattgtata ctgccttttt   14460
   gtaatcctgt acaattgcat cacgggtggg gataaaaaga ggaatattct ggtttatttc   14520
   ctagactgtt atttaaaaaa aaaaaaaaca ttgtgttagg acagcatata aatgtaataa   14580
   gtatcacact gtatataaac atatcaatgt ttgtcctgta taagaattac taaattacaa   14640
   atgcaatttc atttaaactt ctaggttaag tttgagcctg aaattttaat gaagtgcaat   14700
   actgagtgtg cctcattatc ttgcagctgt aaacatattg gaatgtacat gtcaataaaa   14760
   ccactgtaca tttttataca gtgataaagt ctaaaaaaaa aaaaaaa                 14807
<210> 2
   <211> 4641
   <212> PRT
   <213> Homo sapiens
<400> 2
   Met Pro Val Pro Asp Gly Ser Val Ala Ala Ala Gly Leu Gly Leu Gly
   1               5                   10                  15
   Leu Pro Ala Ala Asp Ser Pro Gly His Tyr Gln Leu Leu Leu Ser Gly
               20                  25                  30
   Arg Ala Leu Ala Asp Arg Tyr Arg Arg Ile Tyr Thr Ala Ala Leu Asn
           35                  40                  45
   Asp Arg Asp Gln Gly Gly Gly Ser Ala Gly His Pro Ala Ser Arg Asn
       50                  55                  60
   Lys Lys Ile Leu Asn Lys Lys Lys Leu Lys Arg Lys Gln Lys Ser Lys
   65                  70                  75                  80
   Ser Lys Val Lys Thr Arg Ser Lys Ser Glu Asn Leu Glu Asn Thr Val
   85 90 95
   Ile Ile Pro Asp Ile Lys Leu His Ser Asn Pro Ser Ala Phe Asn Ile
   100 105 110
   Tyr Cys Asn Val Arg His Cys Val Leu Glu Trp Gln Lys Lys Glu Ile
   115 120 125
   Ser Leu Ala Ala Ala Ser Lys Asn Ser Val Gln Ser Gly Glu Ser Asp
   130 135 140
   Ser Asp Glu Glu Glu Glu Ser Lys Glu Pro Pro Ile Lys Leu Pro Lys
   145 150 155 160
   Ile Ile Glu Val Gly Leu Cys Glu Val Phe Glu Leu Ile Lys Glu Thr
   165 170 175
   Arg Phe Ser His Pro Ser Leu Cys Leu Arg Ser Leu Gln Ala Leu Leu
   180 185 190
   Asn Val Leu Gln Gly Gln Gln Pro Glu Val Leu Gln Ser Glu Pro Pro
   195 200 205
   Glu Val Leu Glu Ser Leu Phe Gln Leu Leu Leu Glu Ile Thr Val Arg
   210 215 220
   Ser Thr Gly Met Asn Asp Ser Thr Gly Gln Ser Leu Thr Ala Leu Ser
   225 230 235 240
   Cys Ala Cys Leu Phe Ser Leu Val Ala Ser Trp Gly Glu Thr Gly Arg
   245 250 255
   Thr Leu Gln Ala Ile Ser Ala Ile Leu Thr Asn Asn Gly Ser His Ala
   260 265 270
   Cys Gln Thr Ile Gln Val Pro Thr Ile Leu Asn Ser Leu Gln Arg Ser
   275 280 285
   Val Gln Ala Val Leu Val Gly Lys Ile Gln Ile Gln Asp Trp Phe Ser
   290 295 300
   Asn Gly Ile Lys Lys Ala Ala Leu Met His Lys Trp Pro Leu Lys Glu
   305 310 315 320
   Ile Ser Val Asp Glu Asp Asp Gln Cys Leu Leu Gln Asn Asp Gly Phe
   325 330 335
   Phe Leu Tyr Leu Leu Cys Lys Asp Gly Leu Tyr Lys Ile Gly Ser Gly
   340 345 350
   Tyr Ser Gly Thr Val Arg Gly His Ile Tyr Asn Ser Thr Ser Arg Ile
   355 360 365
   Arg Asn Arg Lys Glu Lys Lys Ser Trp Leu Gly Tyr Ala Gln Gly Tyr
   370 375 380
   Leu Leu Tyr Arg Asp Val Asn Asn His Ser Met Thr Ala Ile Arg Ile
   385 390 395 400
   Ser Pro Glu Thr Leu Glu Gln Asp Gly Thr Val Met Leu Pro Asp Cys
   405 410 415
   His Thr Glu Gly Gln Asn Ile Leu Phe Thr Asp Gly Glu Tyr Ile Asn
   420 425 430
   Gln Ile Ala Ala Ser Arg Asp Asp Gly Phe Val Val Arg Ile Phe Ala
   435 440 445
   Thr Ser Thr Glu Pro Val Leu Gln Gln Glu Leu Gln Leu Lys Leu Ala
   450 455 460
   Arg Lys Cys Leu His Ala Cys Arg Ile Ser Leu Phe Asp Leu Glu Lys
   465 470 475 480
   Asp Leu His Ile Ile Ser Thr Gly Phe Asp Glu Glu Ser Ala Ile Leu
   485 490 495
   Gly Ala Gly Arg Glu Phe Ala Leu Met Lys Thr Ala Asn Gly Lys Ile
   500 505 510
   Tyr Tyr Thr Gly Lys Tyr Gln Ser Leu Gly Ile Lys Gln Gly Gly Pro
   515 520 525
   Ser Ala Gly Lys Trp Val Glu Leu Pro Ile Thr Lys Ser Pro Lys Ile
   530 535 540
   Val His Phe Ser Val Gly His Asp Gly Ser His Ala Leu Leu Val Ala
   545 550 555 560
   Glu Asp Gly Ser Ile Phe Phe Thr Gly Ser Ala Ser Lys Gly Glu Asp
   565 570 575
   Gly Glu Ser Ile Lys Ser Arg Arg Gln Ser Lys Pro Tyr Lys Pro Lys
   580 585 590
   Lys Ile Ile Lys Met Glu Gly Lys Ile Val Val Tyr Thr Ala Cys Asn
   595 600 605
   Asn Gly Ser Ser Ser Val Ile Ser Lys Asp Gly Glu Leu Tyr Met Phe
   610 615 620
   Gly Lys Asp Ala Ile Tyr Ser Asp Ser Ser Ser Leu Val Thr Asp Leu
   625                 630                 635                 640
   Lys Gly His Phe Val Thr Gln Val Ala Met Gly Lys Ala His Thr Cys
                   645                 650                 655
   Val Leu Met Lys Asn Gly Glu Val Trp Thr Phe Gly Val Asn Asn Lys
               660                 665                 670
   Gly Gln Cys Gly Arg Asp Thr Gly Ala Met Asn Gln Gly Gly Lys Gly
           675                 680                 685
   Phe Gly Val Glu Asn Met Ala Thr Ala Met Asp Glu Asp Leu Glu Glu
       690                 695                 700
   Glu Leu Asp Glu Lys Asp Glu Lys Ser Met Met Cys Pro Pro Gly Met
   705                 710                 715                 720
   His Lys Trp Lys Leu Glu Gln Cys Met Val Cys Thr Val Cys Gly Asp
                   725                 730                 735
   Cys Thr Gly Tyr Gly Ala Ser Cys Val Ser Ser Gly Arg Pro Asp Arg
               740                 745                 750
   Val Pro Gly Gly Ile Cys Gly Cys Gly Ser Gly Glu Ser Gly Cys Ala
           755                 760                 765
   Val Cys Gly Cys Cys Lys Ala Cys Ala Arg Glu Leu Asp Gly Gln Glu
       770                 775                 780
   Ala Arg Gln Arg Gly Ile Leu Asp Ala Val Lys Glu Met Ile Pro Leu
   785                 790                 795                 800
   Asp Leu Leu Leu Ala Val Pro Val Pro Gly Val Asn Ile Glu Glu His
                   805                 810                 815
   Leu Gln Leu Arg Gln Glu Glu Lys Arg Gln Arg Val Ile Arg Arg His
               820                 825                 830
   Arg Leu Glu Glu Gly Arg Gly Pro Leu Val Phe Ala Gly Pro Ile Phe
           835                 840                 845
   Met Asn His Arg Glu Gln Ala Leu Ala Arg Leu Arg Ser His Pro Ala
       850                 855                 860
   His Val Lys His Lys Arg Asp Lys His Lys Asp Gly Ser Gly Glu Arg
   865                 870                 875                 880
   Gly Glu Lys Asp Ala Ser Lys Ile Thr Thr Tyr Pro Pro Gly Ser Val
                   885                 890                 895
   Arg Phe Asp Cys Glu Leu Arg Ala Val Gln Val Ser Cys Gly Phe His
   900 905 910
   His Ser Val Val Leu Met Glu Asn Gly Asp Val Tyr Thr Phe Gly Tyr
   915 920 925
   Gly Gln His Gly Gln Leu Gly His Gly Asp Val Asn Ser Arg Gly Cys
   930 935 940
   Pro Thr Leu Val Gln Ala Leu Pro Gly Pro Ser Thr Gln Val Thr Ala
   945 950 955 960
   Gly Ser Asn His Thr Ala Val Leu Leu Met Asp Gly Gln Val Phe Thr
   965 970 975
   Phe Gly Ser Phe Ser Lys Gly Gln Leu Gly Arg Pro Ile Leu Asp Val
   980 985 990
   Pro Tyr Trp Asn Ala Lys Pro Ala Pro Met Pro Asn Ile Gly Ser Lys
   995 1000 1005
   Tyr Gly Arg Lys Ala Thr Trp Ile Gly Ala Ser Gly Asp Gln Thr
   1010 1015 1020
   Phe Leu Arg Ile Asp Glu Ala Leu Ile Asn Ser His Val Leu Ala
   1025 1030 1035
   Thr Ser Glu Ile Phe Ala Ser Lys His Ile Ile Gly Leu Val Pro
   1040 1045 1050
   Ala Ser Ile Ser Glu Pro Pro Pro Phe Lys Cys Leu Leu Ile Asn
   1055 1060 1065
   Lys Val Asp Gly Ser Cys Lys Thr Phe Asn Asp Ser Glu Gln Glu
   1070 1075 1080
   Asp Leu Gln Gly Phe Gly Val Cys Leu Asp Pro Val Tyr Asp Val
   1085 1090 1095
   Ile Trp Arg Phe Arg Pro Asn Thr Arg Glu Leu Trp Cys Tyr Asn
   1100 1105 1110
   Ala Val Val Ala Asp Ala Arg Leu Pro Ser Ala Ala Asp Met Gln
   1115 1120 1125
   Ser Arg Cys Ser Ile Leu Ser Pro Glu Leu Ala Leu Pro Thr Gly
   1130 1135 1140
   Ser Arg Ala Leu Thr Thr Arg Ser His Ala Ala Leu His Ile Leu
   1145 1150 1155
   Gly Cys Leu Asp Thr Leu Ala Ala Met Gln Asp Leu Lys Met Gly
   1160 1165 1170
   Val Ala Ser Thr Glu Glu Glu Thr Gln Ala Val Met Lys Val Tyr
   1175 1180 1185
   Ser Lys Glu Asp Tyr Ser Val Val Asn Arg Phe Glu Ser His Gly
   1190 1195 1200
   Gly Gly Trp Gly Tyr Ser Ala His Ser Val Glu Ala Ile Arg Phe
   1205 1210 1215
   Ser Ala Asp Thr Asp Ile Leu Leu Gly Gly Leu Gly Leu Phe Gly
   1220 1225 1230
   Gly Arg Gly Glu Tyr Thr Ala Lys Ile Lys Leu Phe Glu Leu Gly
   1235 1240 1245
   Pro Asp Gly Gly Asp His Glu Thr Asp Gly Asp Leu Leu Ala Glu
   1250 1255 1260
   Thr Asp Val Leu Ala Tyr Asp Cys Ala Ala Arg Glu Lys Tyr Ala
   1265 1270 1275
   Met Met Phe Asp Glu Pro Val Leu Leu Gln Ala Gly Trp Trp Tyr
   1280 1285 1290
   Val Ala Trp Ala Arg Val Ser Gly Pro Ser Ser Asp Cys Gly Ser
   1295 1300 1305
   His Gly Gln Ala Ser Ile Thr Thr Asp Asp Gly Val Val Phe Gln
   1310 1315 1320
   Phe Lys Ser Ser Lys Lys Ser Asn Asn Gly Thr Asp Val Asn Ala
   1325 1330 1335
   Gly Gln Ile Pro Gln Leu Leu Tyr Arg Leu Pro Thr Ser Asp Gly
   1340 1345 1350
   Ser Ala Ser Lys Gly Lys Gln Gln Thr Ser Glu Pro Val His Ile
   1355 1360 1365
   Leu Lys Arg Ser Phe Ala Arg Thr Val Ser Val Glu Cys Phe Glu
   1370 1375 1380
   Ser Leu Leu Ser Ile Leu His Trp Ser Trp Thr Thr Leu Val Leu
   1385 1390 1395
   Gly Val Glu Glu Leu Arg Gly Leu Lys Gly Phe Gln Phe Thr Ala
   1400 1405 1410
   Thr Leu Leu Asp Leu Glu Arg Leu Arg Phe Val Gly Thr Cys Cys
   1415 1420 1425
   Leu Arg Leu Leu Arg Val Tyr Thr Cys Glu Ile Tyr Pro Val Ser
   1430 1435 1440
   Ala Thr Gly Lys Ala Val Val Glu Glu Thr Ser Lys Leu Ala Glu
   1445 1450 1455
   Cys Ile Gly Lys Thr Arg Thr Leu Leu Arg Lys Ile Leu Ser Glu
   1460 1465 1470
   Pro Leu Asp His Cys Met Val Lys Leu Asp Asn Asp Pro Gln Gly
   1475 1480 1485
   Tyr Leu Ser Gln Pro Leu Ser Leu Leu Glu Ala Val Leu Gln Glu
   1490 1495 1500
   Cys His Asn Thr Phe Thr Ala Cys Phe His Ser Phe Tyr Pro Thr
   1505 1510 1515
   Pro Ala Leu Gln Trp Ala Cys Leu Cys Asp Leu Leu Asn Cys Leu
   1520 1525 1530
   Asp Gln Asp Ile Gln Glu Ala Asn Phe Lys Thr Ser Ser Ser Arg
   1535 1540 1545
   Leu Leu Ala Ala Val Met Ser Ala Leu Cys His Thr Ser Val Lys
   1550 1555 1560
   Leu Thr Ser Ile Phe Pro Ile Ala Tyr Asp Gly Glu Val Leu Leu
   1565 1570 1575
   Arg Ser Ile Val Lys Gln Val Ser Thr Glu Asn Asp Ser Thr Leu
   1580 1585 1590
   Val His Arg Phe Pro Leu Leu Val Ala His Met Glu Lys Leu Ser
   1595 1600 1605
   Gln Ser Glu Glu Asn Ile Ser Gly Met Thr Ser Phe Arg Glu Val
   1610 1615 1620
   Leu Glu Lys Met Leu Val Ile Val Val Leu Pro Val Arg Asn Ser
   1625 1630 1635
   Leu Arg Arg Glu Asn Glu Leu Phe Ser Ser His Leu Val Ser Asn
   1640 1645 1650
   Thr Cys Gly Leu Leu Ala Ser Ile Val Ser Glu Leu Thr Ala Ser
   1655 1660 1665
   Ala Leu Gly Ser Glu Val Asp Gly Leu Asn Ser Leu His Ser Val
   1670 1675 1680
   Lys Ala Ser Ala Asn Arg Phe Thr Lys Thr Ser Gln Gly Arg Ser
   1685 1690 1695
   Trp Asn Thr Gly Asn Gly Ser Pro Asp Ala Ile Cys Phe Ser Val
   1700 1705 1710
   Asp Lys Pro Gly Ile Val Val Val Gly Phe Ser Val Tyr Gly Gly
   1715 1720 1725
   Gly Gly Ile His Glu Tyr Glu Leu Glu Val Leu Val Asp Asp Ser
   1730 1735 1740
   Glu His Ala Gly Asp Ser Thr His Ser His Arg Trp Thr Ser Leu
   1745 1750 1755
   Glu Leu Val Lys Gly Thr Tyr Thr Thr Asp Asp Ser Pro Ser Asp
   1760 1765 1770
   Ile Ala Glu Ile Arg Leu Asp Lys Val Val Pro Leu Lys Glu Asn
   1775 1780 1785
   Val Lys Tyr Ala Val Arg Leu Arg Asn Tyr Gly Ser Arg Thr Ala
   1790 1795 1800
   Asn Gly Asp Gly Gly Met Thr Thr Val Gln Cys Pro Asp Gly Val
   1805 1810 1815
   Thr Phe Thr Phe Ser Thr Cys Ser Leu Ser Ser Asn Gly Thr Asn
   1820 1825 1830
   Gln Thr Arg Gly Gln Ile Pro Gln Ile Leu Tyr Tyr Arg Ser Glu
   1835 1840 1845
   Phe Asp Gly Asp Leu Gln Ser Gln Leu Leu Ser Lys Ala Asn Glu
   1850 1855 1860
   Glu Asp Lys Asn Cys Ser Arg Ala Leu Ser Val Val Ser Thr Val
   1865 1870 1875
   Val Arg Ala Ser Lys Asp Leu Leu His Arg Ala Leu Ala Val Asp
   1880 1885 1890
   Ala Asp Asp Ile Pro Glu Leu Leu Ser Ser Ser Ser Leu Phe Ser
   1895 1900 1905
   Met Leu Leu Pro Leu Ile Ile Ala Tyr Ile Gly Pro Val Ala Ala
   1910 1915 1920
   Ala Ile Pro Lys Val Ala Val Glu Val Phe Gly Leu Val Gln Gln
   1925 1930 1935
   Leu Leu Pro Ser Val Ala Ile Leu Asn Gln Lys Tyr Ala Pro Pro
   1940 1945 1950
   Ala Phe Asn Pro Asn Gln Ser Thr Asp Ser Thr Thr Gly Asn Gln
   1955 1960 1965
   Pro Glu Gln Gly Leu Ser Ala Cys Thr Thr Ser Ser His Tyr Ala
   1970 1975 1980
   Val Ile Glu Ser Glu His Pro Tyr Lys Pro Ala Cys Val Met His
   1985 1990 1995
   Tyr Lys Val Thr Phe Pro Glu Cys Val Arg Trp Met Thr Ile Glu
   2000 2005 2010
   Phe Asp Pro Gln Cys Gly Thr Ala Gln Ser Glu Asp Val Leu Arg
   2015 2020 2025
   Leu Leu Ile Pro Val Arg Thr Val Gln Asn Ser Gly Tyr Gly Pro
   2030 2035 2040
   Lys Leu Thr Ser Val His Glu Asn Leu Asn Ser Trp Ile Glu Leu
   2045 2050 2055
   Lys Lys Phe Ser Gly Ser Ser Gly Trp Pro Thr Met Val Leu Val
   2060 2065 2070
   Leu Pro Gly Asn Glu Ala Leu Phe Ser Leu Glu Thr Ala Ser Asp
   2075 2080 2085
   Tyr Val Lys Asp Asp Lys Ala Ser Phe Tyr Gly Phe Met Cys Phe
   2090 2095 2100
   Ala Ile Gly Tyr Glu Phe Ser Pro Gly Pro Asp Glu Gly Val Ile
   2105 2110 2115
   Gln Leu Glu Lys Glu Leu Ala Asn Leu Gly Gly Val Cys Ala Ala
   2120 2125 2130
   Ala Leu Met Lys Lys Asp Leu Ala Leu Pro Ile Gly Asn Glu Leu
   2135 2140 2145
   Glu Glu Asp Leu Glu Ile Leu Glu Glu Ala Ala Leu Gln Val Cys
   2150 2155 2160
   Lys Thr His Ser Gly Ile Leu Gly Lys Gly Leu Ala Leu Ser His
   2165 2170 2175
   Ser Pro Thr Ile Leu Glu Ala Leu Glu Gly Asn Leu Pro Leu Gln
   2180 2185 2190
   Ile Gln Ser Asn Glu Gln Ser Phe Leu Asp Asp Phe Ile Ala Cys
   2195 2200 2205
   Val Pro Gly Ser Ser Gly Gly Arg Leu Ala Arg Trp Leu Gln Pro
   2210 2215 2220
   Asp Ser Tyr Ala Asp Pro Gln Lys Thr Ser Leu Ile Leu Asn Lys
   2225 2230 2235
   Asp Asp Ile Arg Cys Gly Trp Pro Thr Thr Ile Thr Val Gln Thr
   2240 2245 2250
   Lys Asp Gln Tyr Gly Asp Val Val His Val Pro Asn Met Lys Val
   2255 2260 2265
   Glu Val Lys Ala Val Pro Val Ser Gln Lys Lys Met Ser Leu Gln
   2270 2275 2280
   Gln Asp Gln Ala Lys Lys Pro Gln Arg Ile Pro Gly Ser Pro Ala
   2285 2290 2295
   Val Thr Ala Ala Ser Ser Asn Thr Asp Met Thr Tyr Gly Gly Leu
   2300 2305 2310
   Ala Ser Pro Lys Leu Asp Val Ser Tyr Glu Pro Met Ile Val Lys
   2315 2320 2325
   Glu Ala Arg Tyr Ile Ala Ile Thr Met Met Lys Val Tyr Glu Asn
   2330 2335 2340
   Tyr Ser Phe Glu Glu Leu Arg Phe Ala Ser Pro Thr Pro Lys Arg
   2345 2350 2355
   Pro Ser Glu Asn Met Leu Ile Arg Val Asn Asn Asp Gly Thr Tyr
   2360 2365 2370
   Cys Ala Asn Trp Thr Pro Gly Ala Ile Gly Leu Tyr Thr Leu His
   2375 2380 2385
   Val Thr Ile Asp Gly Ile Glu Ile Asp Ala Gly Leu Glu Val Lys
   2390 2395 2400
   Val Lys Asp Pro Pro Lys Gly Met Ile Pro Pro Gly Thr Gln Leu
   2405 2410 2415
   Val Lys Pro Lys Ser Glu Pro Gln Pro Asn Lys Val Arg Lys Phe
   2420 2425 2430
   Val Ala Lys Asp Ser Ala Gly Leu Arg Ile Arg Ser His Pro Ser
   2435 2440 2445
   Leu Gln Ser Glu Gln Ile Gly Ile Val Lys Val Asn Gly Thr Ile
   2450 2455 2460
   Thr Phe Ile Asp Glu Ile His Asn Asp Asp Gly Val Trp Leu Arg
   2465 2470 2475
   Leu Asn Asp Glu Thr Ile Lys Lys Tyr Val Pro Asn Met Asn Gly
   2480 2485 2490
   Tyr Thr Glu Ala Trp Cys Leu Ser Phe Asn Gln His Leu Gly Lys
   2495 2500 2505
   Ser Leu Leu Val Pro Val Asp Glu Ser Lys Thr Asn Thr Asp Asp
   2510 2515 2520
   Phe Phe Lys Asp Ile Asn Ser Cys Cys Pro Gln Glu Ala Thr Met
   2525 2530 2535
   Gln Glu Gln Asp Met Pro Phe Leu Arg Gly Gly Pro Gly Met Tyr
   2540 2545 2550
   Lys Val Val Lys Thr Gly Pro Ser Gly His Asn Ile Arg Ser Cys
   2555 2560 2565
   Pro Asn Leu Arg Gly Ile Pro Ile Gly Met Leu Val Leu Gly Asn
   2570 2575 2580
   Lys Val Lys Ala Val Gly Glu Val Thr Asn Ser Glu Gly Thr Trp
   2585 2590 2595
   Val Gln Leu Asp Gln Asn Ser Met Val Glu Phe Cys Glu Ser Asp
   2600 2605 2610
   Glu Gly Glu Ala Trp Ser Leu Ala Arg Asp Arg Gly Gly Asn Gln
   2615 2620 2625
   Tyr Leu Arg His Glu Asp Glu Gln Ala Leu Leu Asp Gln Asn Ser
   2630 2635 2640
   Gln Thr Pro Pro Pro Ser Pro Phe Ser Val Gln Ala Phe Asn Lys
   2645 2650 2655
   Gly Ala Ser Cys Ser Ala Gln Gly Phe Asp Tyr Gly Leu Gly Asn
   2660 2665 2670
   Ser Lys Gly Asp Arg Gly Asn Ile Ser Thr Ser Ser Lys Pro Ala
   2675 2680 2685
   Ser Thr Ser Gly Lys Ser Glu Leu Ser Ser Lys His Ser Arg Ser
   2690 2695 2700
   Leu Lys Pro Asp Gly Arg Met Ser Arg Thr Thr Ala Asp Gln Lys
   2705 2710 2715
   Lys Pro Arg Gly Thr Glu Ser Leu Ser Ala Ser Glu Ser Leu Ile
   2720 2725 2730
   Leu Lys Ser Asp Ala Ala Lys Leu Arg Ser Asp Ser His Ser Arg
   2735 2740 2745
   Ser Leu Ser Pro Asn His Asn Thr Leu Gln Thr Leu Lys Ser Asp
   2750 2755 2760
   Gly Arg Met Pro Ser Ser Ser Arg Ala Glu Ser Pro Gly Pro Gly
   2765 2770 2775
   Ser Arg Leu Ser Ser Pro Lys Pro Lys Thr Leu Pro Ala Asn Arg
   2780 2785 2790
   Ser Ser Pro Ser Gly Ala Ser Ser Pro Arg Ser Ser Ser Pro His
   2795 2800 2805
   Asp Lys Asn Leu Pro Gln Lys Ser Thr Ala Pro Val Lys Thr Lys
   2810 2815 2820
   Leu Asp Pro Pro Arg Glu Arg Ser Lys Ser Asp Ser Tyr Thr Leu
   2825 2830 2835
   Asp Pro Asp Thr Leu Arg Lys Lys Lys Met Pro Leu Thr Glu Pro
   2840 2845 2850
   Leu Arg Gly Arg Ser Thr Ser Pro Lys Pro Lys Ser Val Pro Lys
   2855 2860 2865
   Asp Ser Thr Asp Ser Pro Gly Ser Glu Asn Arg Ala Pro Ser Pro
   2870 2875 2880
   His Val Val Gln Glu Asn Leu His Ser Glu Val Val Glu Val Cys
   2885 2890 2895
   Thr Ser Ser Thr Leu Lys Thr Asn Ser Leu Thr Asp Ser Thr Cys
   2900 2905 2910
   Asp Asp Ser Ser Glu Phe Lys Ser Val Asp Glu Gly Ser Asn Lys
   2915 2920 2925
   Val His Phe Ser Ile Gly Lys Ala Pro Leu Lys Asp Glu Gln Glu
   2930 2935 2940
   Met Arg Ala Ser Pro Lys Ile Ser Arg Lys Cys Ala Asn Arg His
   2945 2950 2955
   Thr Arg Pro Lys Lys Glu Lys Ser Ser Phe Leu Phe Lys Gly Asp
   2960 2965 2970
   Gly Ser Lys Pro Leu Glu Pro Ala Lys Gln Ala Met Ser Pro Ser
   2975 2980 2985
   Val Ala Glu Cys Ala Arg Ala Val Phe Ala Ser Phe Leu Trp His
   2990 2995 3000
   Glu Gly Ile Val His Asp Ala Met Ala Cys Ser Ser Phe Leu Lys
   3005 3010 3015
   Phe His Pro Glu Leu Ser Lys Glu His Ala Pro Ile Arg Ser Ser
   3020 3025 3030
   Leu Asn Ser Gln Gln Pro Thr Glu Glu Lys Glu Thr Lys Leu Lys
   3035 3040 3045
   Asn Arg His Ser Leu Glu Ile Ser Ser Ala Leu Asn Met Phe Asn
   3050 3055 3060
   Ile Ala Pro His Gly Pro Asp Ile Ser Lys Met Gly Ser Ile Asn
   3065 3070 3075
   Lys Asn Lys Val Leu Ser Met Leu Lys Glu Pro Pro Leu His Glu
   3080 3085 3090
   Lys Cys Glu Asp Gly Lys Thr Glu Thr Thr Phe Glu Met Ser Met
   3095 3100 3105
   His Asn Thr Met Lys Ser Lys Ser Pro Leu Pro Leu Thr Leu Gln
   3110 3115 3120
   His Leu Val Ala Phe Trp Glu Asp Ile Ser Leu Ala Thr Ile Lys
   3125 3130 3135
   Ala Ala Ser Gln Asn Met Ile Phe Pro Ser Pro Gly Ser Cys Ala
   3140 3145 3150
   Val Leu Lys Lys Lys Glu Cys Glu Lys Gly Arg Asn Lys Lys Ser
   3155 3160 3165
   Lys Lys Glu Lys Lys Lys Lys Glu Lys Ala Glu Val Arg Pro Arg
   3170 3175 3180
   Gly Asn Leu Phe Gly Glu Met Ala Gln Leu Ala Val Gly Gly Pro
   3185 3190 3195
   Glu Lys Asp Thr Ile Cys Glu Leu Cys Gly Glu Ser His Pro Tyr
   3200 3205 3210
   Pro Val Thr Tyr His Met Arg Gln Ala His Pro Gly Cys Gly Arg
   3215 3220 3225
   Tyr Ala Gly Gly Gln Gly Tyr Asn Ser Ile Gly His Phe Cys Gly
   3230 3235 3240
   Gly Trp Ala Gly Asn Cys Gly Asp Gly Gly Ile Gly Gly Ser Thr
   3245 3250 3255
   Trp Tyr Leu Val Cys Asp Arg Cys Arg Glu Lys Tyr Leu Arg Glu
   3260 3265 3270
   Lys Gln Ala Ala Ala Arg Glu Lys Val Lys Gln Ser Arg Arg Lys
   3275 3280 3285
   Pro Met Gln Val Lys Thr Pro Arg Ala Leu Pro Thr Met Glu Ala
   3290 3295 3300
   His Gln Val Ile Lys Ala Asn Ala Leu Phe Leu Leu Ser Leu Ser
   3305 3310 3315
   Ser Ala Ala Glu Pro Ser Ile Leu Cys Tyr His Pro Ala Lys Pro
   3320 3325 3330
   Phe Gln Ser Gln Leu Pro Ser Val Lys Glu Gly Ile Ser Glu Asp
   3335 3340 3345
   Leu Pro Val Lys Met Pro Cys Leu Tyr Leu Gln Thr Leu Ala Arg
   3350 3355 3360
   His His His Glu Asn Phe Val Gly Tyr Gln Asp Asp Asn Leu Phe
   3365 3370 3375
   Gln Asp Glu Met Arg Tyr Leu Arg Ser Thr Ser Val Pro Ala Pro
   3380 3385 3390
   Tyr Ile Ser Val Thr Pro Asp Ala Ser Pro Asn Val Phe Glu Glu
   3395 3400 3405
   Pro Glu Ser Asn Met Lys Ser Met Pro Pro Ser Leu Glu Thr Ser
   3410 3415 3420
   Pro Ile Thr Asp Thr Asp Leu Ala Lys Arg Thr Val Phe Gln Arg
   3425 3430 3435
   Ser Tyr Ser Val Val Ala Ser Glu Tyr Asp Lys Gln His Ser Ile
   3440 3445 3450
   Leu Pro Ala Arg Val Lys Ala Ile Pro Arg Arg Arg Val Asn Ser
   3455 3460 3465
   Gly Asp Thr Glu Val Gly Ser Ser Leu Leu Arg His Pro Ser Pro
   3470 3475 3480
   Glu Leu Ser Arg Leu Ile Ser Ala His Ser Ser Leu Ser Lys Gly
   3485 3490 3495
   Glu Arg Asn Phe Gln Trp Pro Val Leu Ala Phe Val Ile Gln His
   3500 3505 3510
   His Asp Leu Glu Gly Leu Glu Ile Ala Met Lys Gln Ala Leu Arg
   3515 3520 3525
   Lys Ser Ala Cys Arg Val Phe Ala Met Glu Ala Phe Asn Trp Leu
   3530 3535 3540
   Leu Cys Asn Val Ile Gln Thr Thr Ser Leu His Asp Ile Leu Trp
   3545 3550 3555
   His Phe Val Ala Ser Leu Thr Pro Ala Pro Val Glu Pro Glu Glu
   3560 3565 3570
   Glu Glu Asp Glu Glu Asn Lys Thr Ser Lys Glu Asn Ser Glu Gln
   3575 3580 3585
   Glu Lys Asp Thr Arg Val Cys Glu His Pro Leu Ser Asp Ile Val
   3590 3595 3600
   Ile Ala Gly Glu Arg Ala His Pro Leu Pro His Thr Phe His Arg
   3605 3610 3615
   Leu Leu Gln Thr Ile Ser Asp Leu Met Met Ser Leu Pro Ser Gly
   3620 3625 3630
   Ser Ser Leu Gln Gln Met Ala Leu Arg Cys Trp Ser Leu Lys Phe
   3635 3640 3645
   Lys Gln Ser Asp His Gln Phe Leu His Gln Ser Asn Val Phe His
   3650 3655 3660
   His Ile Asn Asn Ile Leu Ser Lys Ser Asp Asp Gly Asp Ser Glu
   3665 3670 3675
   Glu Ser Phe Ser Ile Ser Ile Gln Ser Gly Phe Glu Ala Met Ser
   3680 3685 3690
   Gln Glu Leu Cys Ile Val Met Cys Leu Lys Asp Leu Thr Ser Ile
   3695 3700 3705
   Val Asp Ile Lys Thr Ser Ser Arg Pro Ala Met Ile Gly Ser Leu
   3710 3715 3720
   Thr Asp Gly Ser Thr Glu Thr Phe Trp Glu Ser Gly Asp Glu Asp
   3725 3730 3735
   Lys Asn Lys Thr Lys Asn Ile Thr Ile Asn Cys Val Lys Gly Ile
   3740 3745 3750
   Asn Ala Arg Tyr Val Ser Val His Val Asp Asn Ser Arg Asp Leu
   3755 3760 3765
   Gly Asn Lys Val Thr Ser Met Thr Phe Leu Thr Gly Lys Ala Val
   3770 3775 3780
   Glu Asp Leu Cys Arg Ile Lys Gln Val Asp Leu Asp Ser Arg His
   3785 3790 3795
   Ile Gly Trp Val Thr Ser Glu Leu Pro Gly Gly Asp Asn His Ile
   3800 3805 3810
   Ile Lys Ile Glu Leu Lys Gly Pro Glu Asn Thr Leu Arg Val Arg
   3815 3820 3825
   Gln Val Lys Val Leu Gly Trp Lys Asp Gly Glu Ser Thr Lys Ile
   3830 3835 3840
   Ala Gly Gln Ile Ser Ala Ser Val Ala Gln Gln Arg Asn Cys Glu
   3845 3850 3855
   Ala Glu Thr Leu Arg Val Phe Arg Leu Ile Thr Ser Gln Val Phe
   3860 3865 3870
   Gly Lys Leu Ile Ser Gly Asp Ala Glu Pro Thr Pro Glu Gln Glu
   3875 3880 3885
   Glu Lys Ala Leu Leu Ser Ser Pro Glu Gly Glu Glu Lys Val Tyr
   3890 3895 3900
   Asn Ala Thr Ser Asp Ala Asp Leu Lys Glu His Met Val Gly Ile
   3905 3910 3915
   Ile Phe Ser Arg Ser Lys Leu Thr Asn Leu Gln Lys Gln Val Cys
   3920 3925 3930
   Ala His Ile Val Gln Ala Ile Arg Met Glu Ala Thr Arg Val Arg
   3935 3940 3945
   Glu Glu Trp Glu His Ala Ile Ser Ser Lys Glu Asn Ala Asn Ser
   3950 3955 3960
   Gln Pro Asn Asp Glu Asp Ala Ser Ser Asp Ala Tyr Cys Phe Glu
   3965 3970 3975
   Leu Leu Ser Met Val Leu Ala Leu Ser Gly Ser Asn Val Gly Arg
   3980 3985 3990
   Gln Tyr Leu Ala Gln Gln Leu Thr Leu Leu Gln Asp Leu Phe Ser
   3995 4000 4005
   Leu Leu His Thr Ala Ser Pro Arg Val Gln Arg Gln Val Thr Ser
   4010 4015 4020
   Leu Leu Arg Arg Val Leu Pro Glu Val Thr Pro Ser Arg Leu Ala
   4025 4030 4035
   Ser Ile Ile Gly Val Lys Ser Leu Pro Pro Ala Asp Ile Ser Asp
   4040 4045 4050
   Ile Ile His Ser Thr Glu Lys Gly Asp Trp Asn Lys Leu Gly Ile
   4055 4060 4065
   Leu Asp Met Phe Leu Gly Cys Ile Ala Lys Ala Leu Thr Val Gln
   4070 4075 4080
   Leu Lys Ala Lys Gly Thr Thr Ile Thr Gly Thr Ala Gly Thr Thr
   4085 4090 4095
   Val Gly Lys Gly Val Thr Thr Val Thr Leu Pro Met Ile Phe Asn
   4100 4105 4110
   Ser Ser Tyr Leu Arg Arg Gly Glu Ser His Trp Trp Met Lys Gly
   4115 4120 4125
   Ser Thr Pro Thr Gln Ile Ser Glu Ile Ile Ile Lys Leu Ile Lys
   4130 4135 4140
   Asp Met Ala Ala Gly His Leu Ser Glu Ala Trp Ser Arg Val Thr
   4145 4150 4155
   Lys Asn Ala Ile Ala Glu Thr Ile Ile Ala Leu Thr Lys Met Glu
   4160 4165 4170
   Glu Glu Phe Arg Ser Pro Val Arg Cys Ile Ala Thr Thr Arg Leu
   4175 4180 4185
   Trp Leu Ala Leu Ala Ser Leu Cys Val Leu Asp Gln Asp His Val
   4190 4195 4200
   Asp Arg Leu Ser Ser Gly Arg Trp Met Gly Lys Asp Gly Gln Gln
   4205 4210 4215
   Lys Gln Met Pro Met Cys Asp Asn His Asp Asp Gly Glu Thr Ala
   4220 4225 4230
   Ala Ile Ile Leu Cys Asn Val Cys Gly Asn Leu Cys Thr Asp Cys
   4235 4240 4245
   Asp Arg Phe Leu His Leu His Arg Arg Thr Lys Thr His Gln Arg
   4250 4255 4260
   Gln Val Phe Lys Glu Glu Glu Glu Ala Ile Lys Val Asp Leu His
   4265 4270 4275
   Glu Gly Cys Gly Arg Thr Lys Leu Phe Trp Leu Met Ala Leu Ala
   4280 4285 4290
   Asp Ser Lys Thr Met Lys Ala Met Val Glu Phe Arg Glu His Thr
   4295 4300 4305
   Gly Lys Pro Thr Thr Ser Ser Ser Glu Ala Cys Arg Phe Cys Gly
   4310 4315 4320
   Ser Arg Ser Gly Thr Glu Leu Ser Ala Val Gly Ser Val Cys Ser
   4325 4330 4335
   Asp Ala Asp Cys Gln Glu Tyr Ala Lys Ile Ala Cys Ser Lys Thr
   4340 4345 4350
   His Pro Cys Gly His Pro Cys Gly Gly Val Lys Asn Glu Glu His
   4355 4360 4365
   Cys Leu Pro Cys Leu His Gly Cys Asp Lys Ser Ala Thr Ser Leu
   4370 4375 4380
   Lys Gln Asp Ala Asp Asp Met Cys Met Ile Cys Phe Thr Glu Ala
   4385 4390 4395
   Leu Ser Ala Ala Pro Ala Ile Gln Leu Asp Cys Ser His Ile Phe
   4400 4405 4410
   His Leu Gln Cys Cys Arg Arg Val Leu Glu Asn Arg Trp Leu Gly
   4415 4420 4425
   Pro Arg Ile Thr Phe Gly Phe Ile Ser Cys Pro Ile Cys Lys Asn
   4430 4435 4440
   Lys Ile Asn His Ile Val Leu Lys Asp Leu Leu Asp Pro Ile Lys
   4445 4450 4455
   Glu Leu Tyr Glu Asp Val Arg Arg Lys Ala Leu Met Arg Leu Glu
   4460 4465 4470
   Tyr Glu Gly Leu His Lys Ser Glu Ala Ile Thr Thr Pro Gly Val
       4475                    4480                    4485
   Arg Phe Tyr Asn Asp Pro Ala Gly Tyr Ala Met Asn Arg Tyr Ala
       4490                    4495                    4500
   Tyr Tyr Val Cys Tyr Lys Cys Arg Lys Ala Tyr Phe Gly Gly Glu
       4505                    4510                    4515
   Ala Arg Cys Asp Ala Glu Ala Gly Arg Gly Asp Asp Tyr Asp Pro
       4520                    4525                    4530
   Arg Glu Leu Ile Cys Gly Ala Cys Ser Asp Val Ser Arg Ala Gln
       4535                    4540                    4545
   Met Cys Pro Lys His Gly Thr Asp Phe Leu Glu Tyr Lys Cys Arg
       4550                    4555                    4560
   Tyr Cys Cys Ser Val Ala Val Phe Phe Cys Phe Gly Thr Thr His
       4565                    4570                    4575
   Phe Cys Asn Ala Cys His Asp Asp Phe Gln Arg Met Thr Ser Ile
       4580                    4585                    4590
   Pro Lys Glu Glu Leu Pro His Cys Pro Ala Gly Pro Lys Gly Lys
       4595                    4600                    4605
   Gln Leu Glu Gly Thr Glu Cys Pro Leu His Val Val His Pro Pro
       4610                    4615                    4620
   Thr Gly Glu Glu Phe Ala Leu Gly Cys Gly Val Cys Arg Asn Ala
       4625                    4630                    4635
   His Thr Phe
       4640
<210> 3
   <211> 290040
   <212> DNA
   <213> Homo sapiens
<400> 3

## Description

The invention concerns the use of PAM (Protein Associated with Myc) for the preparation of pharmaceutical compounds and for screening pharmaceuticals for the treatment of pain.

Pain is a complex subjective sensation reflecting real or potential tissue damage and the affective response to it. Acute pain is a physiological signal indicating a potential or actual injury. Chronic pain can either be somatogenetic (organic) or psychogenic. Chronic pain is frequently accompanied or followed by vegetative signs, which often result in depression.

Somatogenetic pain may be of nociceptive origin, inflammatory or neuropathic. Nociceptive pain is judged to be commensurate with ongoing activation of somatic or visceral pain-sensitive nerve fibers. Neuropathic pain results from dysfunction in the nervous system; it is believed to be sustained by aberrant somatosensory processes in the peripheral nervous system, the CNS, or both. (For an overview of pain mechanisms, see for example Scholz and Woolf, 2002; Julius and Basbaum, 2001, Woolf and Mannion, 1999; Wood, J.D., 2000; Woolf and Salter, 2000.)

Chronic pain results in individual suffering and social economic costs of tremendous extent. Existing pharmacological pain therapies are widely unsatisfying both in terms of efficacy and of safety.

Up to now, two classes of analgesics are mainly employed for the treatment of pain: Non-opioid analgesics, mostly acetaminophen and NSAIDS (non-steroidal anti-inflammatory drugs) and opioid (narcotic) agonists (wherein "opioid" is a generic term for natural or synthetic substances that bind to specific opioid receptors in the CNS, producing an agonist action). Unfortunately both analgesic classes, opioids and non-opioids, have several unwanted side effects. The most serious side effects of opioids are the possibility of inhibition of the respiratory system and after long-term treatment the possibility of addiction (Schaible H.G., Vanegas H., 2000). NSAIDs, a major class of non-opioids, on the other hand, can induce a variety of gastrointestinal complications such as ulcers and bleeding, but also kidney damage (Schaible H.G., Vanegas H., 2000). It has been estimated that in the U.S.A. about 16.000 patients die every year because of severe gastro-intestinal complications caused by conventional NSAIDs.

In light of the severe drawbacks connected with state of the art pain treatments, there is a great need for novel classes of pain modulating drugs. Especially in light of the vast gap between the fast advancing understanding of the neurobiology of pain and the unmet clinical need to provide effective treatments without the drawbacks of state of the art treatments, efforts need to be directed to the discovery of new targets for novel classes of analgesics. Thus, it is the object of the present invention to provide a new means for the development and provision of a new class of pain modulating drugs.

This object is solved by the use of PAM or functional fragments thereof for the preparation of pharmaceutical compounds that modulate pain.

The invention is based on findings of the inventors that demonstrate for the first time the implication of PAM in nociceptive processing and its ability to decrease pain. The term functional refers to the ability of PAM to lower intracellular cAMP levels or to interact with AC; more preferably it refers to its ability to inhibit AC activity and even more preferably to its ability to decrease pain.

A fragment of PAM can be any polypeptide or polynucleotide that is shorter than the corresponding wild type, e.g. shorter than homo sapiens (hs) PAM according to one of the sequences according the accession numbers ACC 39928 (SEQ ID No. 1), NP_055872, NM_015057, AF075587 (SEQ ID No.2) or shorter than a polynucleotide from position 24679861 to position 24962245 of the sequence according to accession number NT_024524.11 (SEQ ID No.3).

Also described are derivatives of PAM or of a PAM fragment which can be any modification of the amino acid or nucleotide sequence having PAM function or any other kind of modification, such as a chemical or biological modification e.g. leading to the stabilization of the polypeptide or polynucleotide (such as phosphoorothioate modifications), or enabling a specific targeting of the polypeptide or polynucleotide to certain cells or facilitating its entry into or uptake by cells (such as cell-permeant phosphopeptides or the coupling to cell-permeant peptide vectors, e.g. based on the antennapedia/penetratin, TAT, and signal-peptide based sequences; or coupling to parts of ligands for specific transporters or importers).

PAM or functional fragments thereof may either be used in the form of a polypeptide or a polynucleotide. Useful are suitable modifications or additives for ensuring or facilitating its targeting to the site of need and its entering the cell. On the other hand, a local application, such as an intraspinal application using suitable catheters, etc. or the like is possible for ensuring its targeting to the spinal cord. Other useful additives include salts, buffers or the like for its stabilization, etc.

The PAM polynucleotide or functional fragment thereof can e.g. be put into a suitable vector that ensures its intracellular expression and, preferably also its targeting into the cell. A cell type specific expression can be ensured using appropriate promoters/enhancers of neuron-specific genes which are known in the art The use of PAM oligonucleotides is also possible.

The present invention is based on studies of the inventors, that demonstrate for the first time the surprising implication of PAM in sensitisation mechanisms within the spinal cord and dorsal root ganglia (DRGs).

PAM (Protein Associated with Myc) is a giant protein of 510 kDa. The protein, genomic and coding polynucleotide sequences of PAM are known in the state of the art and are, e. g. publicly available from the NCBI (National Centre for Biotechnology Information; National Library of Medicine, Building 38A, Bethesda, MD 20894, USA; www.ncbi.nhm.nih.gov) data base under the accession numbers AAC39928 (coding sequence; SEQ ID No.1), AF075587 (protein sequence; SEQ ID No.2). Human PAM is located on Chromosome 13q22; its genomic sequence is publicly available under NT_024524.11 (Start: position 24679861; Stop: position 24962245; SEQ ID No.3). Alternatively, the protein and coding sequence are publicly available under KIAA0916 protein Accession NP_055872 (protein sequence) and NM_015057 (coding sequence).

"For rat PAM, the following EST-clone coding sequences are publicly available:
AW921303 (corresponds to bp 960-1394 of hs cDNA; SEQ ID No.4)
AW918711 (corresponds to bp 8188-8632 of hs cDNA; SEQ ID No.5)
BQ201485 (corresponds to bp 8966-9633 of hs cDNA; SEQ ID No.6)
BE112881 (corresponds to bp 10311-10830 of hs cDNA; SEQ ID No.7)
AW441131 (corresponds to bp 13569-14152 of hs cDNA; SEQ ID No.8)
BF409872 (corresponds to bp 13569-14807 of hs cDNA; SEQ ID No.9)..

PAM was originally identified by its ability to interact specifically with the transcriptional activating domain in the N-Terminus of Myc (Guo Q., et al., 1998). PAM has recently been described as a powerful inhibitor of AC activity (Scholich K., Pierre S., Patel T.B.: Protein associated with myc (PAM) is a potent inhibitor of adenylyl cyclase. J. Biol. Chem. 2001, Dec 14;276(50):47583-9.), but there has been no evidence of its function in nociceptive processing and sensitisation, so far.

Rather, PAM is believed to be playing a role in presynaptic growth regulation: PAM mRNA has been known to be highly expressed in specific anatomical regions, including hippocampus, dentate gyrus and cerebellum. Both PAM and Myc expression in the brain of adult rats and mice is confined to the maturing Purkinje cells in the cerebellum and granule and pyramidal cells in the hippocampus (Ruppert C., et al., 1986; Yang H. et al., 2002). None of these cell types, however is known to be involved in pain processing and sensitisation.

PAM homologues in Drosophila (highwire) and C. elegans (rpm-1) have been shown to play a crucial role in presynaptic terminal organization (Zhen et al., 2000), the regulation of synaptic growth (Wan et al.,2000), synaptogenesis, and neurite growth and targeting (Schaefer et al., 2000). These findings led to the assumption that highwire, rpm-1 and their mammalian homolog PAM might act as negative regulators of synaptic growth (Chang et al., 2000; Jin Y. 2002). Accordingly, a dramatic increase in PAM expression in the cerebellum, hippocampus and dentate gyrus was found during the major synaptogenic period in these structures (Yang et al., 2002).

During brain development in rodents, PAM expression is turned on shortly after birth, up-regulated during the first two weeks, and, thereafter, PAM expression remains elevated during adulthood (Yang et al., 2002). So far, nothing has been known about the expression and regulation of PAM in the spinal cord and DRGs and its function in sensitisation mechanisms and regulation of pain.

Previously, it has been demonstrated that human PAM is a potent regulator of cyclic AMP (cAMP)-signaling and inhibits the enzyme activity of several adenylyl cyclase (AC; E.C.4.6.1.1) isoforms at nanomolar concentrations (Scholich et al. 2001).

The ubiquitous cyclic AMP (cAMP) second messenger system is one of different signal transduction mechanisms translating extracellular stimuli to intracellular signals and responses. Upon extracellular stimulation, G-protein coupled receptors (GPCRs) modulate plasma-membrane bound enzymes or ion channels via trimeric GTP-binding regulatory proteins (G-proteins). One of the enzymes modulated in its activity by GPCRs is the adenylyl cyclase (AC), a cAMP generating enzyme. Thus, the incoming extracellular stimuli influence the intracellular concentration of the intracellular mediator cyclic AMP. A rise in cAMP levels affects the cell by stimulating protein kinase A (PKA), which phosphorylates specific intracellular target proteins and thereby alters their activity.

Each type of cell has characteristic sets of GPCRs, enzymes modulated by those GPCRs, specific subsets of adenylyl cyclase (AC) and target proteins, that, acting together with more unspecific or generally occurring players (such as the ubiquitous cAMP), enable each cell to make its own distinctive response to incoming extracellular signals. It is for example known that the cyclic AMP (cAMP)-second messenger plays a major role in the regulation of synaptic plasticity (Bailey et al., 1996; Xia et al., 1997; Brandon et al., 1997); on the other hand it is involved in metabolic processes and cellular proliferation. Thus, the role of the ubiquitous cAMP messenger system and its different components varies according to different specializations of different tissue and cell types.

A further aspect of the invention regards the use of PAM or functional fragments thereof for the modulation of pain. This modulation is a lessening or prevention or total inhibition.

Moreover, the use of PAM or functional fragments thereof for identifying compounds that treat and/or prevent pain is encompassed by present invention. The compounds are PAM activating compounds, and have the ability to prevent, lessen or abolish pain.

The compounds can for example be identified by their ability to
a) activate or enhance PAM function (i.e. its ability to lower intracellular cAMP levels, to interact with other factors like AC, especially with AC, to inhibit AC or its ability to lower the pain perception) or
b) activate PAM on RNA level (i.e. by activation of PAM transcription or transcript stabilisation) or
c) activate PAM on protein level (i.e. by activation of PAM translation or its posttranslational processing; by modulation of PAM posttranslational modification or by activation of its stabilisation or inhibition of its degradation).

In this aspect, PAM (or its functional fragment) can either be used as a polypeptide or oligo- or polynucleotide.

Another aspect of present invention provides means for lessening pain comprising administering a sufficient amount of PAM or a functional fragment thereof to an individual.

Administration should suitably be performed in a way that allows for targeting of PAM to the site of action (DRG or spinal cord), e.g. by systemical administration of PAM derivatives or formulations targeting themselves to site of need or local (e.g. intraspinal) application of PAM or its functional fragments.

Another aspect of the invention concerns a method of screening for pharmaceuticals useful for treating and/or preventing pain, comprising the steps
a. Providing two samples
b. contacting one sample containing PAM or a functional fragment or derivative thereof with a compound,
c. determining the PAM activity in the presence of compound,
d. determining the PAM activity in the absence of compound, and
e. comparing the PAM activity according to c) with that according to d).

An enhanced PAM activity according to e) identifies a compound as having the ability to treat/Prevent Pain. PAM can be derived from an sequence available that allows for its specific purpose according to the different aspects of the present invention. Preferably, PAM is human PAM.

For the different aspects of present invention it is also preferred, if PAM is an isolated polypeptide or a oligo- or polynucleotide. Isolated in the context of the different aspects of present invention means at least partially purified from a natural source or recombinant PAM (which can, of course, also be purified or partially purified).

An assay is any type of analytical method to monitor a biological process. For the use in drug screening, the assay needs to be reproducible and is preferably also scalable and robust. The assay is preferably suitable for high throughput screening of chemical substances for their ability of modulating (preferably diminishing) and / or preventing pain. The type of assay depends e.g. on the type of PAM used (either polypeptide or polynucleotide) and the "read out", i.e. the way in which PAM activity is determined (see below).

Different types of such assays are commonly known in the state of the art and commercially available from commercial suppliers. Suitable assays encompass radioisotopic or fluorescent assays, for example fluorescence polarization assays (such as those offered commercially by Panvera, Perkin-Elmer life sciences (e.g. LANCE) or Packard BioScience (e.g. HTRF or ALPHAscreen^{™})) for measuring the interaction of a labeled member with a non-labeled member (e.g. PAM or fragments thereof could be labeled and their interaction with AC could be monitored).

More examples include cell based assays, wherein a cell line stably (inducibly or not; chromosomal or episomal) or transiently expresses a recombinant protein of interest. These assays comprise e.g. reporter gene assays, wherein the regulation of a certain promotor or a signal transduction pathway of a member of a signal transduction cascade is measured according to the activity of a reporter enzyme, the expression of which is under the control of said certain promotor. For this type of assay, a recombinant cell line has to be constructed containing the reporter gene under the control of a defined promotor that is to be investigated itself or that is regulated by the signaling cascade under investigation. Suitable reporter enzymes are commonly known within the state of the art and comprise firefly luciferase, renilla luciferase (e.g. commercially available by Packard reagents), ß-Galactosidase. Suitable cell lines depend on the aim of the assay but comprise mostly cell lines that are easy to transfect and easy to cultivate, such as, e.g. HeLA, COS, CHO, NIH-3T3, etc.

Assays for measuring the intracellular ion level comprise e.g. FLIPR (fluorometric imaging plate reader, commercially available from Molecular Devices) assays, wherein an argon laser light source combined with a cooled CCD camera allows for parallel measurements in 384 well plates transient ion-signals (such as Ca²⁺, etc) within cells (e.g. neuronal cells or other cells (e.g. cells recombinantly or naturally expressing certain ion channels). FLIPR assays allow e.g. for monitoring of intracellular calcium using certain fluorochromes, such as Fluo-3, Fluo-4, or monitoring intracellular pH using BCECF or BCPCF pr specific FLIPR assay kits, or detecting membrane potential changes using e.g. DiBAC or specific FLIPR assay kits, or monitoring of membrane polarization. For the monitoring of other intracellular ions, e.g. zinc or sodium, other dyes known in the state of the art can be used. Other types of assays and other types of read outs are commonly known to persons with skills in the art.
For the measurement of cAMP levels, e.g. AlphaScreen, fluorescence polarization or HTRF technology is suitable.

For the determination of ion channel activity (which control e.g. intracellular ion concentrations and can thus be employed for measurement of intracellular ion concentrations) e.g. membrane potential sensitive assays and dyes can be used such as DiBAC or Molecular Devices' membrane potential assay kit on FLIPR technology; mitochondrial membrane polarization measuring JC-1 dye with FLIPR technology; ion sensitive dyes such as Fluo-3, Fluo-4 or Molecular Devices calcium assay kit for intracellular calcium concentration measurement; sodium sensitive dye e.g. from Molecular Probes for measurement of intracellular sodium; assays based on patch-clamping or atomic adsorption spectroscopy-based Rubidium ion efflux measurement for determining of intracellular potassium concentrations, and so on. Further automatical devices and analytical methods for detecting certain changes and states within cells are known to the person of skill in the art and comprise, e.g. the Acumen detector (flureescence-based laser scanning reader that allows for 3dimensional reconstitution of distribution of suitably labeled objects) by ACUMEN bioscience.

The PAM polypeptide is preferably a polypeptide that comprises or consists of the sequence according to SEQ ID No 2 or is encoded by a polynucleotide comprising or consisting of the sequence according to SEQ ID No 1 or 3.

The PAM polynucleotide is preferably a polynucleotide comprising or consisting of the sequence according to SEQ ID No 1 or 3 or a polynucleotide comprising or consisting of a sequence that is able to hybridize with the above polynucleotides under stringent conditions.

Stringency describes reaction conditions that influence the specificity-of hybridisation or annealing of two single stranded nucleic acid molecules. Stringency, and thus specificity of a reaction depends, inter alia, of the temperature and buffer-conditions used for a reaction: Stringency, and thus specificity, can e.g. be increased by increasing the reaction temperature and/or lowering the ion strength of the reaction-buffer. Conditions of low stringence (and thus low reaction and hybridisation specificity) exist for example, if a hybridisation is performed at room temperature in 2xSSC-solution. Conditions of high stringency comprise e.g. a hybridisation reaction at 68°C in 0,1xSSC and 0,1% SDS solution.

Hybridisation under conditions of stringency within the different aspects of present invention is preferably understood to be:
1) Hybridising a labelled probe with a nucleic acid sample to be analysed at 65°C, or in the case of oligonucleotide probes, at 5°C below the annealing or melting temperature of the duplex consisting of oligonucleotide and sample (annealing and melting temperature are in the following understood to be synonyms) over night in 50mM Tris pH 7,5, 1 M Nacl, 1% SDS, 10% Dextran Sulfate, 0,5 mg/ml denatured salmon or hering sperm DNA.
2) Washing for 10 minutes in 2xSSC at room temperature.
3) Washing for 30 minutes in 1xSSC/0,1%SDS at 65°C (or in the case of oligonucleotides: 5°C below the annealing temperature).
4) Washing for 30 minutes in 0,1xSSC/0,1%SDS at 65°C (or in the case of oligonucleotides: 5°C below the annealing temperature).

Oligonucleotides for the use as hybridisation probes are polynucleotide and preferably DNA-fragments having a length of 15 to 30, preferably 20 nucleotides. The annealing temperature is determined according to the formula Tm=2x (number of A+T) + 4x (number of G+C)°C.

For preparing a 2xSSC or a 0,1xSSC (or any other kind of SSC dilution), e.g. a 20x SSC solution is diluted accordingly. 20xSSC consists of 3M NaCl/0,3 M Na-Citrate x2H₂O.

Before performing a hybridisation reaction, the polynucleotides are, if wanted after performing electrophoretic separation (then: Southern Blot (DNA) or Northern Blot (RNA)) or without electrophoretic separation (then: slot or dot Blot), transferred to a suitable membrane, e.g. a nylon or nitrocellulose membrane. Hybridisation is performed using a suitably labelled probe. Suitable labelling techniques are e.g. radioactive labelling or labelling using fluorescence dyes. The probe is a single stranded polyribo- or polydesoxyribonucleotide being single stranded naturally or being usually double stranded and having been made single stranded by denaturation. This probe binds to the DNA or RNA sample (which is also in single stranded state) by means of base pairing.

The PAM fragments are preferably fragments comprised within the above sequences ID No. 1, 2 or 3 and the derivatives are preferably derived from the above sequences ID No. 1, 2 or 3. or from fragments thereof.

The functional fragments thereof are preferably capable of inhibiting adenylyl cyclases (AC) activity, more preferably that of AC Type I, V or VI.

According to a preferred embodiment of the different aspects of present invention, the functional fragments thereof comprise or consist of amino acids 400 to 1400, preferably 446 to 1062, 499 to 1065 or 1028 to 1231, and more preferably 1000 to 1 300 and even more preferably 1000 to 1100 and even more preferably 1028 to 1065 of the human PAM sequence, preferably of the human PAM sequence according to SEQ ID No. 2, or if they are encoded by the respective polynucleotide fragments, especially if comprised within the sequences according to SEQ ID No.2 or 3.

If the functional fragments thereof are polynucleotides, it is preferred, if they comprise or consist of polynucleotides encoding the above polypeptide fragments. More specifically, it is preferred if they comprise or consist of positions 1482 to 3332 (encoding amino acids 446 to 1062) or 1641 to 3341 (encoding amino acids 498 to 1066) or 3228 to 3839 (encoding amino acids 1038 to 1231) of the human PAM cds. It is even more preferred, if the human PAM cds from which the fragments are derived has the sequence according to SEQ ID No.2.

According to one preferred embodiment of present method for identifiying pain modulating compounds, a cell expressing, PAM, preferably recombinant PAM is used. The cell can be any type of cell, e.g. a eucaryotic or prokaryotic single cell organism (such as bacteria, e.g. e. coli, or yeast, e.g. s. pombe or s. cerevisiae) or cell lines derived from multicellular organisms (such as HeLa, COS, NIH-3T3, CHO, etc), wherein mammalian cell lines are preferred.

According to another preferred embodiment, a modified cell, having a lower PAM activity as compared to its unmodified state, is used. This way, it can be tested, if the chemical compounds to be tested for their ability of modulating (preferably diminishing) and / or preventing pain, are able to enhance or restore the lowered or totally abolished PAM activity.

The modification can be any type of modification (stable or transient, preferably stable), that leads to a decrease of PAM activity (i.e. its ability to lower intracellular cAMP levels, to interact with other factors like AC, especially with AC, to inhibit AC or its ability to lower the pain perception), PAM transcript steady state level (i.e. by activation of PAM transcription or transcript stabilisation) or PAM protein steady state level (i.e. by activation of PAM translation or its posttranslational processing; by modulation of PAM posttranslational modification or by activation of its stabilisation or by inhibition of its degradation). This can for example be achieved by using dominant negative mutants of PAM, antisense oligonucleotides, RNAi constructs of PAM, by generating functional or genomic PAM knock outs (which can e.g. be inducible) or other suitable techniques known within the state of the art. For an overview of the above techniques, see for example: Current protocols in Molecular biology (2000) J.G. Seidman, Chapter 23, Supplemtent 52, John Wiley and Sons, Inc.; Gene Targeting: a practical approach (1995), Editor: A.L. Joyner, IRL Press; Genetic Manipulation of Receptor Expression and Function, 2000; Antisense Therapeutics, 1996; Scherr et al, 2003.

According to a preferred embodiment, a PAM knock-out-cell is used. Suitable cell lines for the generation of knock-outs are well known in the state of the art and comprise e.g Current protocols in Molecular Biology (2000) J.G. Seidman, Chapter 23, Supplement 52, John Wiley and Sons, Inc; or Gene Targeting a practical approach. (1995) Ed. A.L. Joyner, IRL Press.

The PAM activity can either be determined directly, e.g. by its ability (or the ability of its fragments and derivatives) to interact with AC or inactivate AC, or it can be determined indirectly, e.g. by its ability (or the ability of its fragments and derivatives) to lower intracellular cAMP levels, to modulate ion concentrations within the neurons or its ability to modulate, especially decrease pain perception. Suitable techniques for measuring the above parameters are well known in the state of the art (see also above): The cAMP levels can e.g. be measured by HTRF or ALPHAscreen^{™}, the ion concentrations can e.g. be estimated by patch clamping or suitable dyes, the pain perception can e.g. be measured by means of the formalin test or tests of mechanical or thermal hyperalgesia, or the hot plate test etc.

Another aspect described herein concerns a method of identifying a compound that modulates pain comprising
a) Selecting a compound that modulates the activity of PAM as a test compound, and
b) Administering said test compound to a subject to determine whether the pain is modulated.

The subject can be any subject with the ability of perceiving pain, preferably it is a mammal, either a non-human mammal or a human (i.e. within a patient study).

In the following, the invention is illustrated in more detail by means of examples and figures. However, the examples are not meant to limit the scope of the invention.

### Examples: Investigation of PAM expression pattern and function

### 1. Preparation of animal sections:

Wild type Sprague Dawley rats were purchased from Charles River Wiga GmbH (Sulzfeld, Germany). The animals had free access to food and water prior to the experiments. They were maintained in climate- and light-controlled rooms (24 + 0.5°C). Each animal was used at one occasion only. In all experiments the ethics guidelines for investigations in conscious animals were obeyed, and the procedures were approved by the local Ethics Committee. After killing, adult rats were fixed by perfusion with 4% paraformaldehyde in 0.1 M phosphate buffered saline (PBS, pH 7.2) for one hour. Tissues were cryostat-sectioned in the horizontal plane at a thickness of 14-16 µm. Sections were mounted on Superfrost Plus Slides (Fisher Scientific Co., Pittsburgh, PA) and stored at -80°C until use.

### 2. Preparation of riboprobes:

The riboprobes were generated as described previously (Yang et al., 2002). Antisense and sense riboprobes of rat PAM were obtained with T7 and T3 polymerases, after linearizing the plasmid with Hind III (antisense) and BamHl (sense), respectively (see Yang et al., 2002). In vitro transcription was performed in the presence of [35S] UTP-αS (ICN, Irvine CA), linearized PAM cDNA, NTP at 37°C for 1 hour according to the manufacturer's recommendation (Promega, Madison, WI). The RNA transcripts were purified using RNA Probe Purification Kit (Pequlab, Erlangen, Germany).

### 3. In situ hybridization:

In situ hybridization was performed as described earlier (Yang et al. 2002): Sections were fixed in 4% paraformadehyde in 0.1 M phosphate-buffered saline (pH 7.2), pretreated with 0.25% acetic anhydride and 0.1 M triethanolamine, rinsed with 0.2x SSC and dehydrated with serially increasing concentrations of alcohol. Sections were prehybridized with prehybridization solution (50% deionized formamide, 0.6 M sodium chloride, 10 mM Tris-HCl (pH 7.6), 50 mM EDTA, 0.025% sodium pyrophosphate, 0.02% Ficoll, 0.02% BSA, 0.02% polyvinyl pyrrolidone, 10 mM DTT, and heat-denatured, heterologous nucleic acids (0.005% yeast tRNA, type X, 0.05% yeast total RNA, type I, 0.05% salmon-testes DNA, type III)) for 2 hours at room temperature; hybridized with riboprobes in hybridization solution (2.5x106 cpm/section), 50% deionized formamide and 50% hybridization buffer containing 0.6 M sodium chloride, 10 mM Tris-HCl (pH 7.6), 50 mM EDTA, 0.025% sodium pyrophosphate, 0.02% Ficoll, 0.02 % BSA, 0.02% polyvinyl pyrrolidone, heat-denatured, heterologous nucleic acids (0.005% yeast tRNA, type X, 0.005% yeast total RNA, type I, 0.05% salmon testes DNA, type III), 100 mM DTT, 0.0005% polyadenylic acid, 10% dextran sulfate) at 50°C overnight. Sections were rinsed with 2x, 1x, 0.5x SSC at RT (room temperature). After digestion in 20 µg/ml RNase A (Sigma, St. Louis, MO), sections were washed in 1x RNase buffer, 2x, 1×, 0.5x SSC at room temperature, and in 0.1× SSC overnight at 45°C. Sections were dehydrated with serially increasing concentrations of alcohol, exposed to Kodak Biomax MR film (Kodak, Rochester, NY) for 3-7 days at -80°C.

### 4. Antibody generation and Immunofluorescence staining:

Antisera were raised commercially in rabbits using peptides consisting of amino acid residues 135-153 and 4601-4614 of human PAM corresponding to the SEQ ID No.1, respectively (BioTrend, Cologne, Germany). The antiserum was commercially produced by BioTrend, Cologne, Germany, according to standard procedures. To monitor the distribution of PAM in spinal cord and DRG slices, the slices were permeablized in 0.1 % Triton X-100 for 5 minutes. The slices were -blocked for 1 hour in 3% BSA in PBS and then incubated for 1 hour with anti-PAM antiserum (1:50 dilution). This was followed by incubation with FITC-labeled goat anti-rabbit antibody in PBS containing 3% BSA. The slices were then washed with PBS and mounted using fluoromount^{™}.

### 5. RT-PCR:

Total RNA from rat spinal cords and DRGs was isolated by guanidinium isothiocyanate/phenol/chloroform extraction (Chomczynski and Sacchi 1987). 2 µg of total RNA were annealed with 0.6 µM of each of oligo (dT) primer and reverse-transcribed using reverse transcriptase (Promega, Madison, WI) for 30 minutes at 37 °C. The cDNA was then immediately used for amplification. Oligonucleotide primers used for the amplification of rat GADPH were 5'-GAAGGGTGGGGCCAAAAG-3' (sense; SEQ ID No.10) and 5'-GGATGCAGGGATGATGTTCT-3' (antisense; SEQ ID No.11; Trajkovic et al. 2000). Primers for the amplification of AC isoforms were chosen as published by Bek et al. (Bek et al. 2001). Primers for rat PAM were 5'-GGTGGTGAAGCTCGCTGTGATGCT-3' (sense; SEQ ID No.12) and 5'-CGTGTGAGCATTTCTGCACAC TCC-3' (antisense; SEQ ID No.13). The PCR product corresponds to the human PAM cDNA nucleotides 13692-14064. The corresponding rat sequence was derived from the EST clone AW441131 (SEQ ID No.8). For semiquantitative PCR, SAWDAY DNA Polymerase (Peqlab, Erlangen, Germany) was used. After an initial denaturation step at 95 °C for 5 minutes, 30 cycles were performed with 1 minute at 95°C, 30 seconds at 55°C, and 10 seconds at 72°C, followed by a final 10-minute extension step at 72°C. Quantitative PCR was performed using the TaqMan^{™} system and reagents (Applied Biosystems, Weiterstadt, Germany) according to the instructions of the manufacturer.

### 6. Purification of full length PAM:

PAM purification was performed with some modifications as published previously (Scholich et al. 2001). Shortly, HeLa cells were grown in DMEM medium with 10% fetal bovine serum and 1% penicillin/streptomycin. Confluent cells of forty 150 mm dishes were harvested with 1x PBS, 1 mM EDTA and pelleted for 5 minutes at 400 x g. The cells were resuspended in TED buffer (50 mM Tris-HCl, pH 8.0; 1 mM EDTA, 1 mM DTT) containing 125 mM NaCl, 20 µg/ml of aprotinin, 20 µg/ml leupeptin, 1 mM benzamidine, 5 µg/ml soybean trypsin inhibitor and lysed by 2x 5 seconds of sonication. The homogenate was centrifuged at 27000 x g for 30 min at 4 °C, and the supernatant was loaded on a Q-Sepharose XK16 column (Amersham, Pharmacia, Piscataway, NJ) and eluted with a gradient of 150-350 mM NaCl in TED according to instructions of the manufacturer. The fractions were analyzed by Western blotting according to standard procedures; positive fractions were pooled and the NaCl concentration adjusted to 1 M. The protein was then loaded on a Phenyl-Sepharose XK16 column (Amersham, Pharmacia, Piscataway, NJ) and washed with 300 mM NaCl in TED according to instructions of the manufacturer. The flow through and wash fractions contained PAM. They were pooled and the buffer exchanged for the aforementioned TED buffer containing 100 mM NaCl using Centricon 50 (Amicon, Beverly, MA) according to the manufacturer's instructions. The protein was then loaded on a MonoS 5/5 FPLC column (Amersham, Pharmacia, Piscataway, NJ) and washed with the loading buffer (100 mM NaCl in TED) according to instructions of the manufacturer. The flow through was collected and applied to a Mono Q 5/5 FPLC column (Amersham, Pharmacia, Piscataway, NJ). The protein was eluted with a gradient from 150-400 mM NaCl in TED. Positive fractions were pooled and the buffer exchanged for 50 mM Tris-HCl, pH 8.0, 1 mM DTT using Centricon 50 (Amicon, Beverly, MA) and stored at -80 °C. The stored PAM was used within 3 weeks.

### 7. Expression and purification of recombinant Gsα:

The hexahistidyl tagged constitutively active Q213L mutant of Gsα (Gsα*) was expressed and purified as described in Graziano et al., 1991. To ensure maximal activation of the Gsα*, the G protein was incubated with 1 µM GTPγS in the presence of MgCl2 (25 mM) for 30 minutes prior to use in AC activity assays.

### 8. Adenylyl Cyclase Activity Assays (AC activity assays):

Spinal cords were lysed in 25 mM Hepes, pH 7.4, 1 mM EGTA and cell membranes were prepared as described by Kassis and Fishman. Aliquots were stored at -80°C until use. AC activity assays were performed in a volume of 100 µl for 15 min at room temperature in the presence of 100 µM MgCl₂ as previously described (Patel et al., 2002). Gsα* (80 nM) or forskolin (100 µM) were used to stimulate AC enzyme activity in membranes (10 µg protein).

### 9. Spinal delivery of PAM antisense and sense oligonucleotides:

Rats were anesthetized with ketamine (60 mg/kg i.p.) and midazolam (0.5-1 mg/kg i.p.). The skin was incised above the vertebral column from vertebrae Th13 up to L3. Muscle tissue around L2-3 was cleared away. The processus spinosus of L3 was removed and a laminectomy was done at L2. Polyethylene catheters (ID 0.28 mm, OD 0.61 mm) were then inserted into the peridural space so that the tip of the catheter reached Th9-10. The catheter was fixed with cyanacrylate glue, externalized in the neck region, and the skin was sutured.

### 10. Infusion of PAM oligonucleotides:

The sequences of the oligodeoxynucleotides (ODNs) were chosen from the rat PAM sequence as follows. Sense: 5'-GACTGGTTTAGCAATGGC-3'(SEQ ID No.14), antisense: 5'-GCCATTGCTAAACCAGTC-3' (SEQ ID No.15), and antisense ODN harboring three mutations (3M-as; mutations are underlined): 5'- GCAATTGCTAAATCAGTA -3' (SEQ ID No.16). Three days after surgery, rats were placed into a "freely moving system" (CMA, Stockholm, Sweden) and antisense (n=5) or sense (n=5) oligonucleotides (2.5 mg/ml in artificial cerebrospinal fluid) were infused through the catheter at a flow rate of 0.05-0.1 µl/min for 100 hours using a microinfusion pump (CMA, Stockholm, Sweden).

### 11. Formalin test:

Within 15 min after stopping the infusion, the formalin test was performed. 50 µl of a 5% formaldehyde solution was injected subcutaneously (s.c.) into the dorsal surface of one hind paw. Flinches were counted in one-minute intervals up to 60 min starting right after formalin injection. Flinches of 5 min intervals were summarized as mean flinches per minute. To compare the nociceptive behavior between groups, the sum of flinches during the two phases of the one-hour observation period were submitted to the Students t-test. α was set at 0.05.

At the end of the formalin test, the rats were killed, the lumbar spinal cord and dorsal root ganglia (DRGs) were excised, snap frozen in liquid nitrogen and stored at -80°C until further analysis. To determine PAM expression, spinal cord slices were analyzed immunhistochemically using the above anti PAM antibodies.

### 12. Zymosan-evoked inflammation:

For induction of an inflammation, 2.5 mg zymosan A (Sigma, St. Louis, MO) suspended in 30µl 0.03 M phosphate buffered saline (PBS, pH 7.5) was injected subcutaneously into the midplantar region of the right hindpaw. Such intraplantar zymosan injection is known to induce a reliable model of thermal and mechanical hyperalgesia rats (Meller and Gebhart 1997). Rats were killed by cardiac puncture under deep isoflurane anesthesia 24-96 hours after the Zymosan injection. The lumbar spinal cord and dorsal root ganglia (DRGs) were excised, snap frozen in liquid nitrogen and stored at -80°C until further analysis.

### 13 Results:

The above experiments of the inventors using RT-PCR, immunhistochemistry and in situ hybridisation, demonstrate for the first time that PAM is expressed in sensory neurons of the spinal cord as well as in dorsal root ganglia (DRGs) of adult rats. PAM mRNA was detected by RT-PCR at similar levels in the spinal cord and dorsal root ganglia throughout development (E14-adult). PAM-expression is up-regulated 24-48 hours after zymosan treatment of rats as shown by western blot and RT-PCR.

The major adenylyl cyclase isoforms, which are expressed in the spinal cord and DRGs, are AC type 5 and 6 and AC type 4 and 6, respectively. No major changes in AC isoform expression were observed after zymosan treatment in spinal cord. Hence, Gαs stimulated AC activity in membrane preparations from spinal cord and DRG was inhibited by PAM. Consequently, it was found that treatment with antisense but not sense oligonucleotides against PAM increased formalin induced paw flinching in adult rats. Accordingly cAMP accumulation in the spinal cord of rats treated with antisense oligonucleotides to PAM was elevated as compared to control rats.

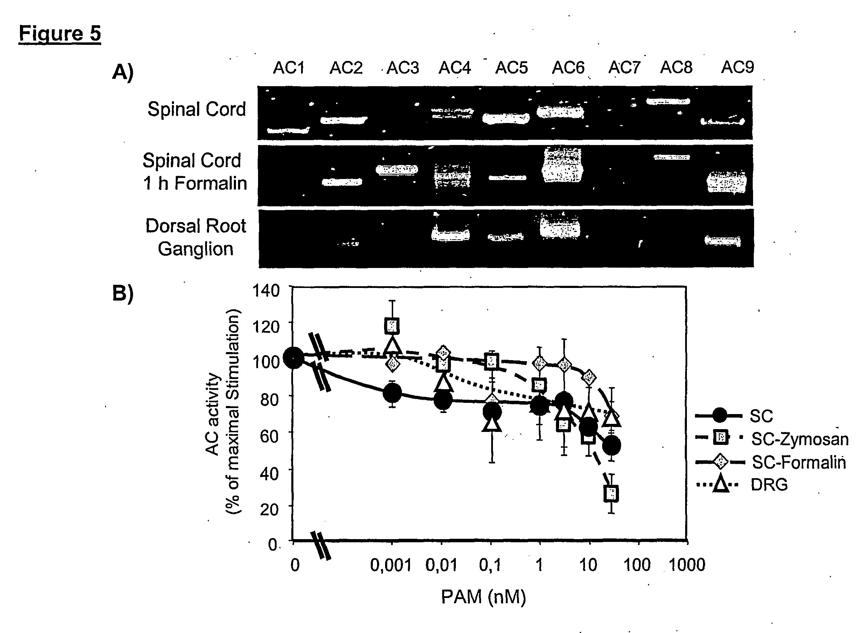

Addition of purified PAM to spinal cord lysates resulted in an inhibition of Gas-stimulated AC activity of spinal cord lysates from control and zymosan treated animals (Fig. 5b). At 30 nM Gas-stimulated AC activity was decreased by 50% in spinal cord lysates of control animals and 70% in lysates derived from rats treated with zymosan for 96 hours.

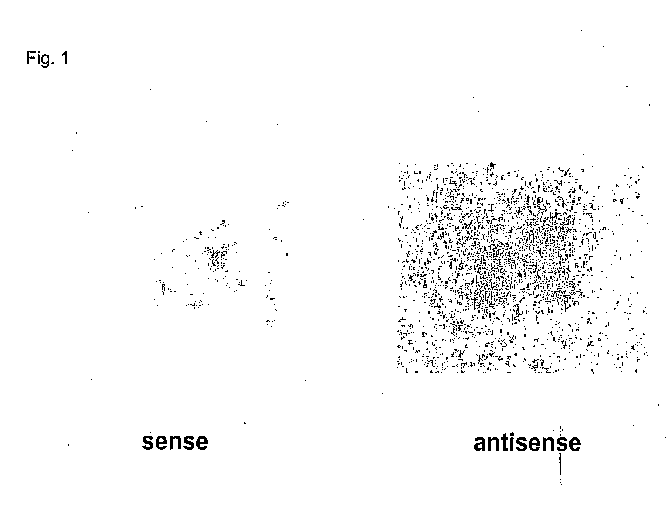

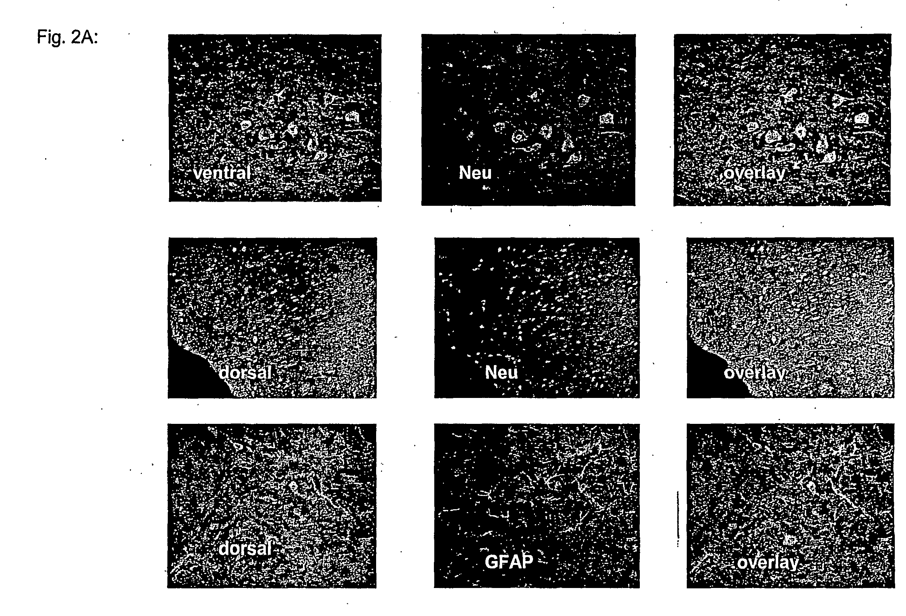

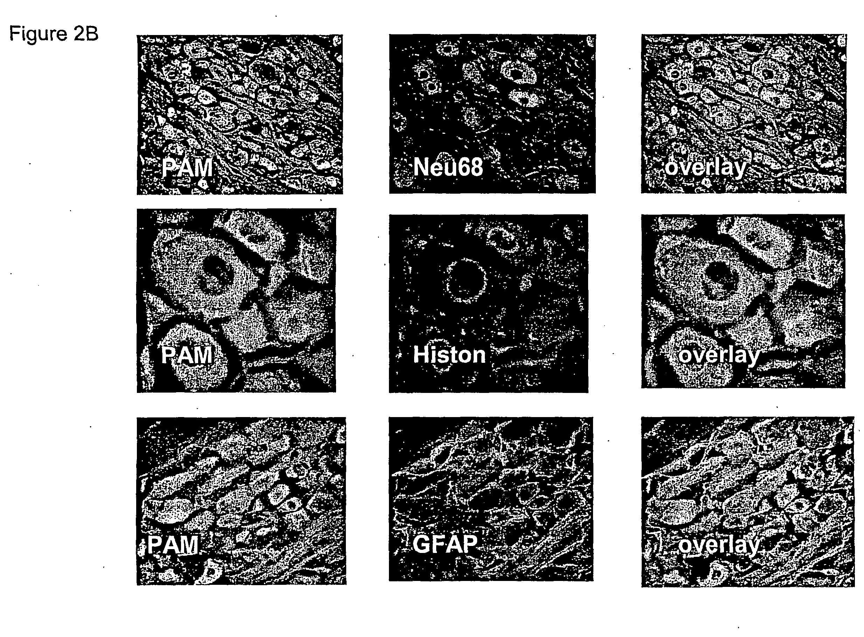

To determine if PAM is expressed in the spinal cord, first in situ hybridization was performed. This led to the detection of a clear signal for PAM mRNA throughout the gray matter but not in the white matter of the spinal cord of adult rats (Fig. 1). To define more precisely the cell populations that express PAM in the spinal cord as well as in DRGs, antibodies against PAM using peptides corresponding to the amino acid residues 135-153 and 4601-4614 of human PAM were generated. The immunhistochemical analysis revealed that PAM was co-localized with anti-NeuN but not with anti-GFAP immunoreactivity (Fig. 2a): More specifically, PAM expression was detected predominantly in dorsal horn neurons (Fig. 2a) while non-neuronal cell populations exhibited very little PAM expression. Especially high PAM expression could be detected in DRG neurons (Fig. 2b). Here, PAM immunoreactivity was located in the axons as well as in the cell body of both large- and small-diameter neurons (Fig. 2b). Interestingly, no PAM was detected in the nuclei of the cells as demonstrated by co-staining with anti-Histone antibody (Fig. 2b). As could also be seen in the spinal cord, PAM expression was not detected in GFAP expressing cells.

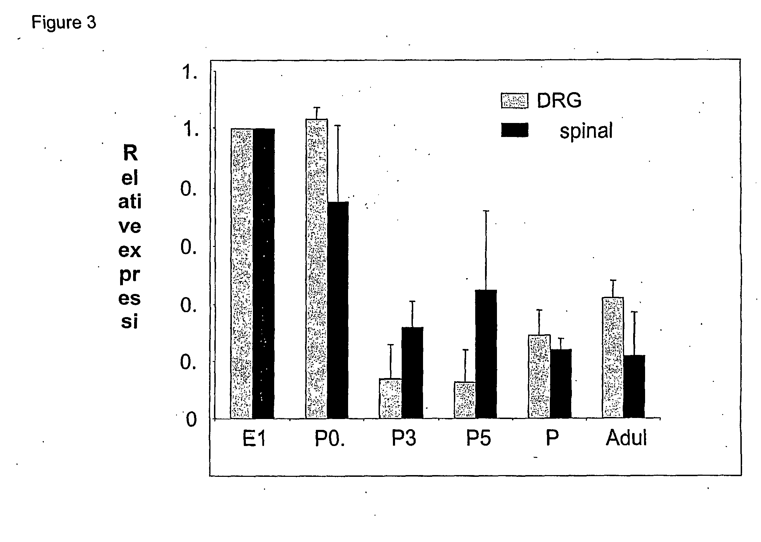

Since PAM expression in the brain is differentially regulated during-development in rats and mice (Yang et al. 2002), the inventors investigated if PAM mRNA expression also changes during development in rat spinal cord and DRG. To this end, PAM mRNA expression was determined using quantitative RT-PCR. PAM mRNA was found to be highly expressed in the spinal cord and DRG in late embryonic stages (E16) until shortly after birth (P0.5; Fig. 3).
Interestingly, shortly after birth the expression declined to 30-40% of the embryonic expression and then remained constant throughout adulthood (Fig. 3).

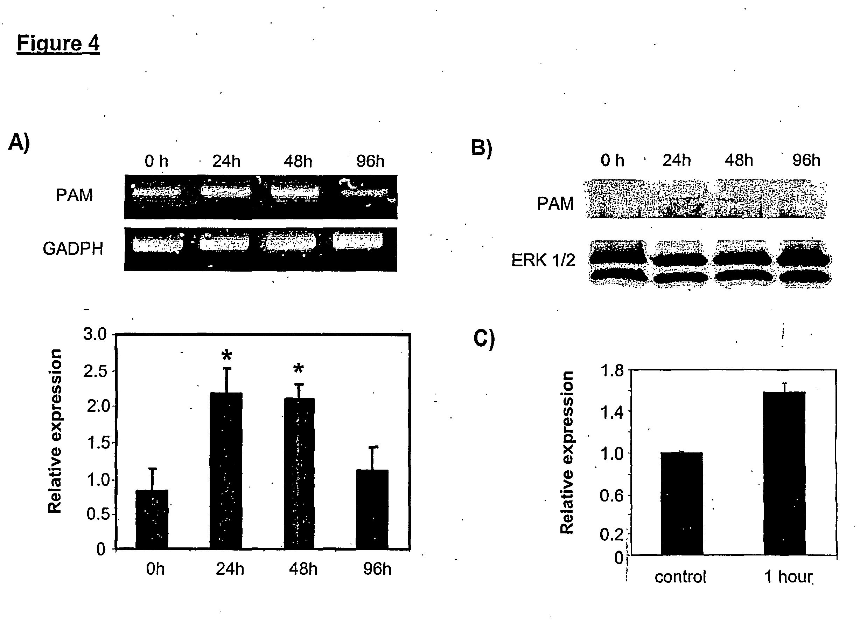

Next, it was examined if PAM expression in the spinal cord is regulated by nociceptive stimuli. Therefore, spinal PAM expression was monitored after zymosan and formalin injection in the hind paws of adult rats. PAM mRNA was up-regulated about two-fold at 24 and 48 hours after zymosan treatment (Fig. 4a). Accordingly, PAM expression was up-regulated at the protein level 24 hours after zymosan injection and stayed elevated for 96 hours (Fig. 4b). 96 hours after zymosan injection, PAM mRNA expression declined. This reduction in PAM mRNA expression was not reflected at the protein level (comp. Fig. 4a and b). Notably, PAM mRNA was also upregulated in the spinal cord of rats 1 hour after formalin injection (Fig. 4c).

Since PAM is known to be a powerful inhibitor of adenylyl cyclases type 1, 5 and 6 (Scholich et al. 2001) it was investigated if PAM is able to inhibit AC activity in spinal cord and DRG lysates. Two major AC isoforms are detected by semi-quantitative RT-PCR in the spinal cord. These AC isoforms are type 5 and 6 (Fig. 5a). Notably, both isoforms are inhibited at nanomolar concentrations of PAM (Scholich et al. 2001). The AC isoform expression pattern in the spinal cord was not significantly altered 24-96 hours after zymosan injection. Interestingly, 1 hour after formalin injection a shift in AC isoform expression was detected (Fig. 5a). The mRNA of AC type 5 is down-regulated and the mRNA of AC type 3 and 9 are up-regulated.

As could be shown by the above experiments, addition of purified PAM to spinal cord lysates resulted in inhibition of Gas-stimulated AC activity in spinal cord preparations. Addition of 30 nM PAM decreased Gas-stimulated AC activity by 49% in spinal cord lysates of control animals (Fig. 5b). Gas-stimulated AC activity in spinal cord lysates derived from rats 96 hours after zymosan injection exhibited higher sensitivity to PAM inhibition as compared to untreated animals (70% inhibition at 30 nM; Fig 5b). In contrast, inhibition of AC activity by PAM in spinal cord lysates from animals treated for 1 hour with formalin was inhibited to a lesser extent (25% inhibition at 30 nM; Fig. 5b).

In DRGs the predominantly expressed AC isoforms are AC type 4 and 6 (Fig. 5a). Gas-stimulated AC activity in DRG lysates was decreased by 32% in presence of 30 nM PAM in contrast to spinal cord lysates (Fig. 5b).

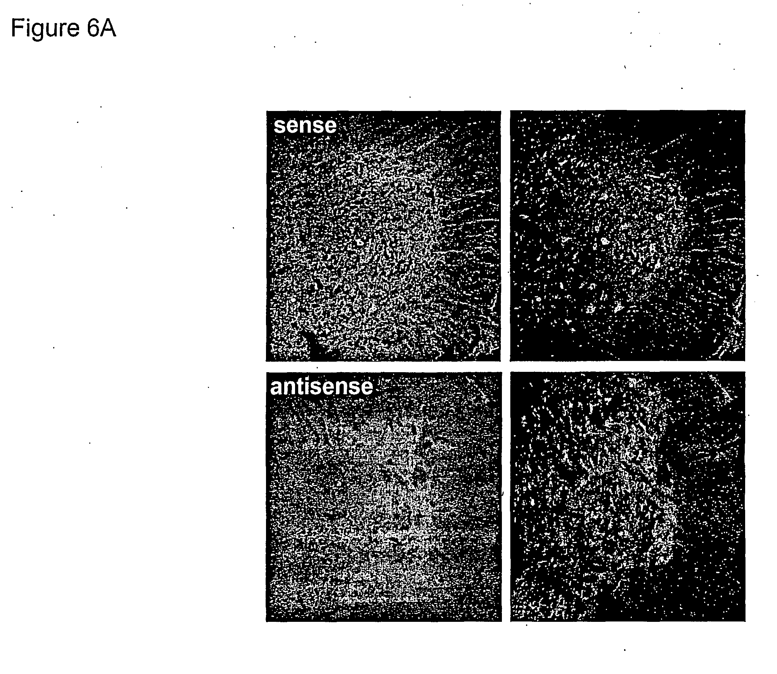

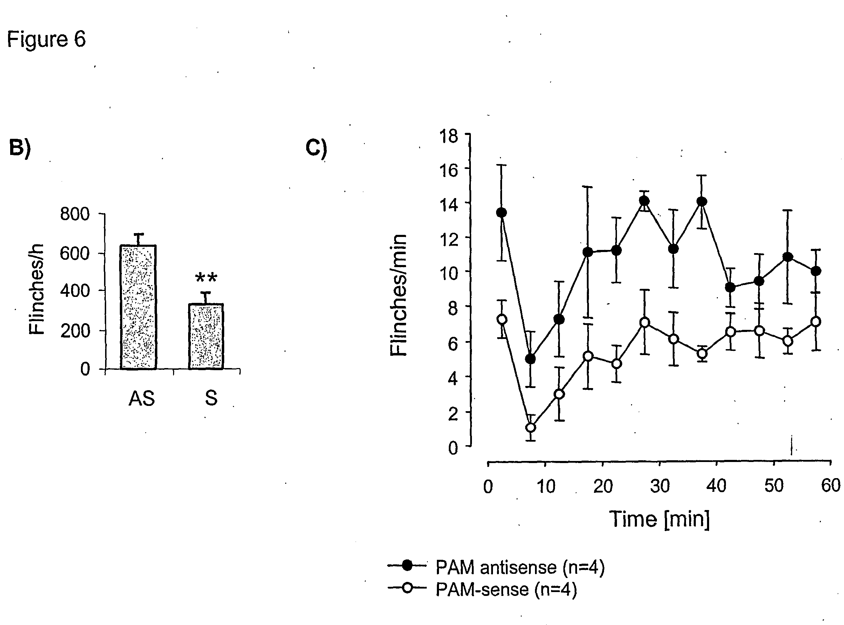

To examine a possible role of PAM in spinal nociceptive transmission, animals were infused with PAM sense and antisense oligonucleotides by lumbar intrathecal catheters before performing a formalin assay. PAM expression was decreased in spinal cord neurons as observed by immunhistochemistry (Fig. 6a). Infusion of PAM antisense oligonucleotides caused a significant increase of the nociceptive response following formalin injection as compared to PAM sense treatment (p=0.007; Fig. 6b and c). The hyperalgesia in PAM antisense-treated rats was accompanied by increased licking and biting behavior. Since PAM is an inhibitor of AC activity (Scholich et al. 2001), basal, Gas- and forskolin-stimulated AC activities were determined in spinal cord lysates of sense and antisense-ODN treated rats. The experiments showed a significant increase (20.7 %) of the basal AC activity in antisense treated rats (Table 1). In contrast, no-significant changes were detected in Gas- and forskolin-stimulated AC activities indicating that the total amount of AC was not altered by the ODN treatment (Table 1).

The above experiments showed that PAM is localized in the cell body and axons of both spinal cord and DRG neurons (Fig. 2a,b). Only very little immunreactivity was detectable in the nucleus suggesting different functions for PAM in neurons and cancer cell lines.

Central sensitization after prolonged nociceptive stimuli is based on neuronal and synaptic changes in the spinal cord (Woolf and Costigan 1999; Woolf and Salter 2000; Ji and Woolf 2001). The finding of the inventors that Pam is expressed in sensory neurons of the spinal cord and DRGs led to the newly formed hypothesis, that PAM could be implicated in synaptic changes during spinal nociceptive processing. The above findings that PAM was up-regulated after nociceptive stimuli (Fig. 4a) supported this hypothesis that PAM may play a role in synaptic changes during spinal nociceptive processing.

Furthermore, the surprising finding of the inventors, that PAM is expressed in sensory nervous of the spinal cord and DRGs led to the question whether PAM is capable of inhibiting AC activity in spinal cord and DRG, as well.

The above experiments showed for the first time that PAM is a potent inhibitor of Gas-stimulated AC activity in spinal cord preparations (Fig. 5b). AC activity was decreased by 50 % after addition of 30 nM PAM. To achieve comparable inhibition using the α-subunit of the inhibitory G-protein, Gai, 200-800 nM Gαi has to be used (Wittpoth et al. 1999). The inhibitory action of PAM was even stronger in spinal cord preparations of animals treated for 96 hours with zymosan (Fig. 5b) and could be explained by the elevated amounts of endogenous PAM in the spinal cord after zymosan injection (Fig.4a,b). Inhibition of Gαs-stimulated AC activity in spinal cord preparations from formalin-treated animals (25% inhibition) was less pronounced as compared to control or zymosan-treated animals (50% and 75%, respectively).

Notably, in animals treated with formalin for 1 hour a shift in AC isoform expression was observed (Fig.5a). AC of type 3 and 9 are up-regulated while AC type 5 is down-regulated. To date it is not known if PAM is an inhibitor of AC type 3 and 9. Therefore, these isoforms may not be inhibited by PAM or the tested PAM concentrations were too low to achieve an inhibitory effect. Since PAM is a giant protein of 510 kDa, it is technically not possible to test PAM concentrations greater than 30 nM. Nonetheless, according to the dose responses curves shown in Figure 5b, higher PAM concentrations might result in a stronger inhibition of Gαs-stimulated AC activity in the tested spinal cord preparations.

Interestingly PAM was a less effective inhibitor of AC enzyme activity in DRG than in spinal cord preparations. The different inhibitory efficiencies of PAM in spinal cord and DRG preparations are most likely due to the observed differences in AC isoform expression. The major AC isoforms that are expressed in the spinal cord are type 5 and 6 that are both strongly inhibited by PAM (Fig. 5a; (Scholich et al. 2001)). In DRGs AC type 4 and 6 are the dominant AC isoforms (Fig. 5a). Since it is unknown if PAM inhibits AC type 4 either this isoform is not inhibited by PAM or, again, the tested PAM concentrations were too low to achieve the inhibitory effect. However, according to the dose response curve shown in Figure 5b it is seems likely that higher concentrations of PAM would result in a stronger inhibition of Gαs-stimulated AC activity in the DRG preparations.

Most surprising, however, were the findings, that PAM activity had an influence on the nociceptive behavior of the test animals: This could be demonstrated for the first time by experiments of the inventors showing a significant increase in basal AC activity (Table 1) and - more important - a significant increase of the nociceptive response following formalin injection as compared to PAM sense treatment (Fig. 6b and c) when endogenous PAM expression in the spinal cord was decreased by infusing animals with PAM antisense oligonucleotides (Fig. 6a).

### 14. determination of the analgesic effect of PAM

The above-listed evidence for the analgesic effect of PAM could for example be supported by the following hypothetic experiment: The analgesic effect of PAM, e.g. in the formalin model of acute pain, could be determined directly by intrathecal application of e.g. a peptide corresponding to amino acid residues 1028 to 1065. This peptide represents the minimal region found to be capable of mediating PAM-adenylyl cyclase interactions as determined by the yeast-two-hybrid system and AC activity assays. The peptide could be applied in a complex with the bioporter lipofection reagent (commercially available at Peqlab, Germany). This approach would allow the peptide to enter the tissue and mimic the actions of physiological PAM towards ACs.

### Literature

Bailey C.H., Bartsch D., Kandel E.R.: Toward a molecular definition of long-term memory storage. Proc. Natl. Acad. Sci. U.S.A. 1996 Nov 26;93(24):13445-52;
Brandon E.P., Idzerda R.L., McKnight G.S.: PKA isoforms, neural pathways, and behaviour: making the connection. Curr. Opin. Neurobiol. 1997 Jun;7(3):397-403;
Bek M. J., Zheng S., Xu J., Yamaguchi I., Asico L. D., Sun X. G. and Jose P. A. (2001) Differential expression of adenylyl cyclases in the rat nephron. Kidney Int 60, 890-899;
Chang Q. and Balice-Gordon R. J. (2000) Highwire, rpm-1, and futsch: balancing synaptic growth and stability. Neuron 26, 287.-290;
Chomczynski, P., Sacchi, N.: Single-step method of RNA isolation by acid guanidinium thiocyanate-phenol-chloroform extraction. Anal. Biochem. 162, (1987) 156-159;
Graziano, M.P. FreissmuthM. Gilman A.G.: Purification of recombinant Gs alpha. Meth. Enz. (1991) 195: 192-215;
Graziano M. P., Freissmuth M. and Gilman A. G. (1991) Purification of recombinant Gs alpha., Methods Enzymol 195, 192-202;
Guo Q., Xie J., Dang C.V., Liu E.T., Bishop J.M.: Identification of a large Myc-binding protein that contains RCC1-like repeats. Proc. Natl. Acad. Sci. U.S.A. 1998 Aug 4;95(16):9172-7),
Jin Y. (2002) Synaptogenesis: insights from worm and fly. Curr Opin Neurobiol 12, 71-79.);
Julius and Basbaum " Molecular mechanisms of nociception", Nature, volume 413, 13. September 2001, pp. 203 - 209;
Kassis, S., and Fishman, P. H. (1982) J Biol Chem 257(9),5312-5318;
Meller S.T., Gebhart G.F. (1997), intraplantar zymosan as a reliable, quantifiable model of thermal and mechanical hyperalgesia in the rat; Eur. J. Pain 1, 43-52;
Nair, B. G., Parikh, B., Milligan, G., and Patel, T. B. (1990) J Biol Chem 265(34), 21317-21322;
Patel T. B., Wittpoth C., Barbier A. J., Yigzaw Y. and Scholich K. (2002) Functional analyses of type V adenylyl cyclase. Methods Enzymol 345, 160-187.
Snyder S. H. (1985) Adenosine as a neuromodulator. Annu Rev Neurosci 8,103-124.
Ruppert C., Goldowitz D., and Wille W. (1986), Proto-oncogene-c-mycis expressed in cerebellar neurons at different developmental stages, Embo J5, 1897-1901;
Schaefer A. M., Hadwiger G. D. and Nonet M. L. (2000) rpm-1, a conserved neuronal gene that regulates targeting and synaptogenesis in C. elegans. Neuron 26, 345-356);
Schaible H.G., Vanegas H.: How do we manage chronic pain? Baillieres Best. Pract. Res. Clin. Rheumatol. 2000 Dec;14(4):797-811;
Scherr M., Morgan M.A., Eder M., Gene Silencing Mediated by Small Interfering RNAs in Mammalian Cells, Curr Med Chem. 2003, Feb; 10 (3): 245-256;
Scholich K., Pierre S., Patel T.B.: Protein associated with myc (PAM) is a potent inhibitor of adenylyl cyclase. J. Biol. Chem. 2001, Dec 14;276(50):47583-9;
Scholz and Woolf "Can we conquer pain" , Nature neuroscience supplement, volume 5, November 2002, pp. 1062 -1067;
Snyder S.H. (1985), Adenosine as a neuromodulator. Annual Reviews of Neuroscience 8, 103-104;
Trajkovic V, Samardzic T, Stosic-Grujicic S, Ramic Z, Mostarica Stojkovic M. Muramyl dipeptide potentiates cytokine-induced activation of inducible nitric oxide synthase in rat astrocytes. Brain Res. 2000 Nov 10;883(1):157-63;
Wan H. I., DiAntonio A., Fetter R. D., Bergstrom K., Strauss R. and Goodman C. S. (2000) Highwire regulates synaptic growth in Drosophila. Neuron 26, 313-329.)
Wittpoth C., Scholich K., Yigzaw Y., Stringfield T.M. and Patel T.B. (1999), Regions on adenylyl cyclase that are necessary for inhibition of activity by beta gamma G(iα) subunits of heterotrimeric G proteins. Proc. Natl. Acad. Sci. USA 96, 7723-7730;
Wood, J.D. "Pathobiology of Visceral Pain: Molecular Mechanisms and Therapeutic Implications II. genetic approaches to pain therapy" , American Journal pf Physiological Gastrointestinal Liver Physiology, 2000, volume 278, G507-G512; Woolf and Mannion "Neuropathic pain: aetiology, symptoms mechanisms, and management", The LANCET, volume 353, June 5, 1999, pp. 1959 -1964;
Woolf J. and Salter M.W. "Neuronal Plasticity: Increasing the Gain in Pain", Science, volume 288, June 9, 2000, pp. 1765-1768;
Xia Z., Storm D.R.: Calmodulin-regulated adenylyl cyclases and neuromodulation. Curr. Opin. Neurobiol. 1997 Jun;7(3):391-6;
Yang H., Scholich K., Poser S., Storm D., Patel T.B., Goldowitz D.: Developmental expression of protein associated with myc (PAM) in the rodent brain. Brain Res Dev Brain Res 136, 2002, 35-42;
Zhen M., Huang X., Bamber B. and Jin Y. (2000) Regulation of presynaptic terminal organization by C. elegans RPM-1, a putative guanine nucleotide exchanger with a RING-H2 finger domain. Neuron 26, 331-343);

The literature listed above is incorporated herein by reference.

### Standard Literature for Laboratory Methods:

If not indicated otherwise, laboratory methods were or can be performed according to standard methods listed in the below standard literature:
Sambrook et al. (1989) Molecular Cloning: A Laboratory Manual. Second edition. Cold Spring Harbor Laboratory Press, Cold Spring Harbor, NY. 545 pp or in Molecular Biology;
Current Protocols in Molecular Biology; ; regularly updated, e.g. Volume 2000; John Wiley & Sons, Inc; Editors: Fred M. Ausubel, Roger Brent, Robert Eg. Kingston, David D. Moore, J.G. Seidman, John A. Smith, Kevin Struhl.
Current Protocols in Human Genetics; regularly uptdated, e.g. Volume 2003; John Wiley & Sons, Inc; Editors: Nicholas C. Dracopoli, Honathan L. Haines, Bruce R. Korf, Cynthia C. Morton, Christine E. Seidman, J.G. Seigman, Douglas R. Smith.
Current Protocols in Protein Science; regularly updated, e.g. Volume 2003; John Wiley & Sons, Inc; Editors: John E. Coligan, Ben M. Dunn, Hidde L. Ploegh, David W. Speicher, Paul T. Wingfield.
Molecular Biology of the Cell; third edition; Alberts, B., Bray, D., Lewis, J., Raff, M., Roberts, K., Watson, J.D.; Garland Publishing, Inc. New York & London, 1994;
Gene Targeting: a practical approach (1995), Editor: A.L. Joyner, IRL Press
Genetic Manipulation of Receptor Expression and Function; D. Accili, Wiley-Liss., USA, 2000; ISBN: 0-471-35057-5.
Antisense Therapeutics, S. Agrawal, Humana Press, USA, 1996, ISBN: 0-89603-305-8 .

### Abbreviations used:

AC, adenylyl cyclase; Gsα, α subunit of the stimulatory G protein of adenylyl cyclase, Gsα*, constitutively active (Q213L) mutant of Gsα; Giα, α subunit of the inhibitory G protein Gi; Gβy, βy subunits of heterotrimeric G proteins; PAM, protein associated with Myc; RCC1, regulator of chromosome condensation; TED, 50 mM Tris-HCl, pH 8.0, 1 mM EDTA, 1 mM DTT; ODN, oligodeoxynucleotide; RT, room temperature;

### Figures:

Figure 1: PAM is highly expressed in spinal cord neurons. In situ hybridization using horizontal sections of spinal cords hybridized with sense or antisense probes against rat PAM as described above.
Figure 2: PAM is expressed in DRG neurons as well as in neuronal cells in rat spinal cord.
   **Panel A:** Immunhistochemical analysis of rat spinal cord sections. The sections were stained with anti-PAM antibody (green) and anti-NeuN or anti-GFAP (red) to visualize neurons or glia cells, respectively. The overlay of both signals is presented in the right panels. The objects were magnified 20x.
   **Panel B:** Immunhistochemical analysis of rat DRGs sections. The sections were stained with anti-PAM antibody (green) and anti-Neu68, anti-Histon or anti-GFAP (red) to visualize neurons, nuclei or glia cells, respectively. The overlay of both signals is presented in the right panels. The objects were magnified 40x except for the Histon staining, which was magnified 63x.
Figure 3: PAM is differentially expressed in DRGs and spinal cord during different developmental stages. Quantitative RT-PCR (Taqman™) was used to detect PAM in RNA (40 ng) of spinal cord, and DRGs of embryonic rats day 16 (E16), postnatal day 0,5 (P0.5), 3 (P3), 5 (P5), 9 (P9) and adult rats. The mean ± SEM of at least 3 determinations is shown.
Figure 4: PAM is upregulated in the rat spinal cord after zymosan and formalin treatment.
   **Panel A:** RT-PCR analysis with RNA (40 ng) of spinal cords from control animals or animals treated with zymosan after 24h, 48h and 96h. The lower panel shows the mean ± SEM of 7 experiments. Student T test: * p< 0.001.
   **Panel B:** Western blot analysis using a 7% SDS-PAGE gel with rat spinal cord lysates of control animals or animals treated with zymosan after 24h, 48h and 96h (40µg) with anti-PAM antibody and anti-ERK1/2.
   **Panel C:** Quantitative RT-PCR analysis with RNA (40 ng) of spinal cords from control animals or animals treated with formalin for 1 hour.
Figure 5: PAM inhibits Gas-stimulated AC activity in spinal cord lysates.
   **Panel A:** RT-PCR was used to determine AC isoform expression in spinal cord and DRG RNA (40 ng).
   **Panel B:** Lysates of spinal cords or DRG (10 µg) were assayed for AC activity in the presence of 80 nM Gαs as described in Material and Methods. The mean ± SEM of at least 3 determinations done in triplicates is shown.
Figure 6: Intrathecal application of antisense ODNs against PAM increases nociceptive behavior.
   **Panel** A: Adult rats were given intrathecal sense and antisense ODN as described. After formalin treatment, the spinal cord was removed and subjected to immunhistological analysis using anti-PAM antibodies (green) or anti NeuN (red).
   **Panel B:** Formalin assay of animals treated with sense or antisense ODNs as described in the material section. The total amount of flinches over 1 hour is shown. The mean ± SE of at least 4 determinations is shown.
   **Panel C:** Formalin assay of animals treated with sense or antisense ODNs as described in the material section. The number of flinches during 1 hour is shown. The mean ± SE of at least 4 determinations is shown.

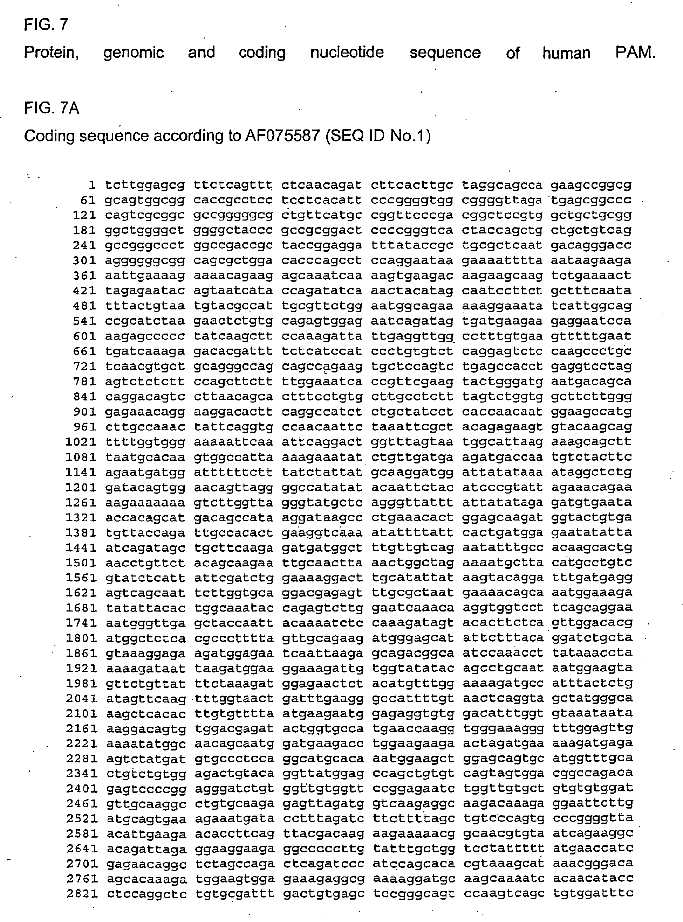

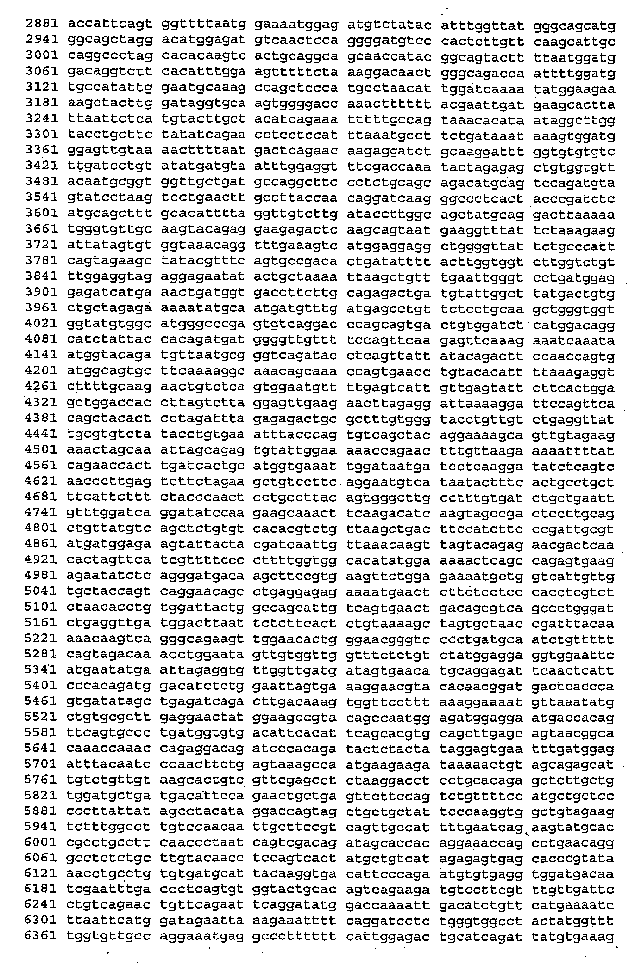

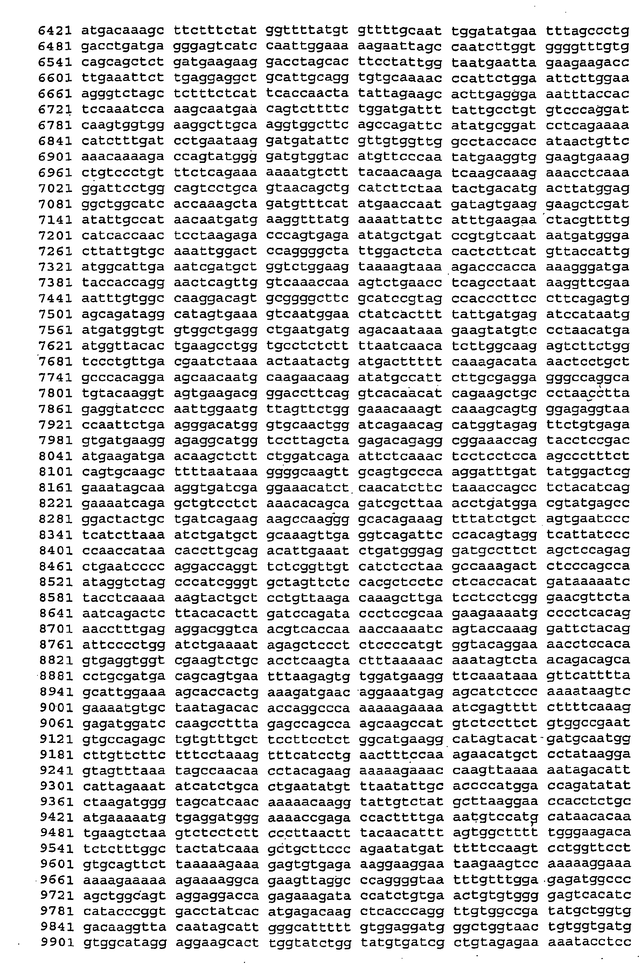

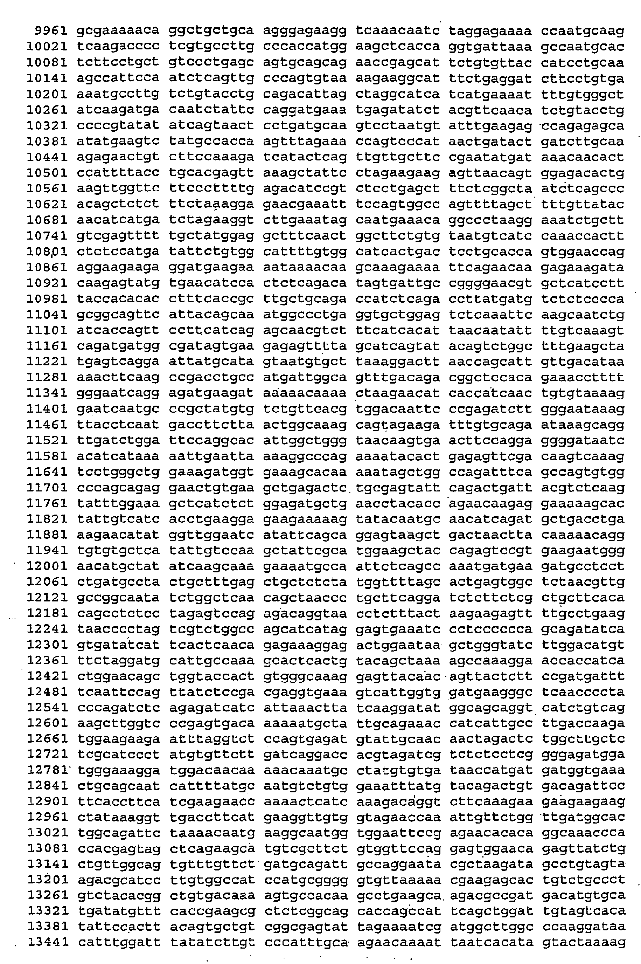

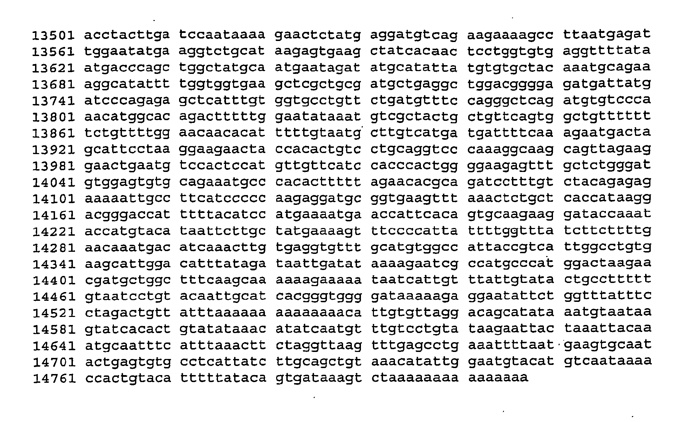

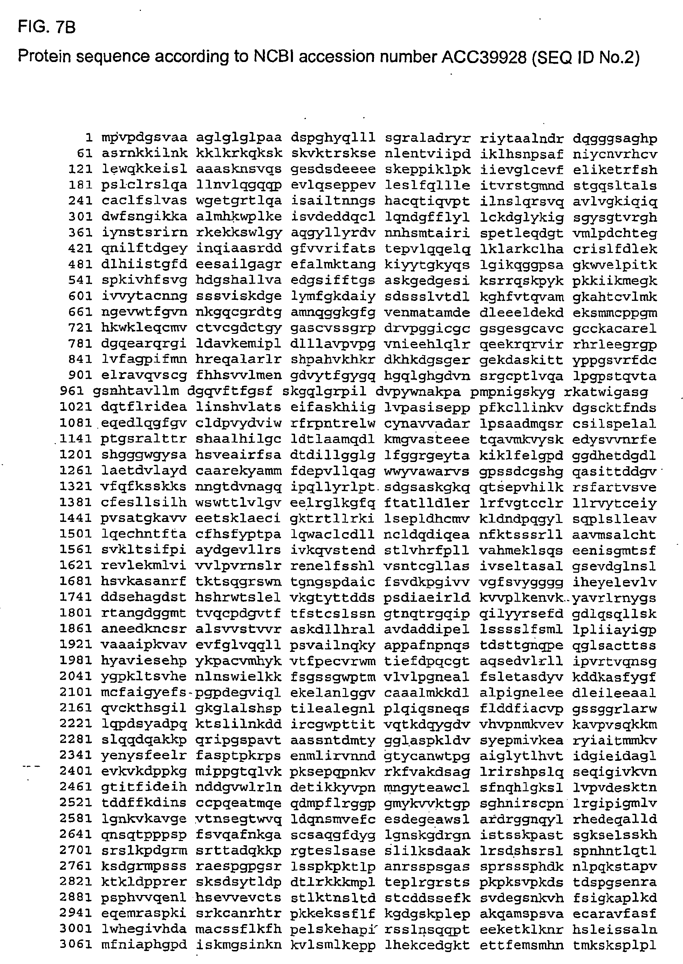

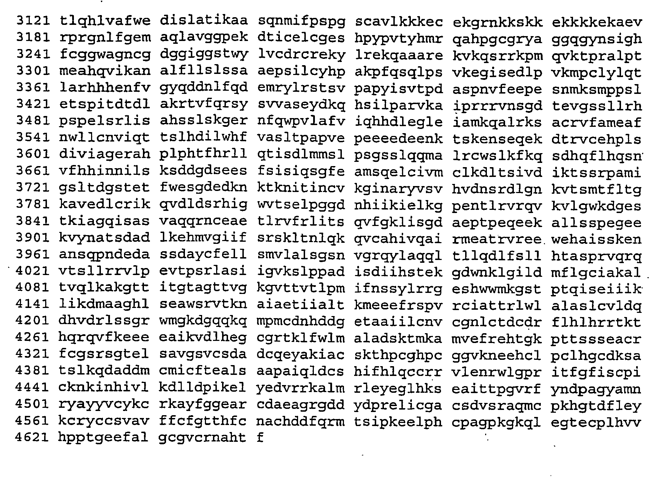

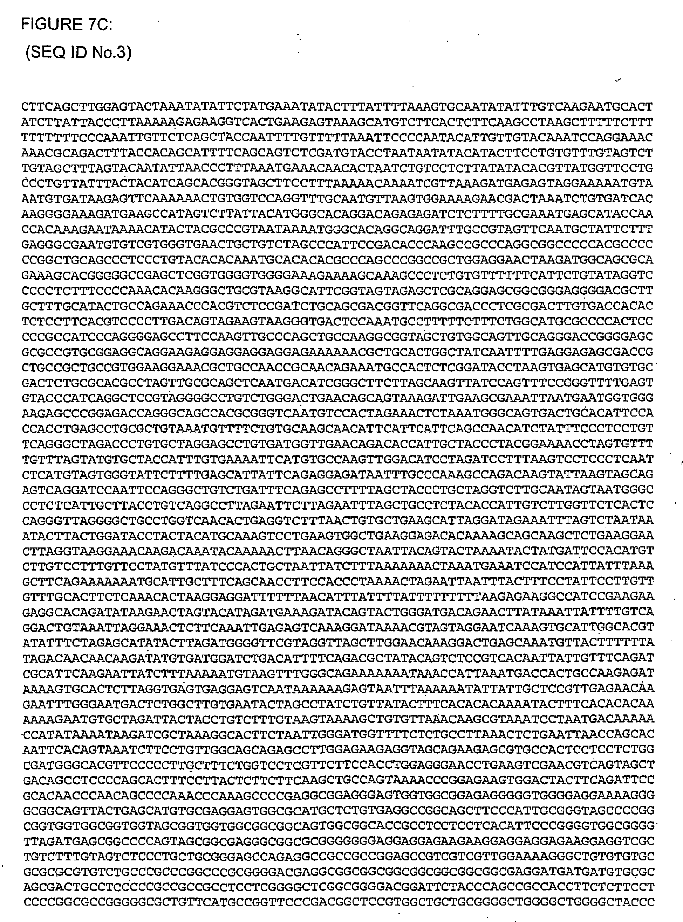

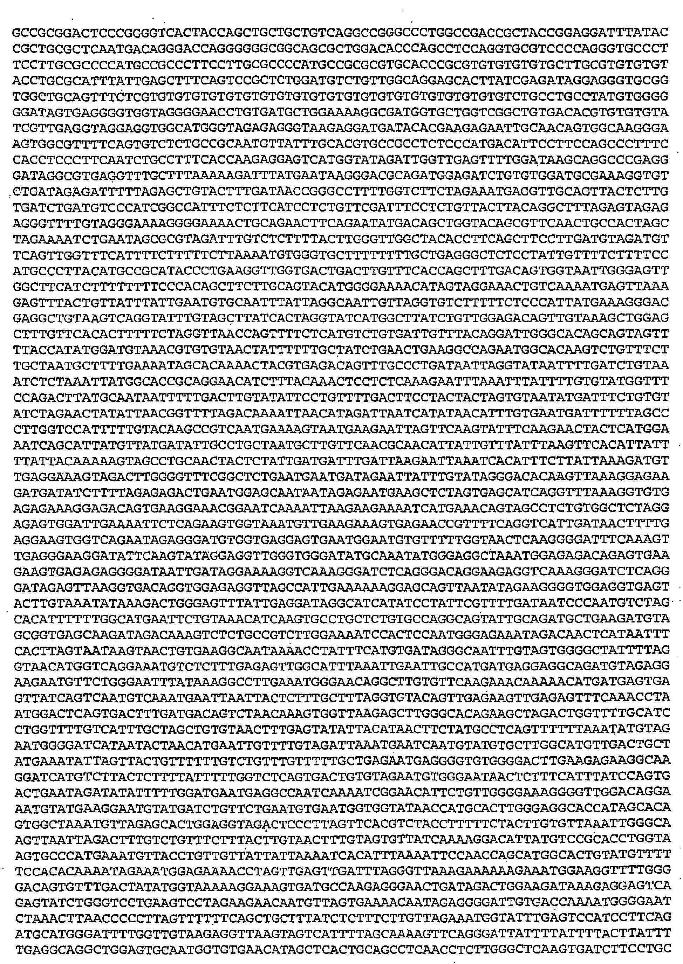

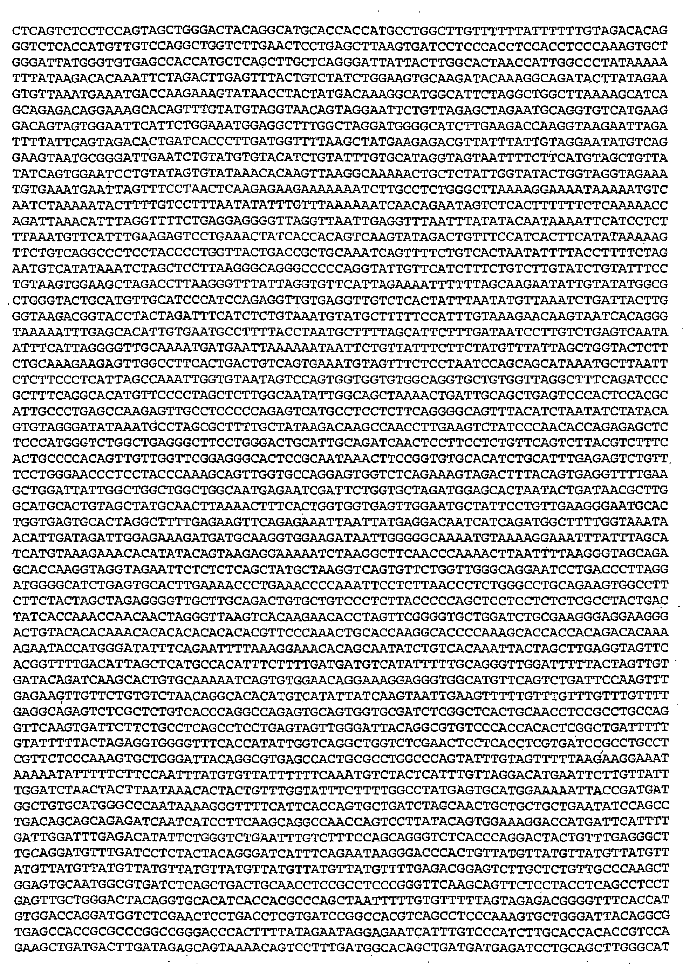

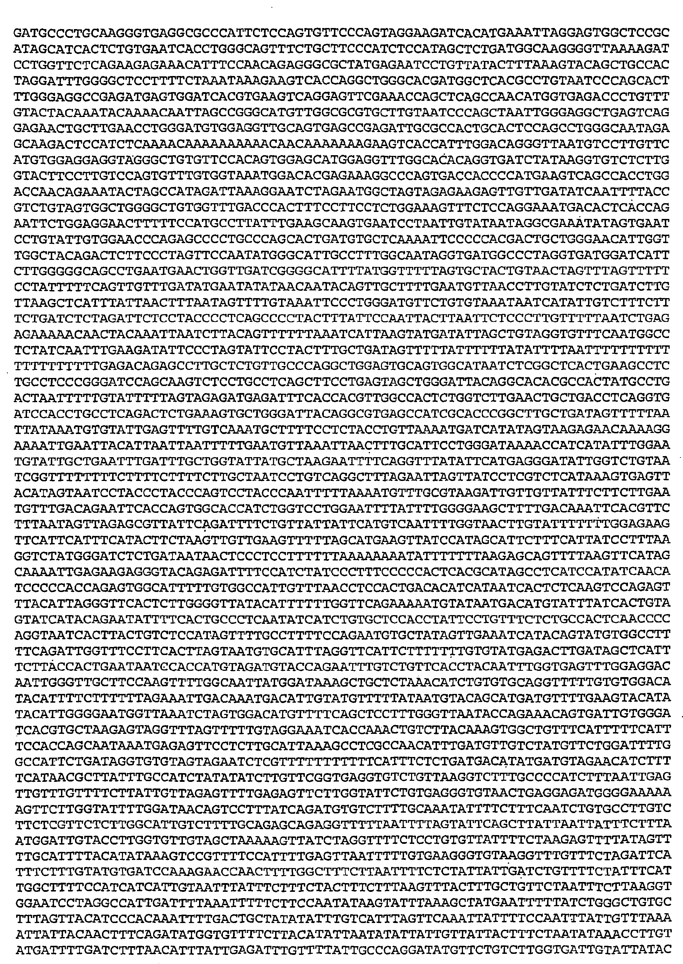

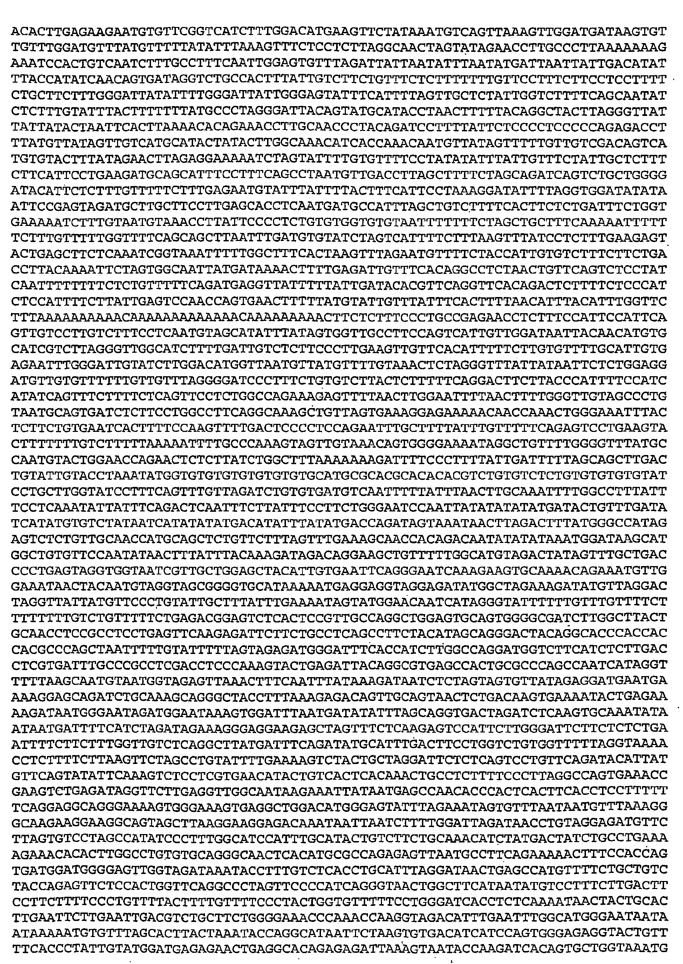

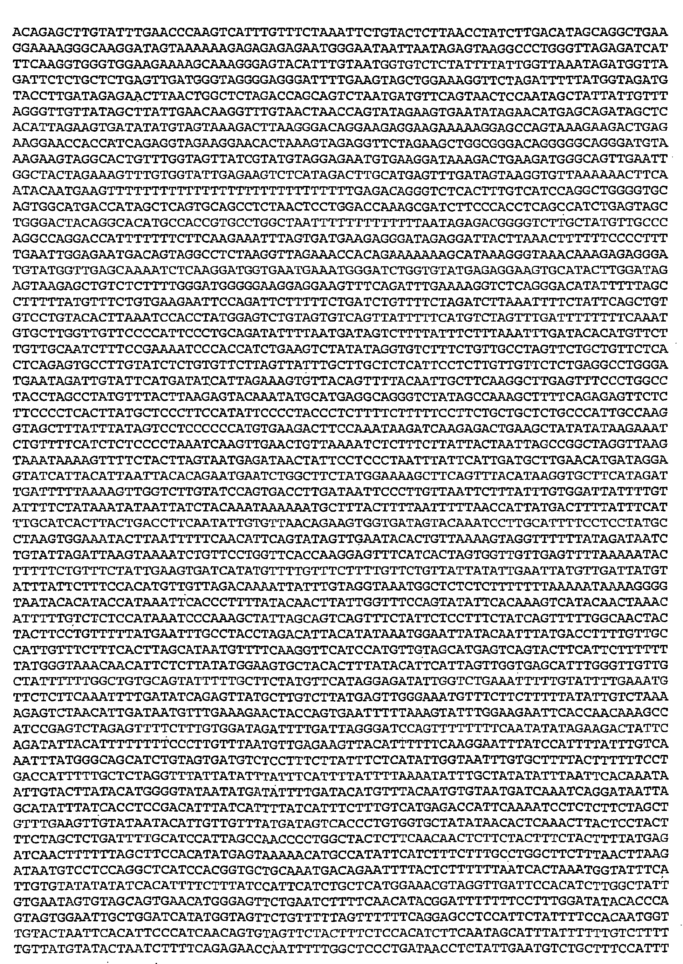

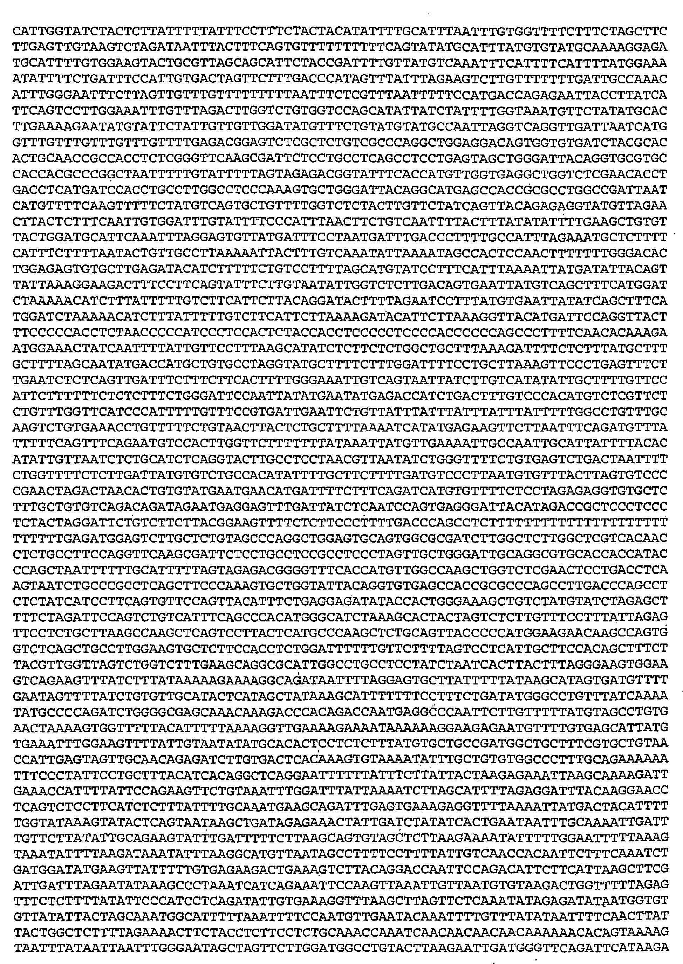

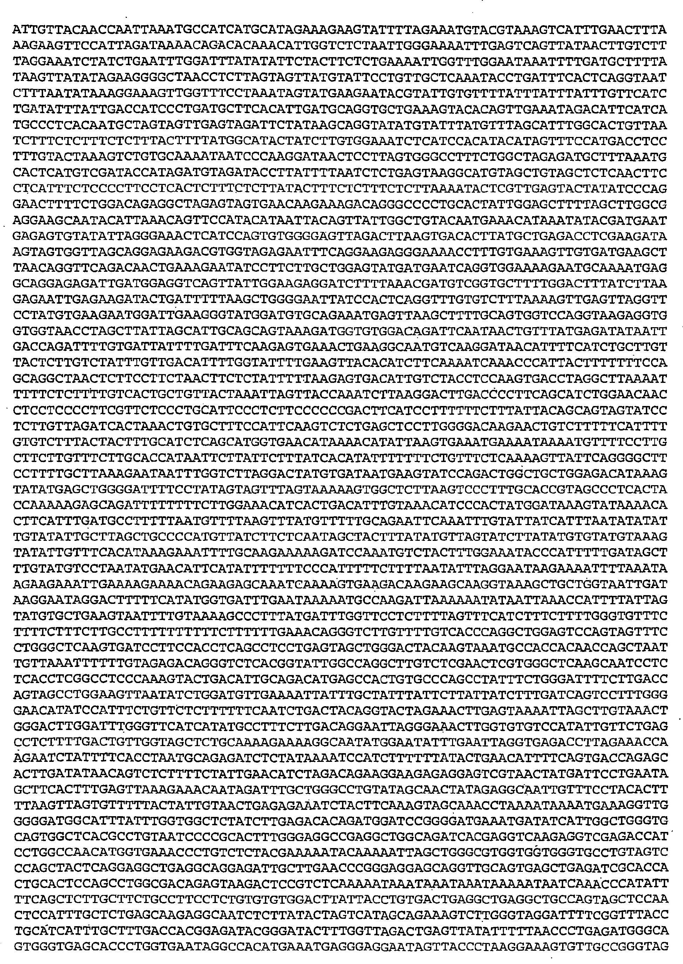

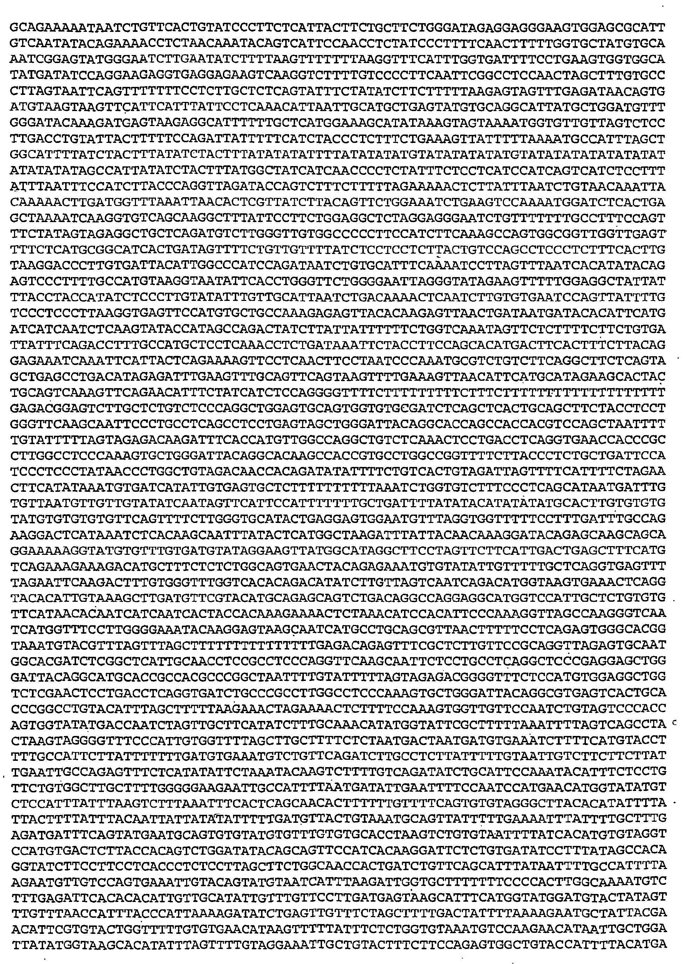

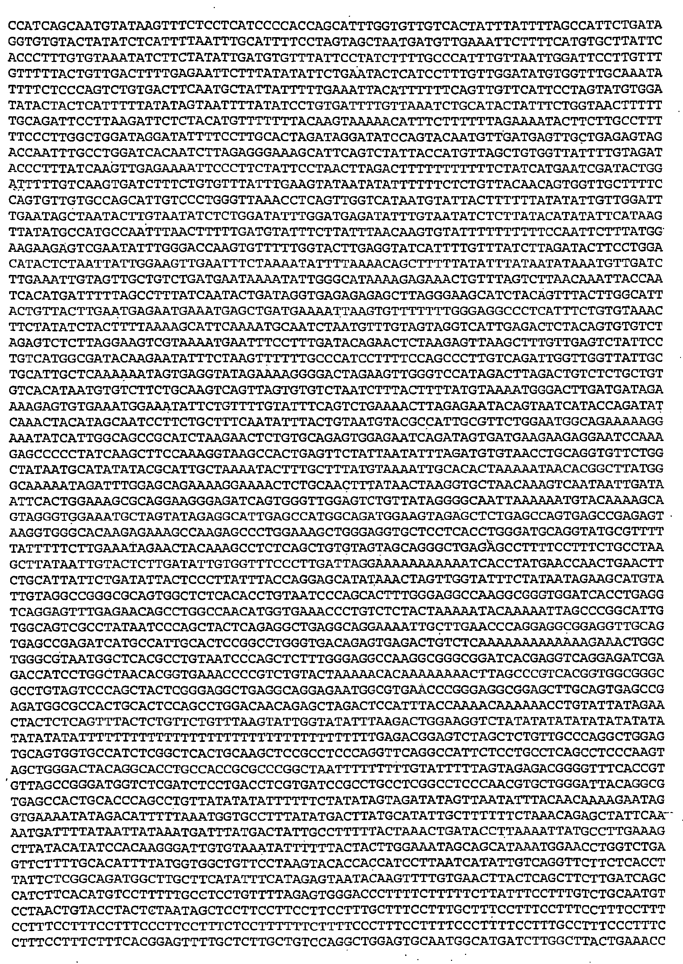

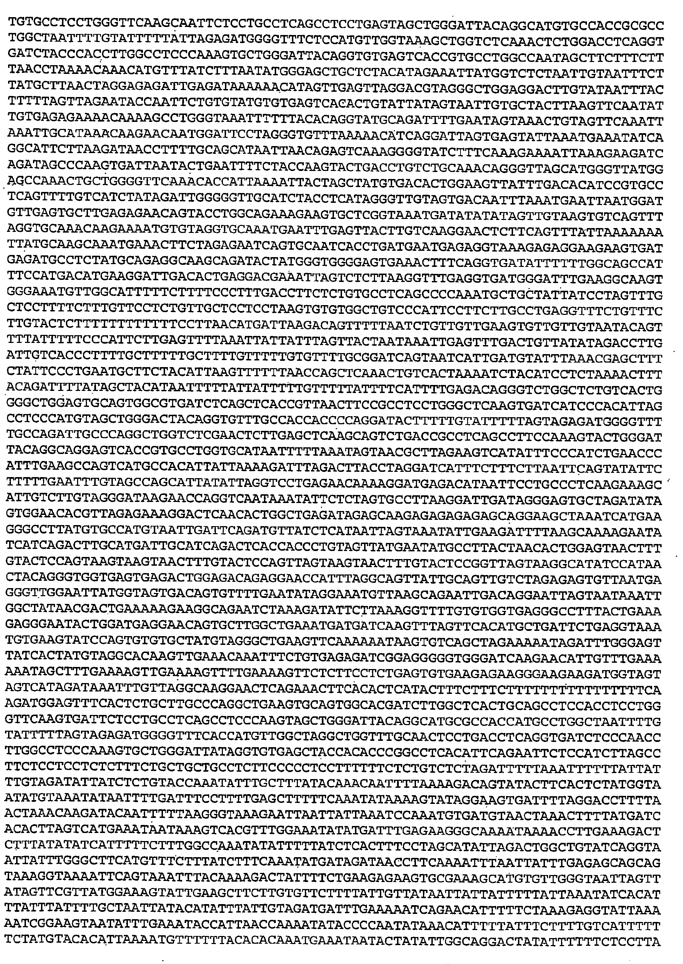

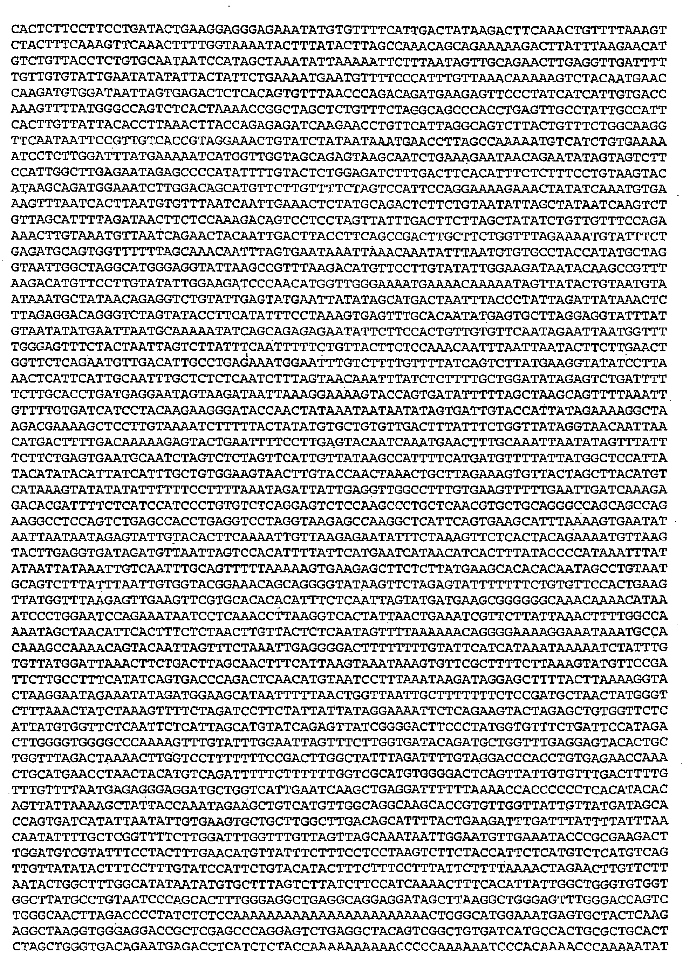

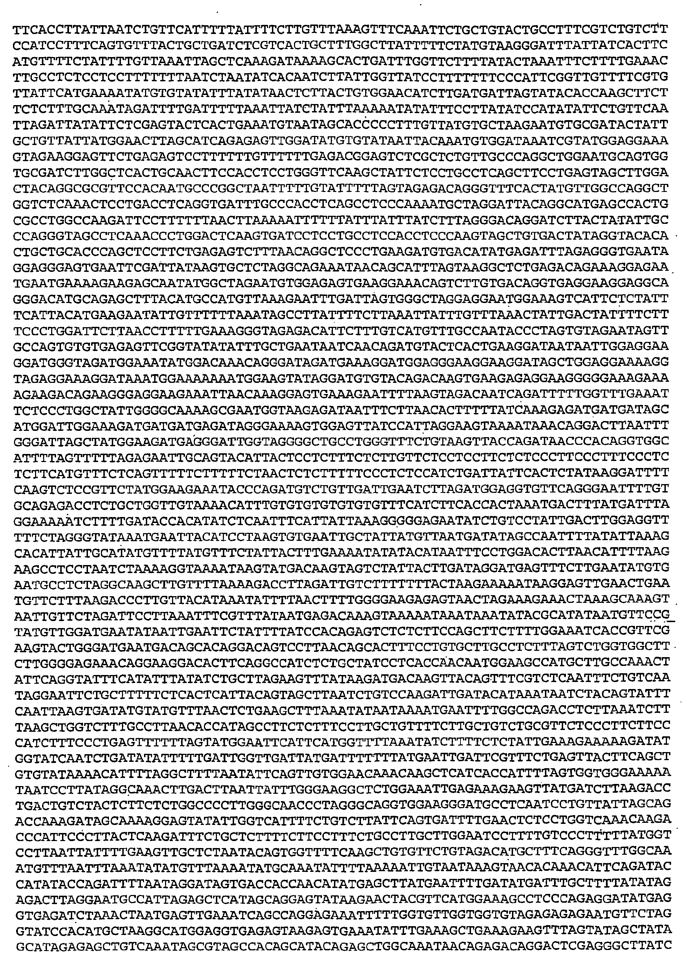

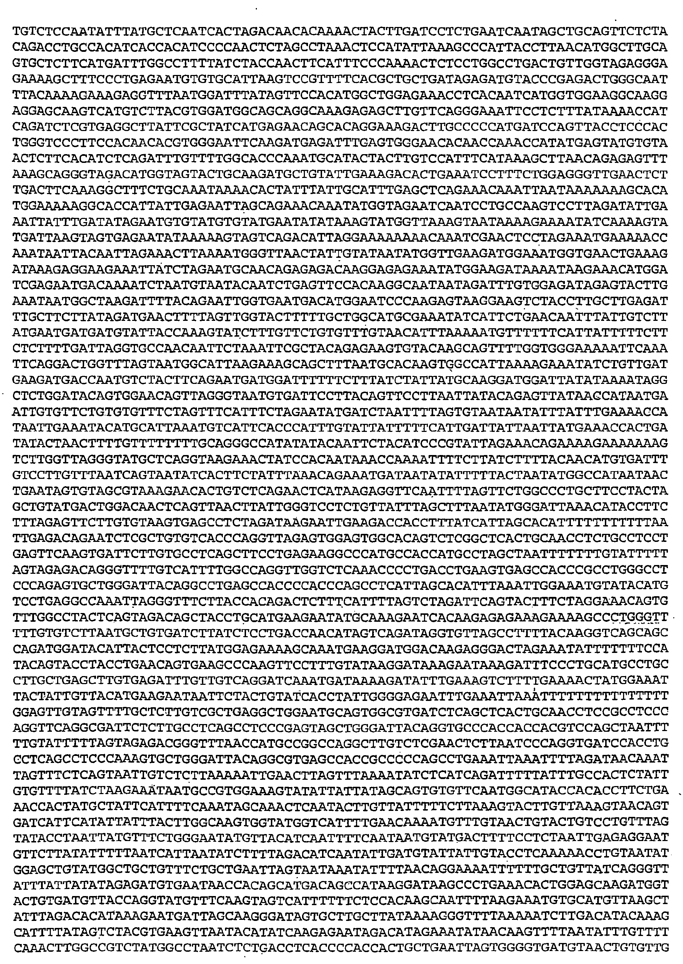

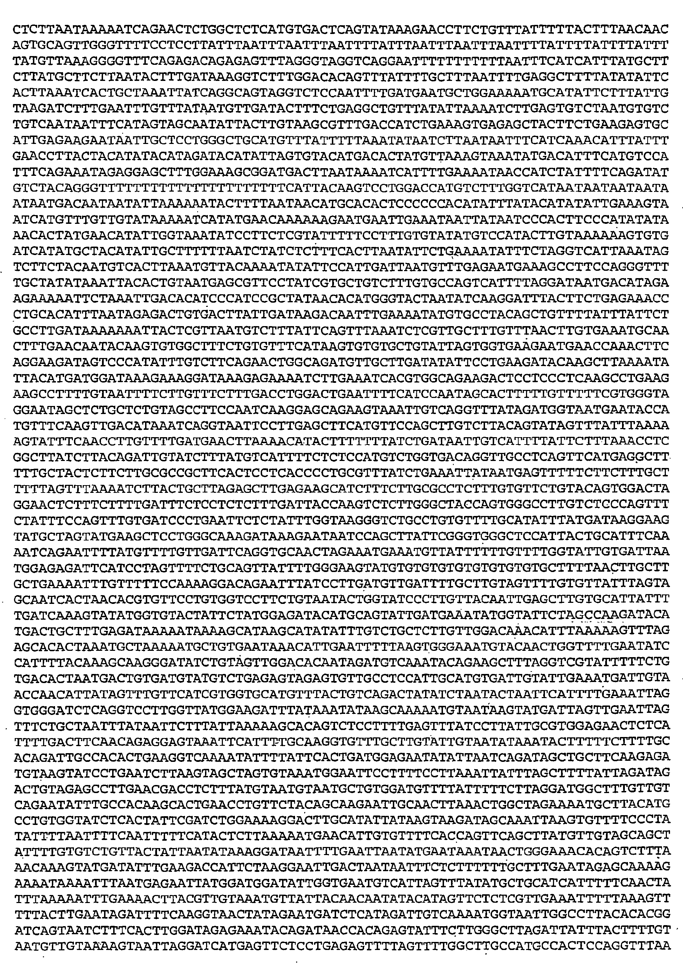

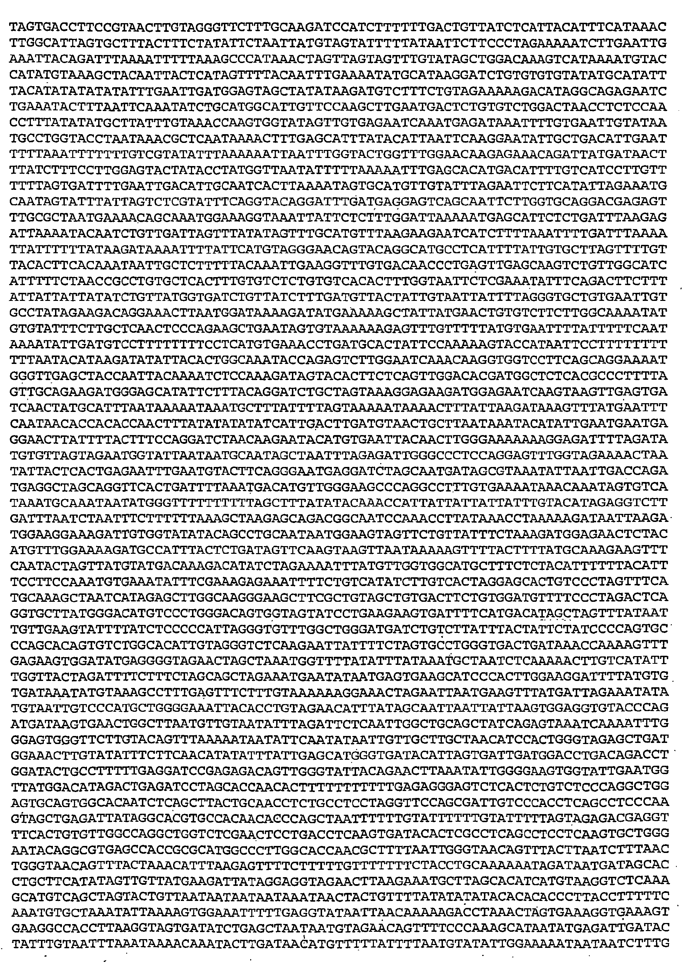

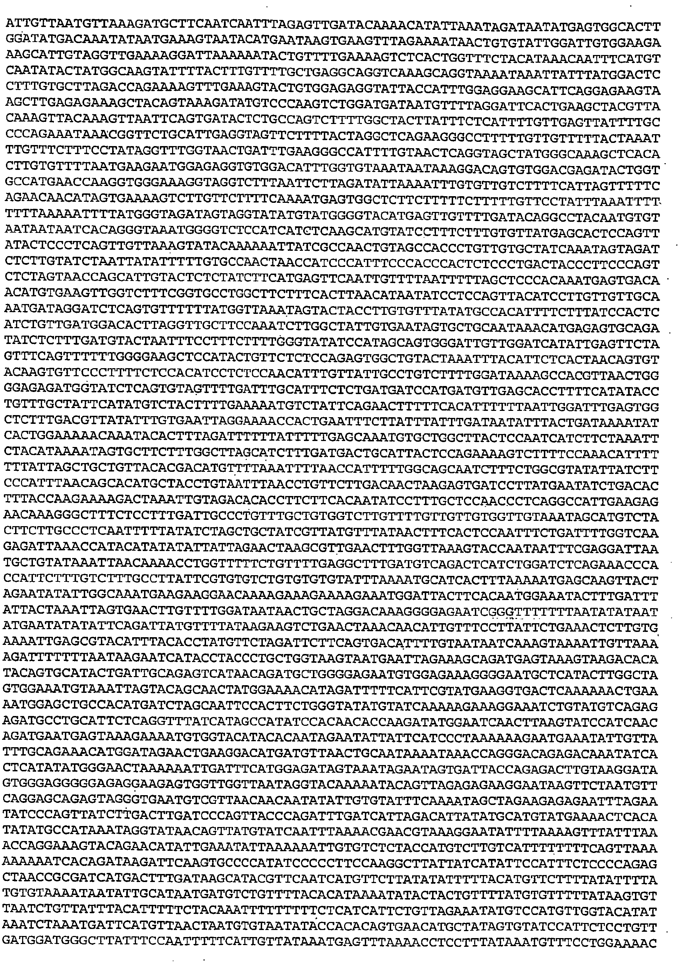

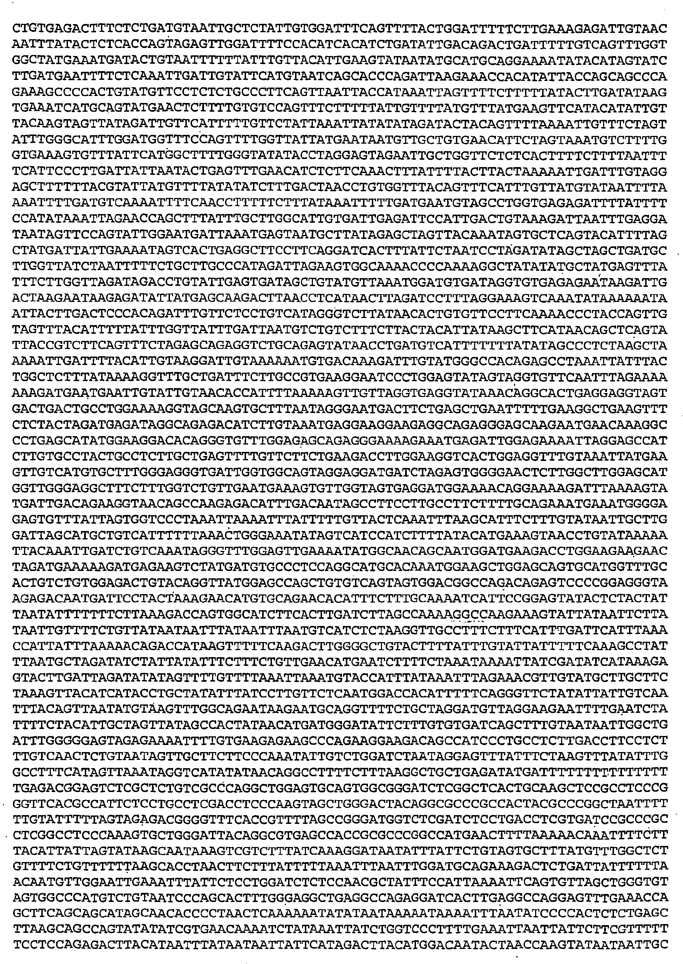

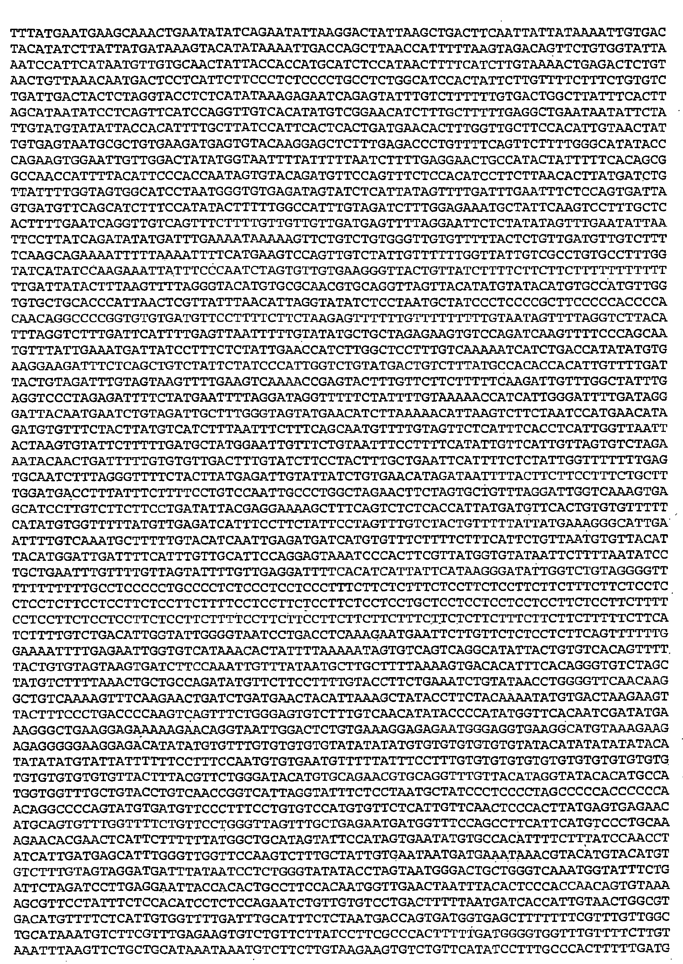

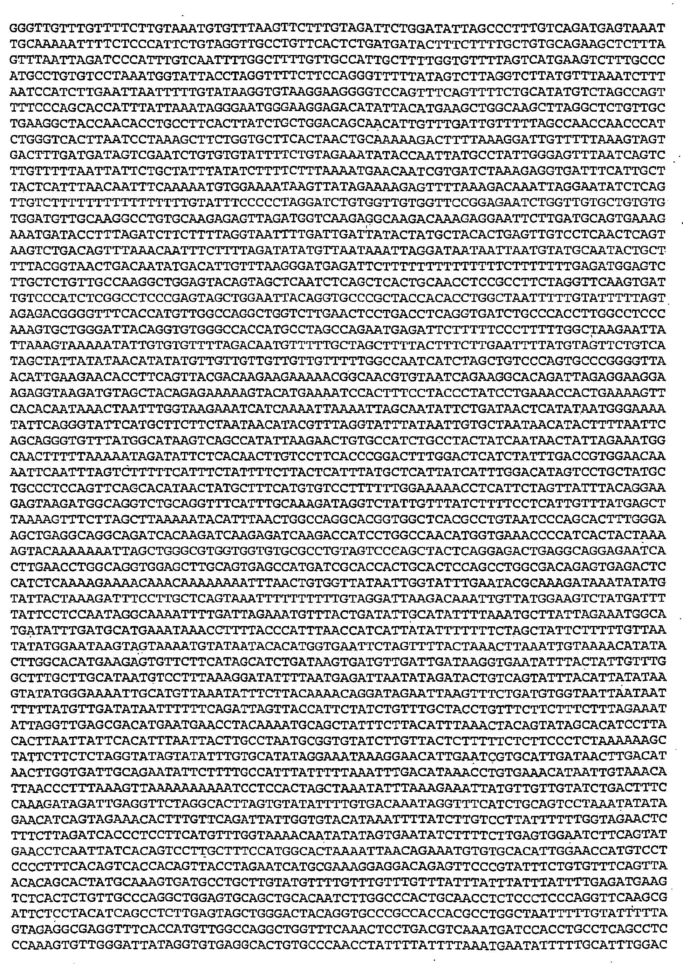

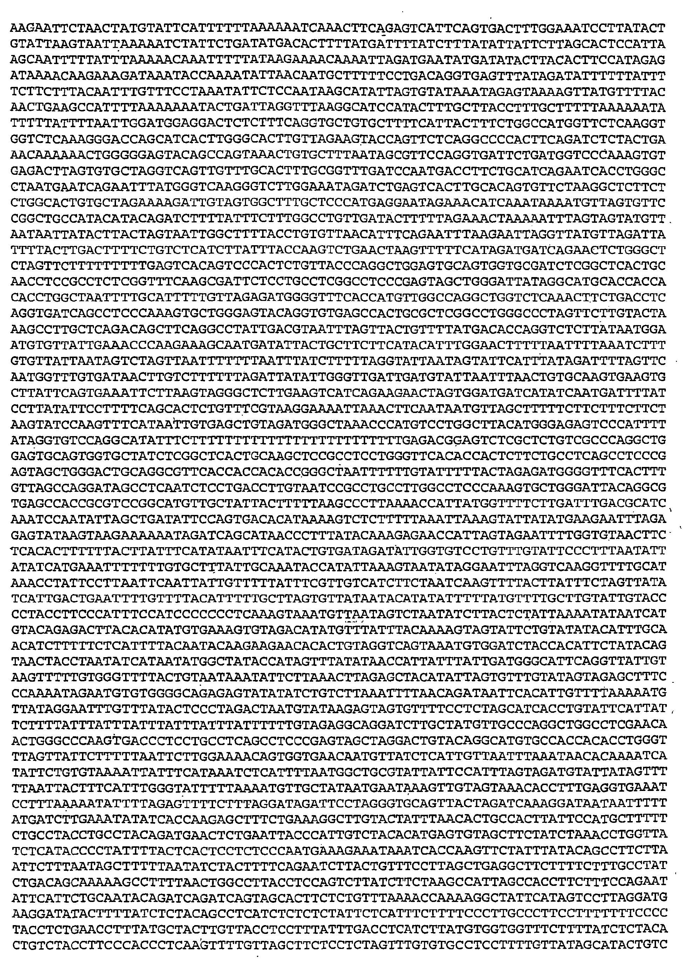

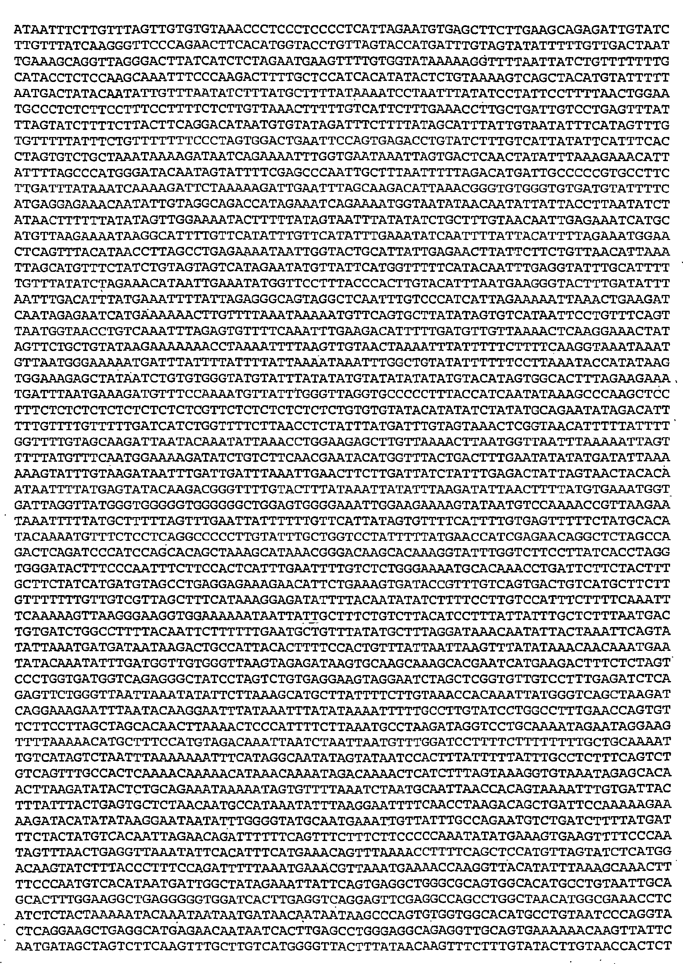

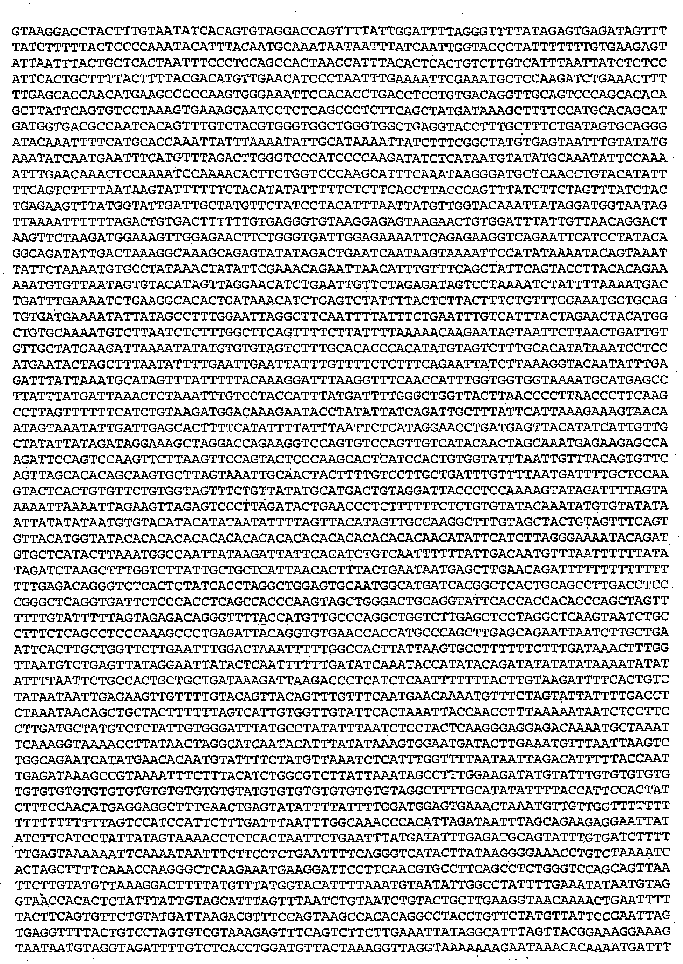

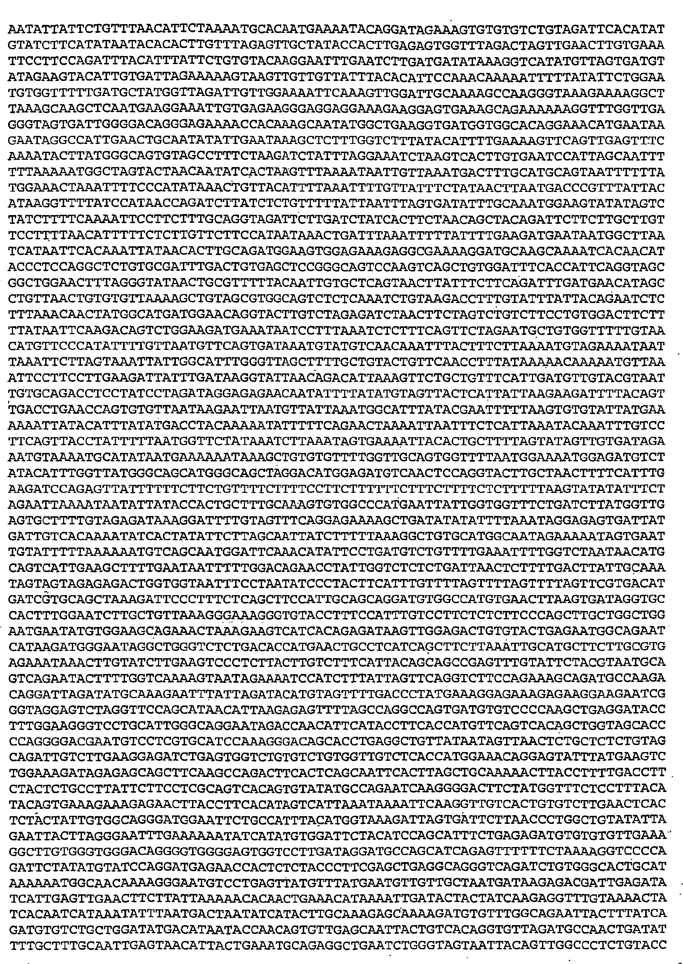

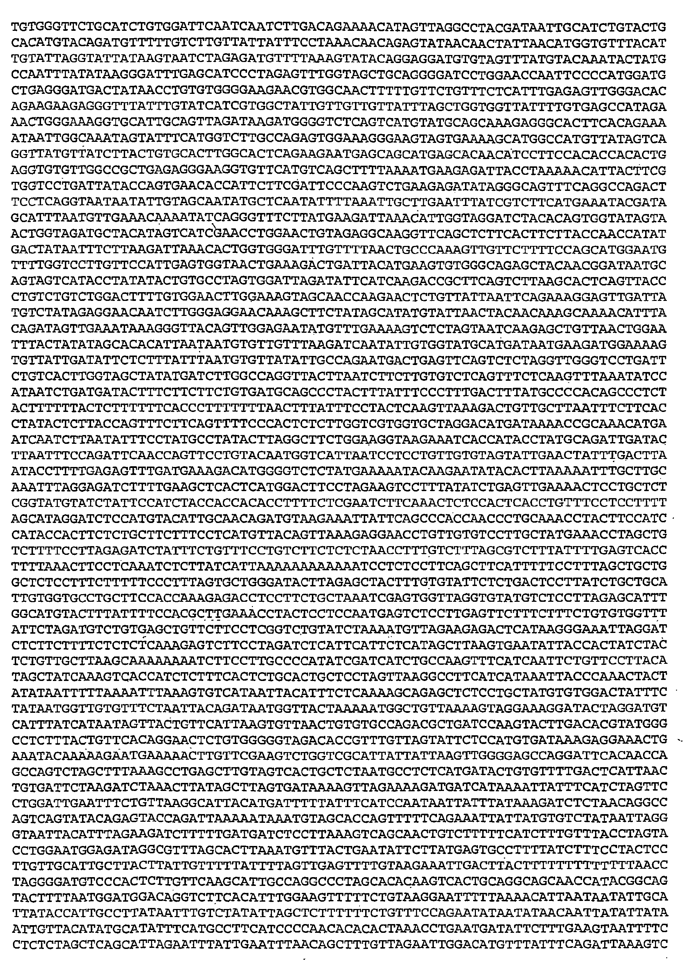

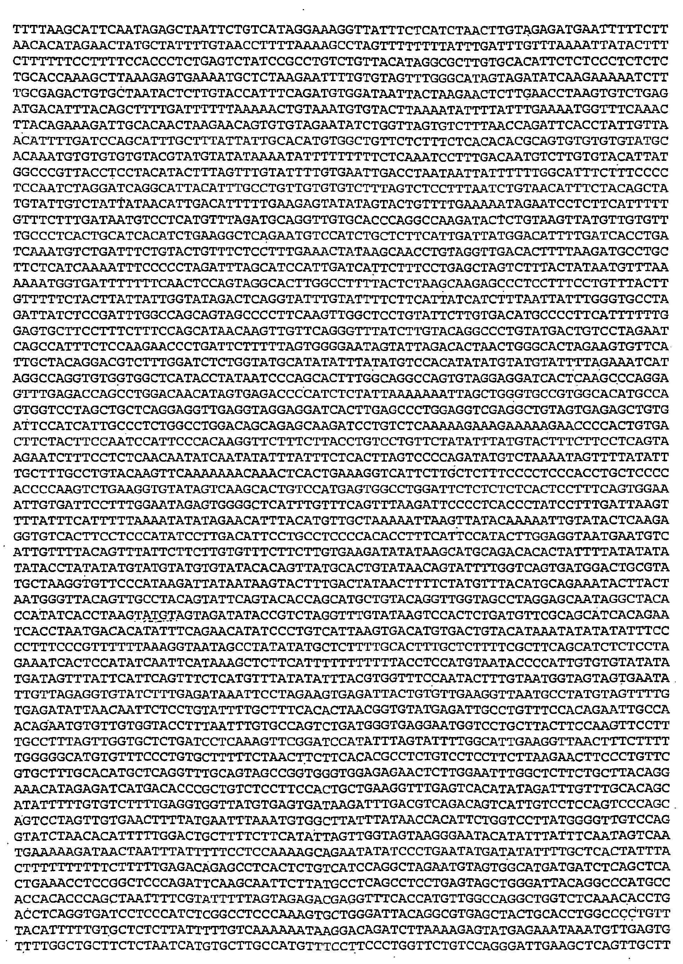

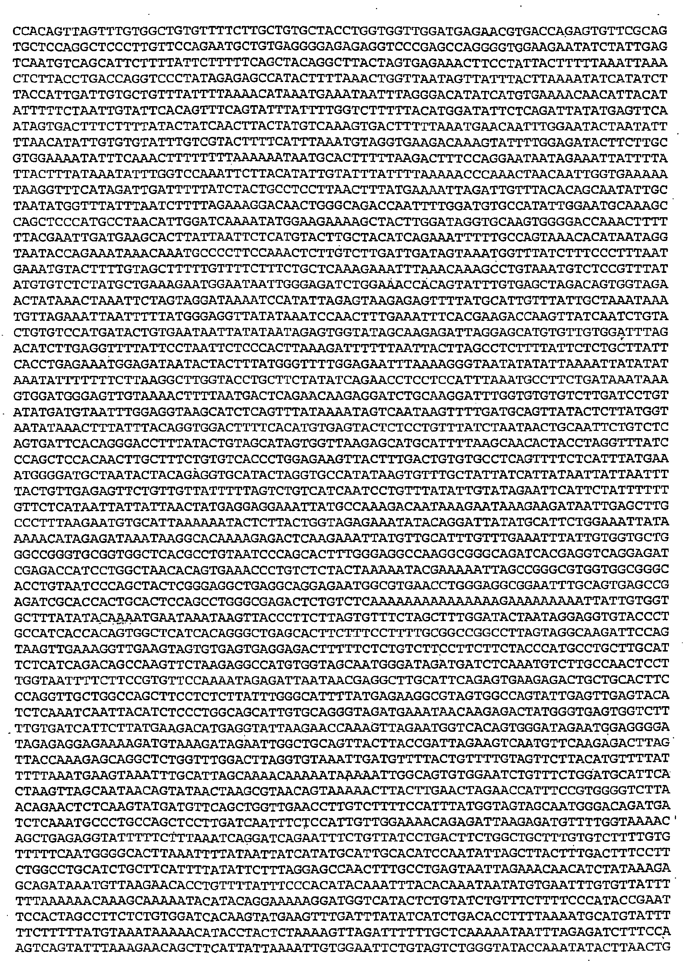

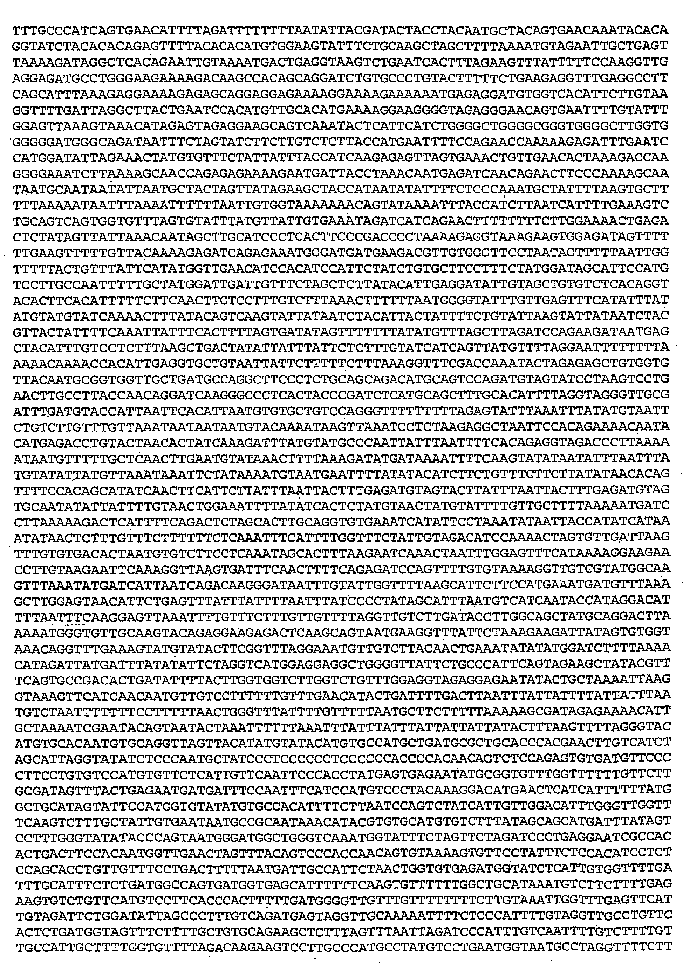

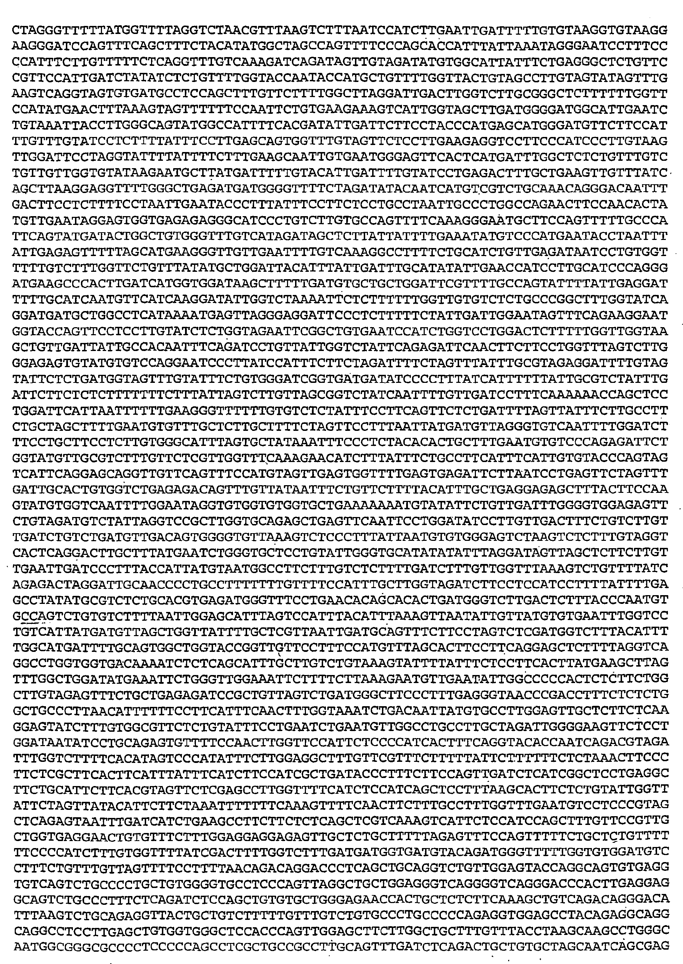

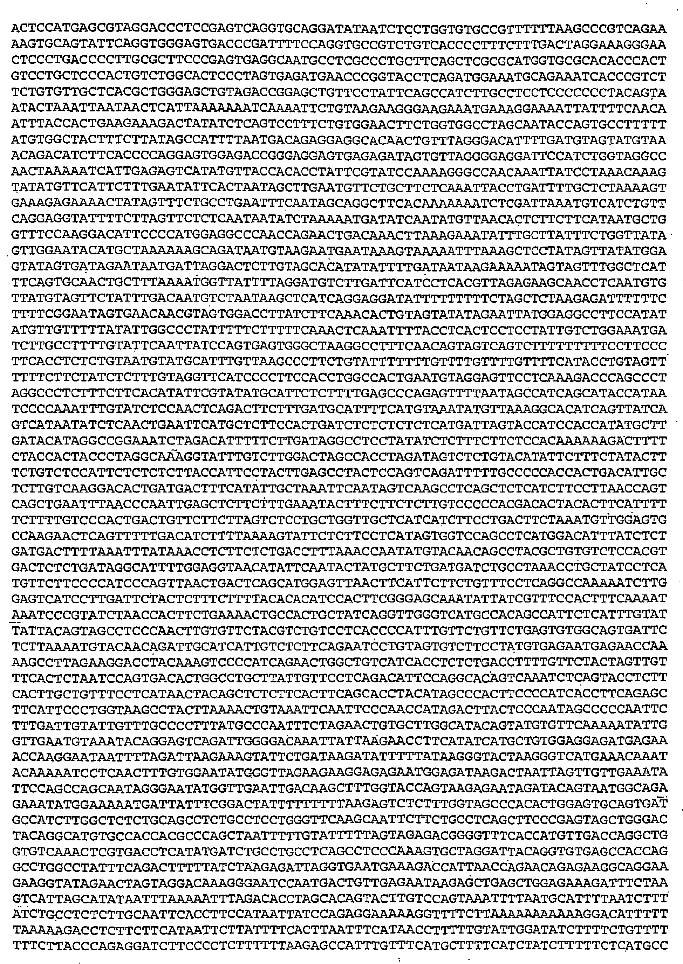

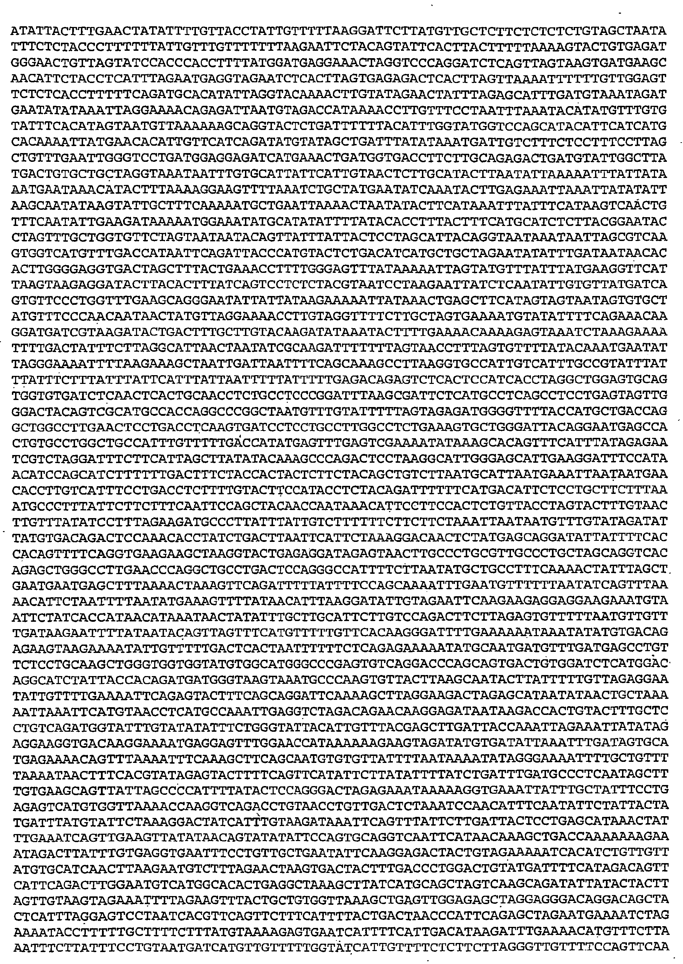

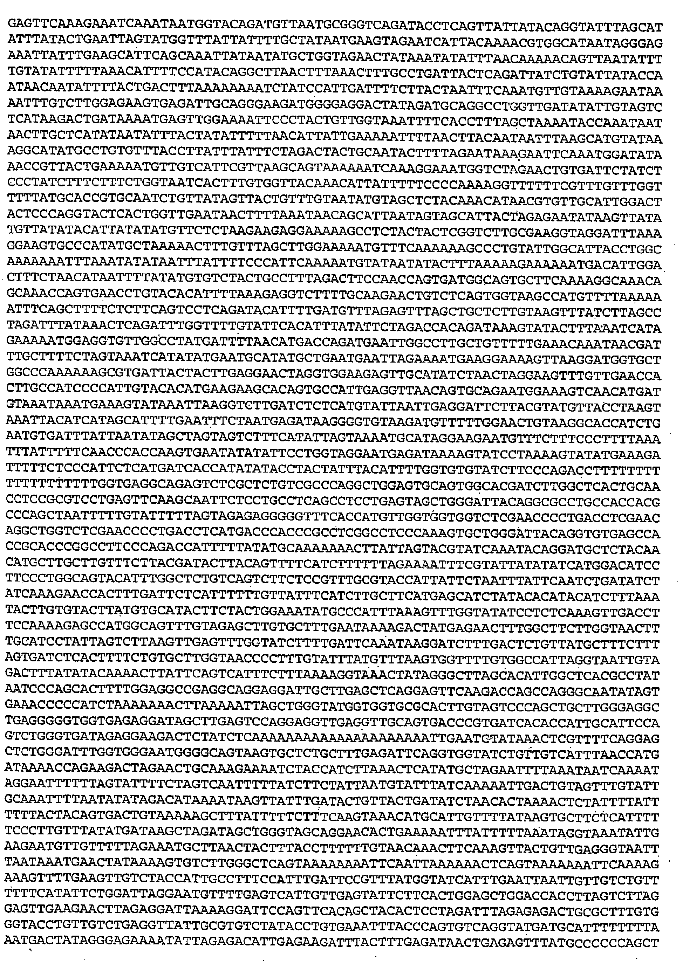

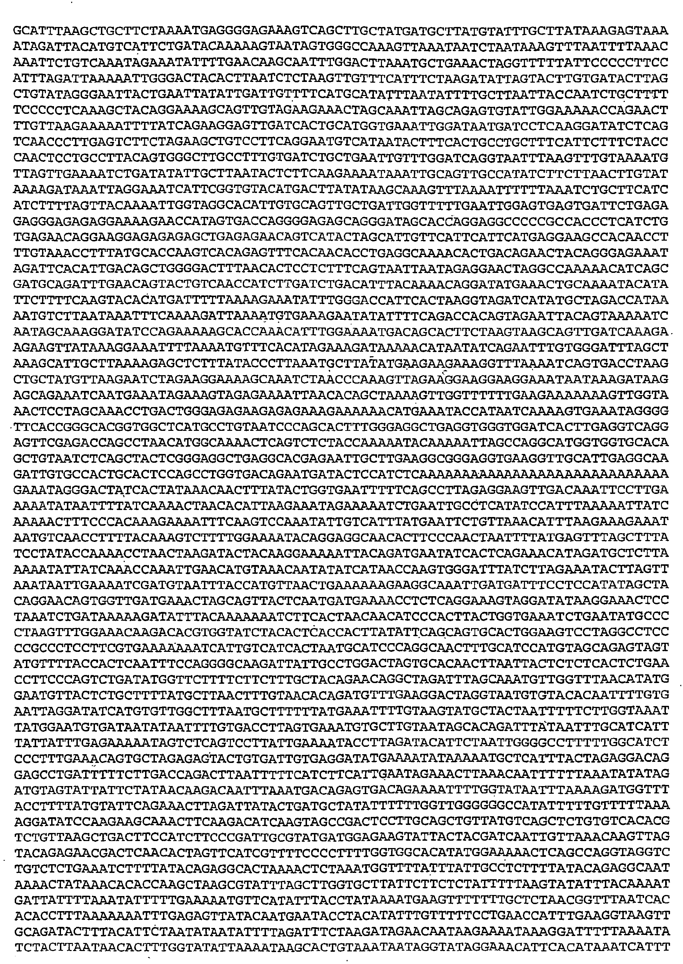

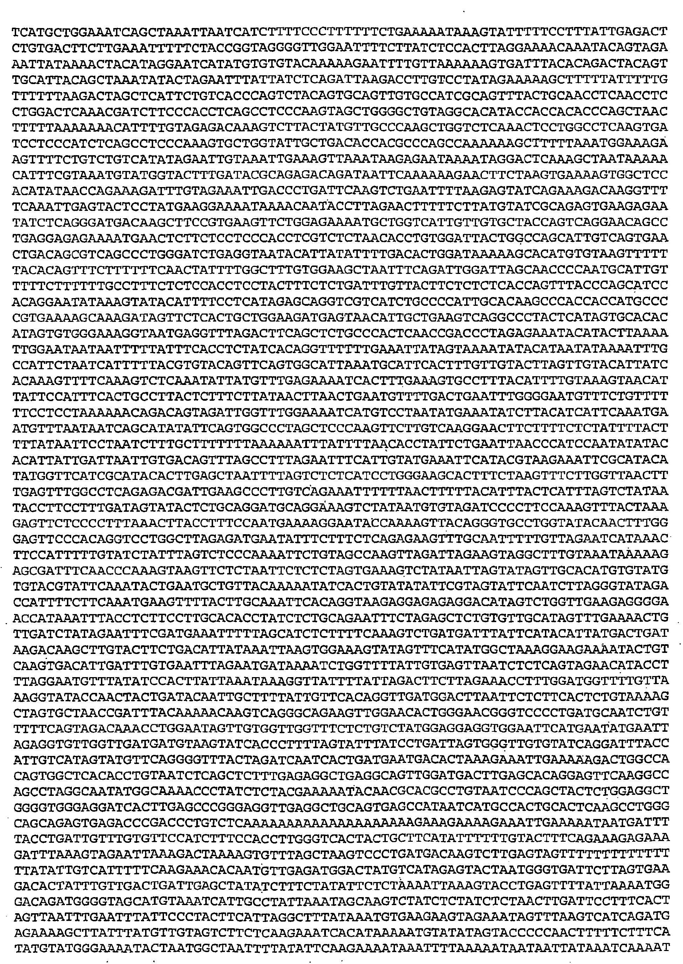

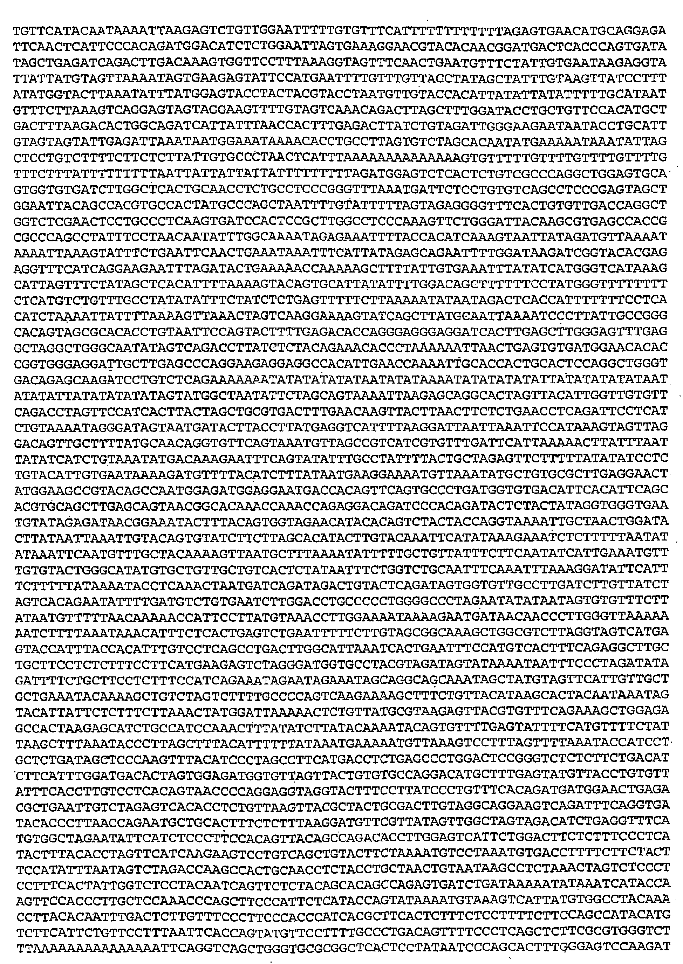

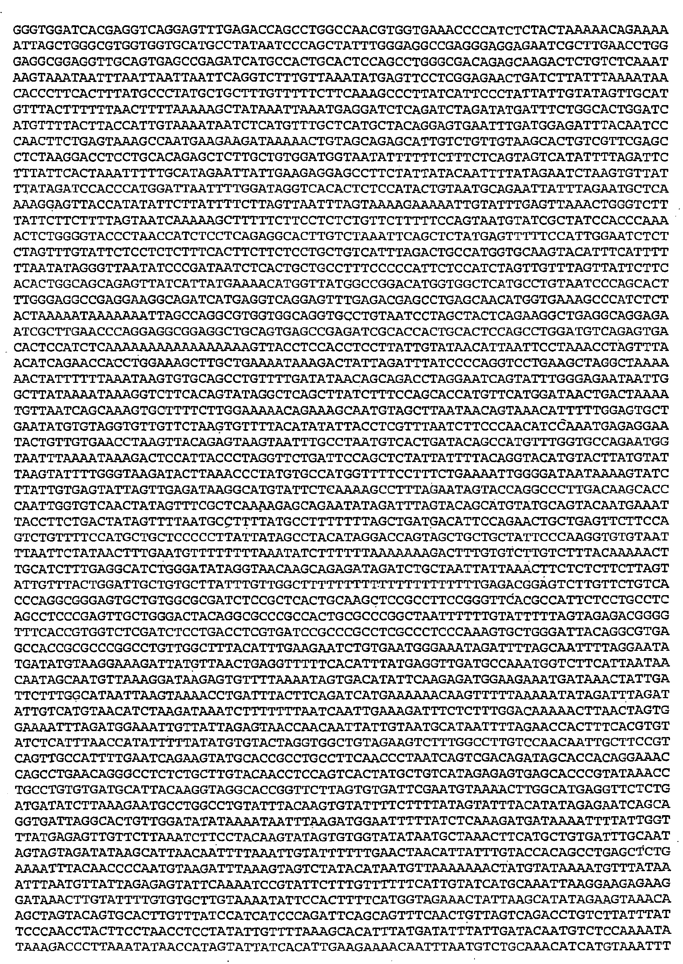

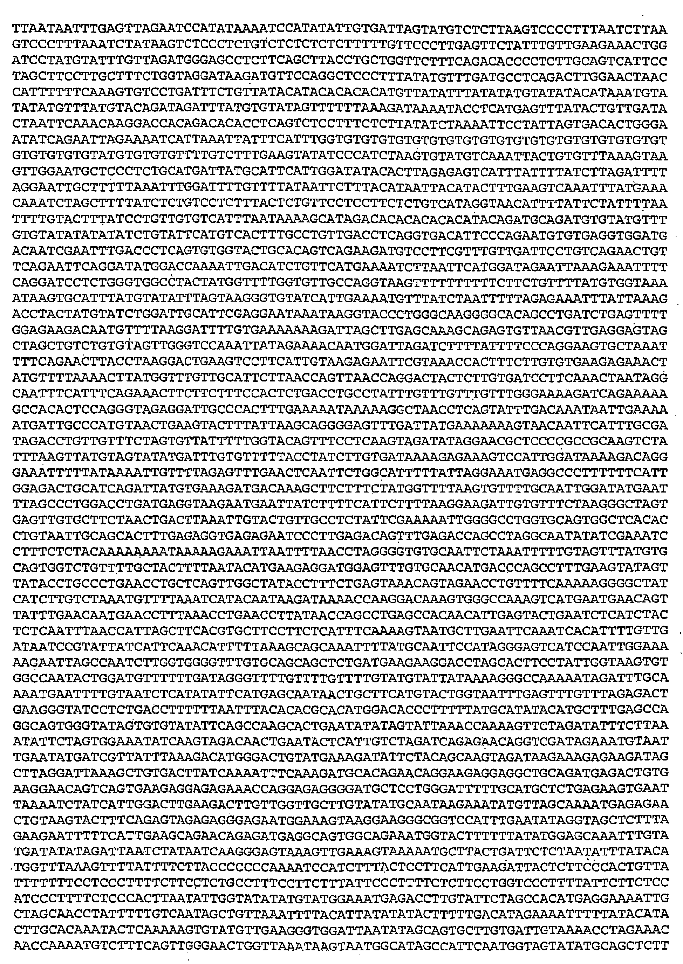

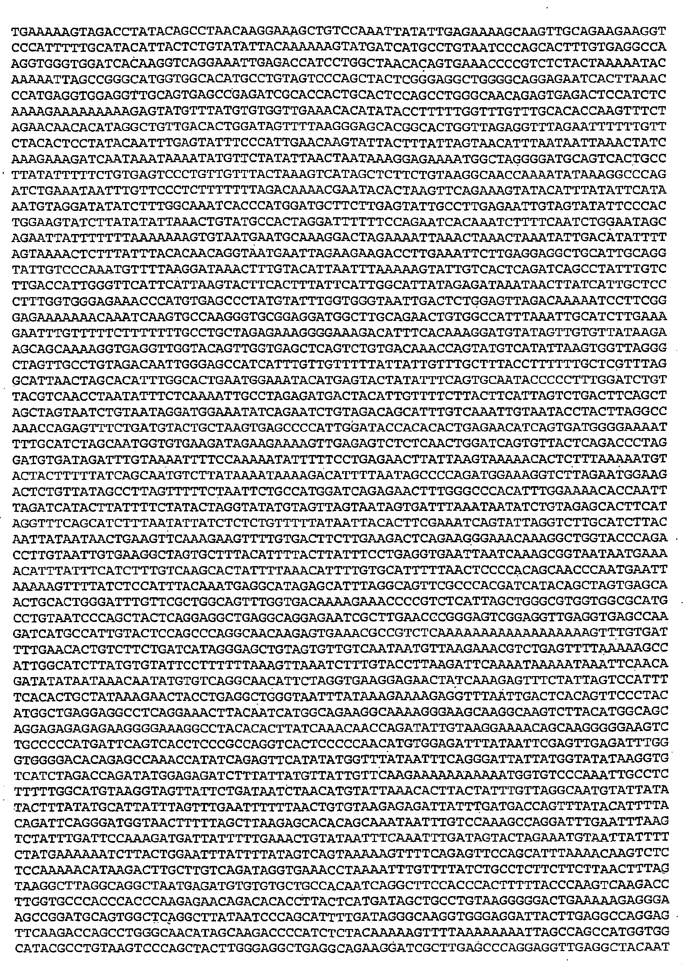

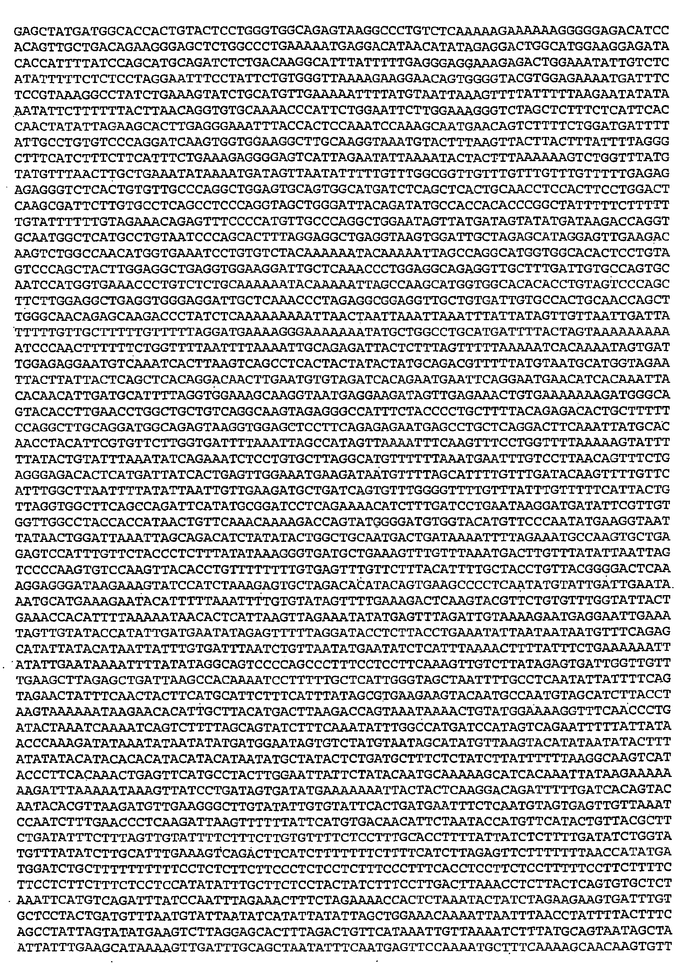

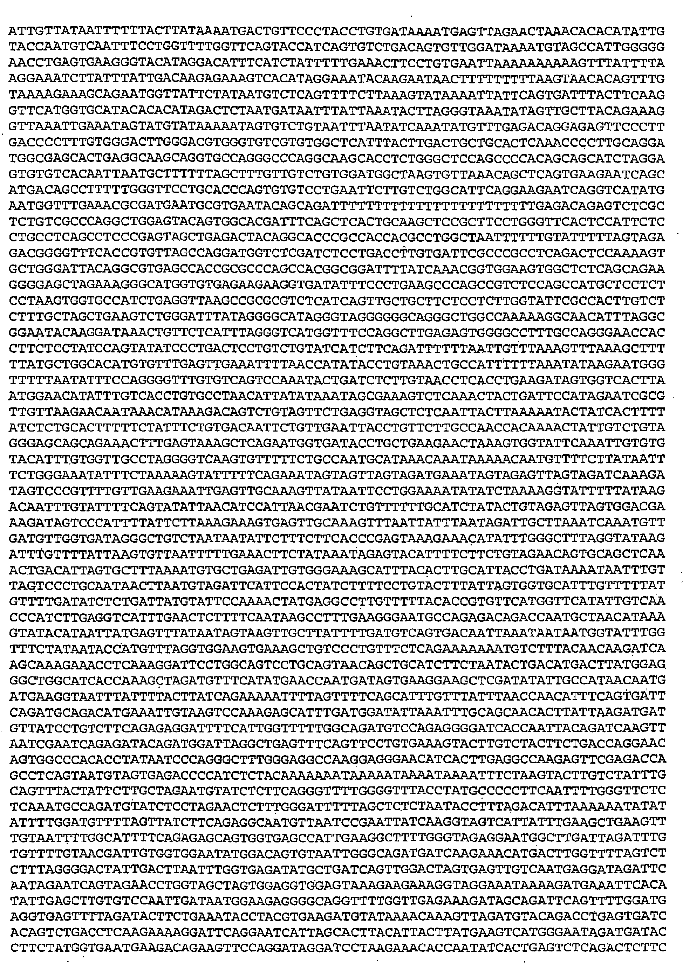

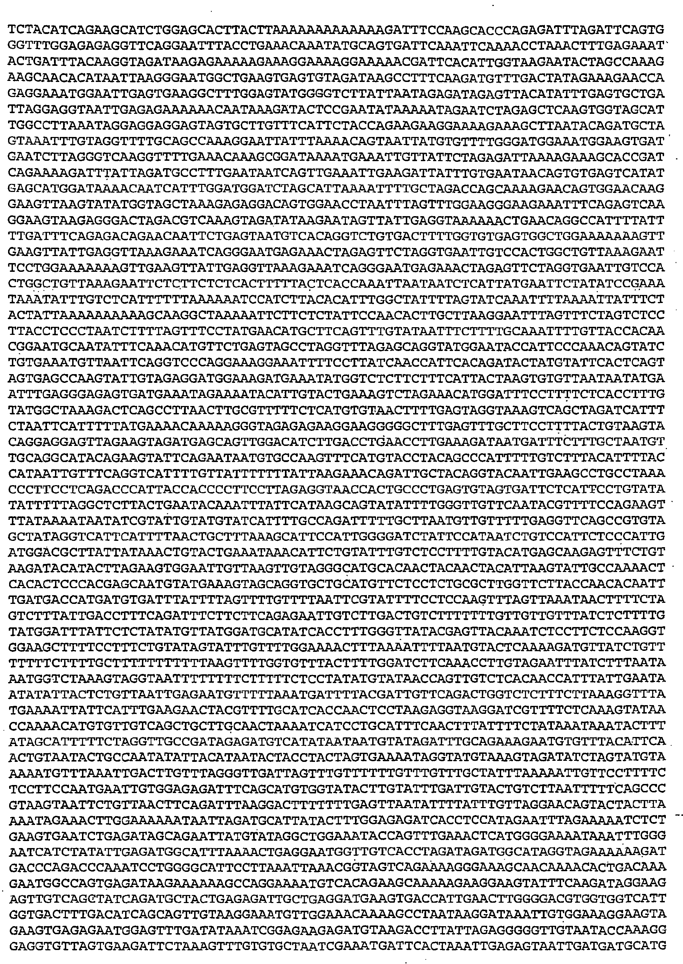

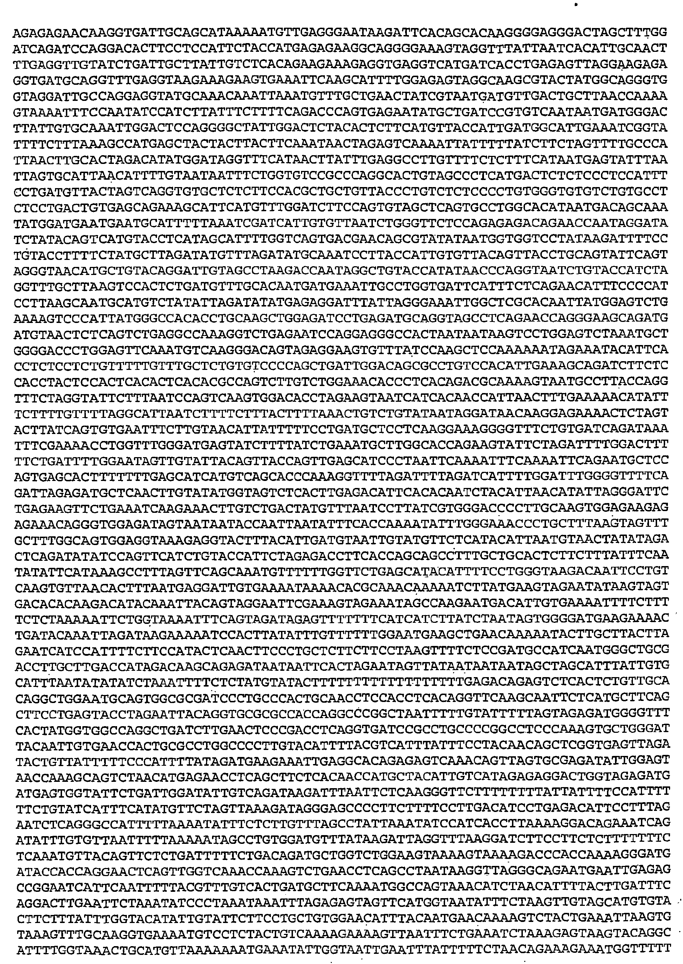

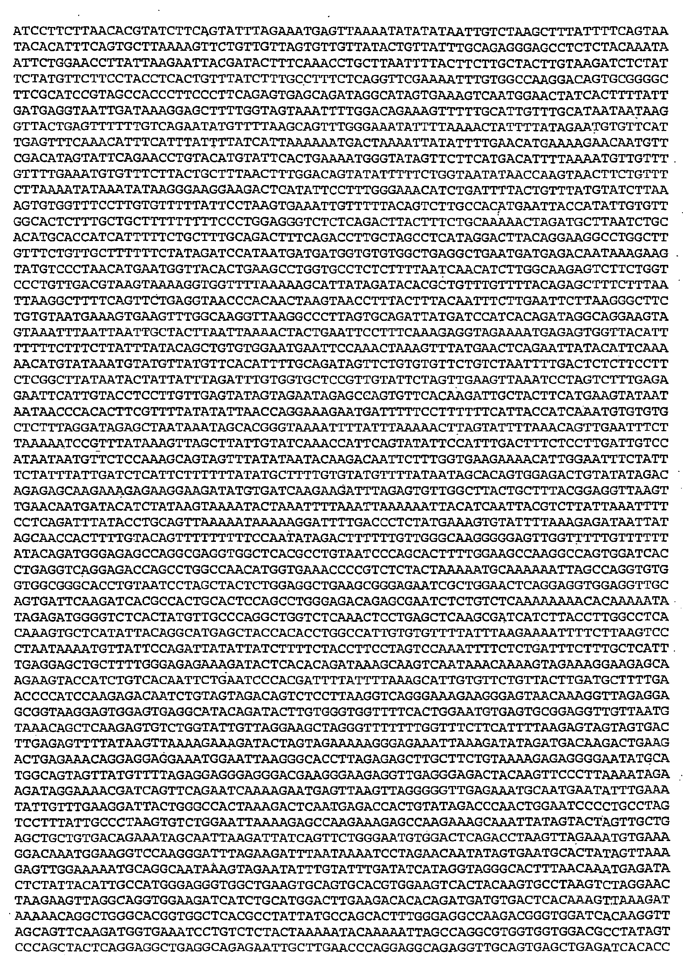

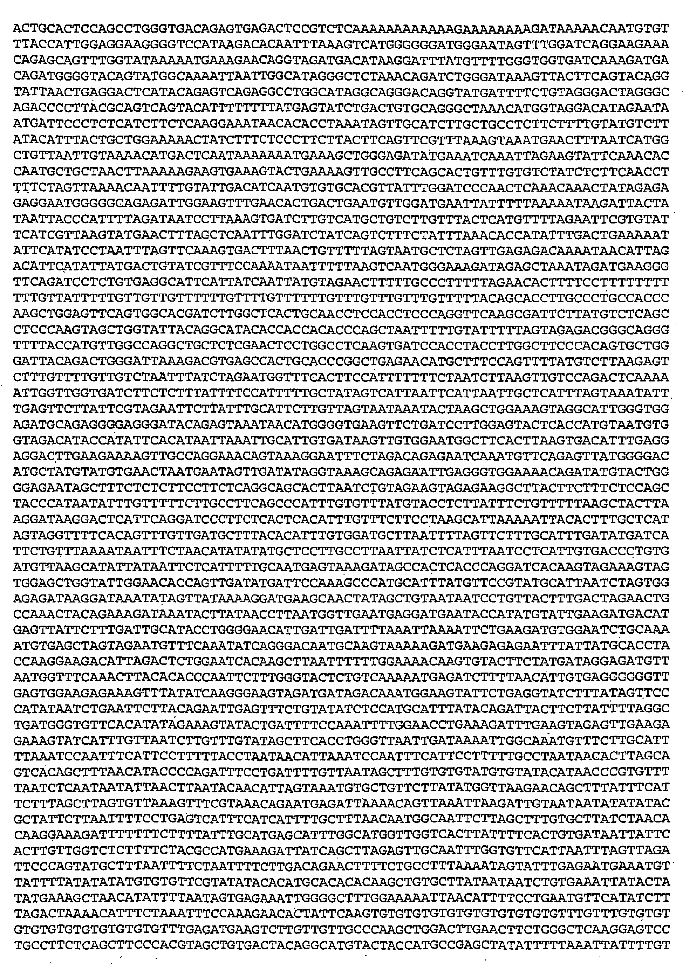

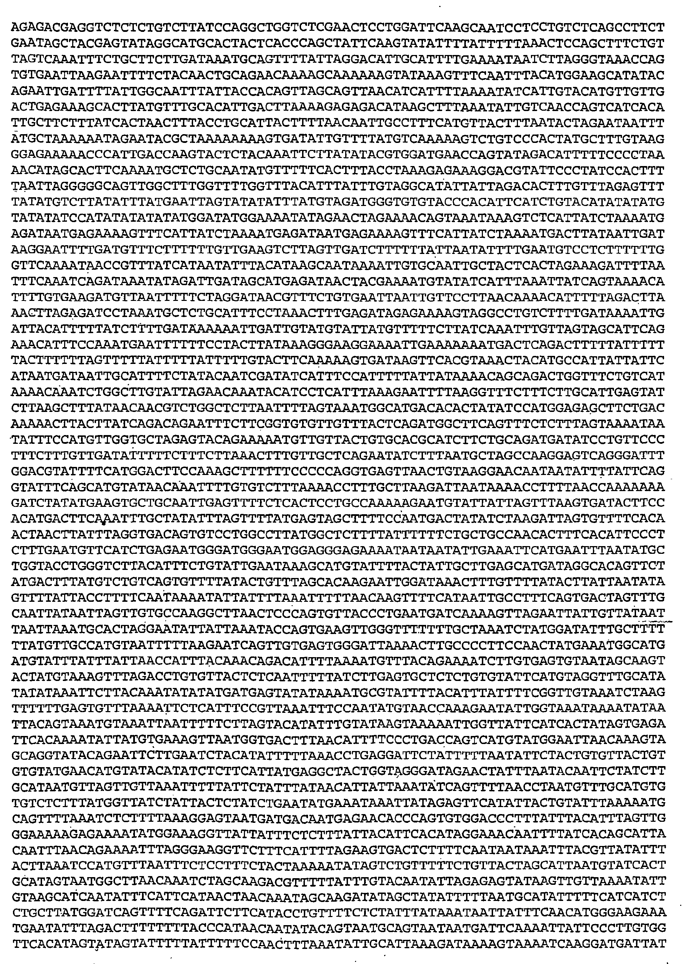

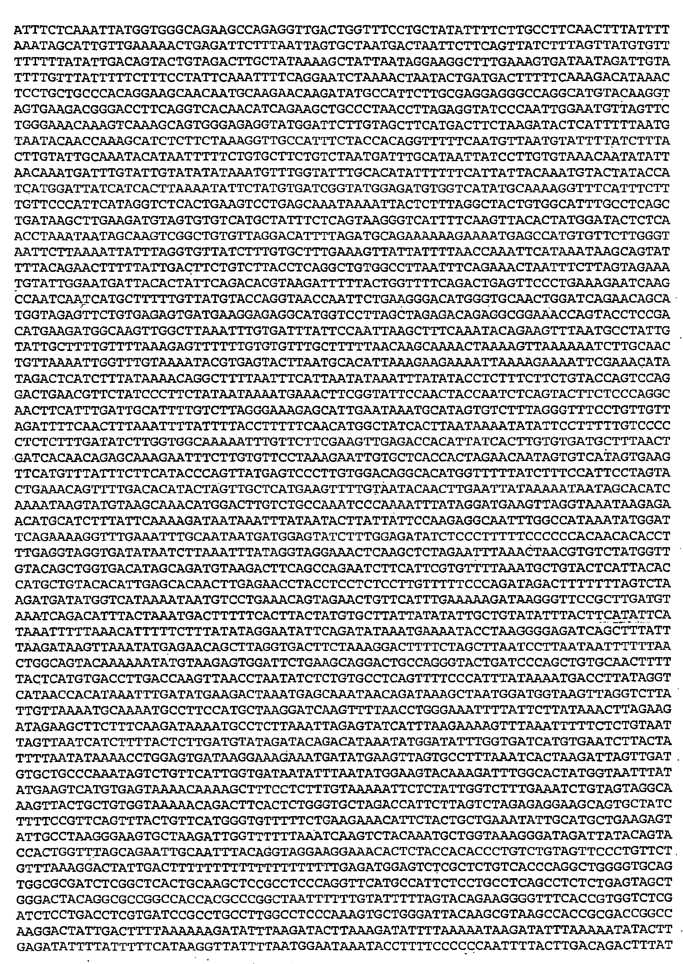

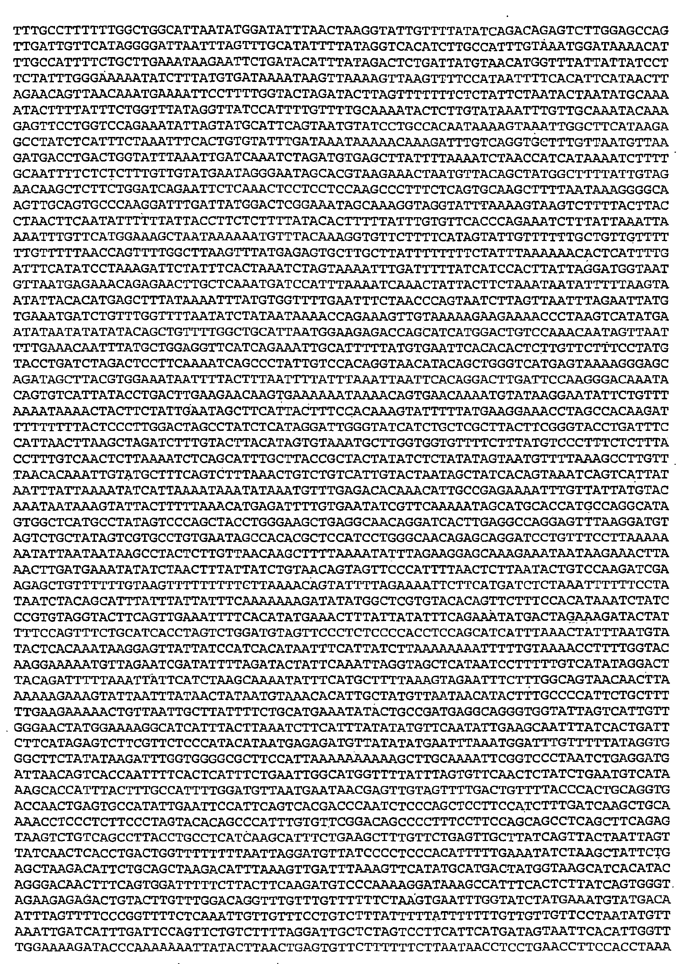

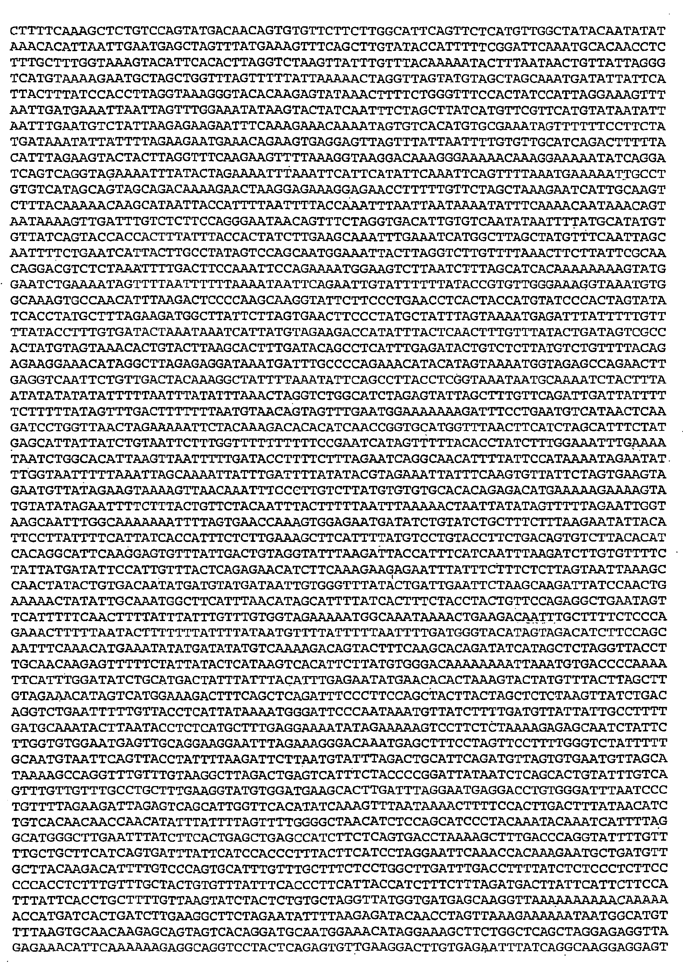

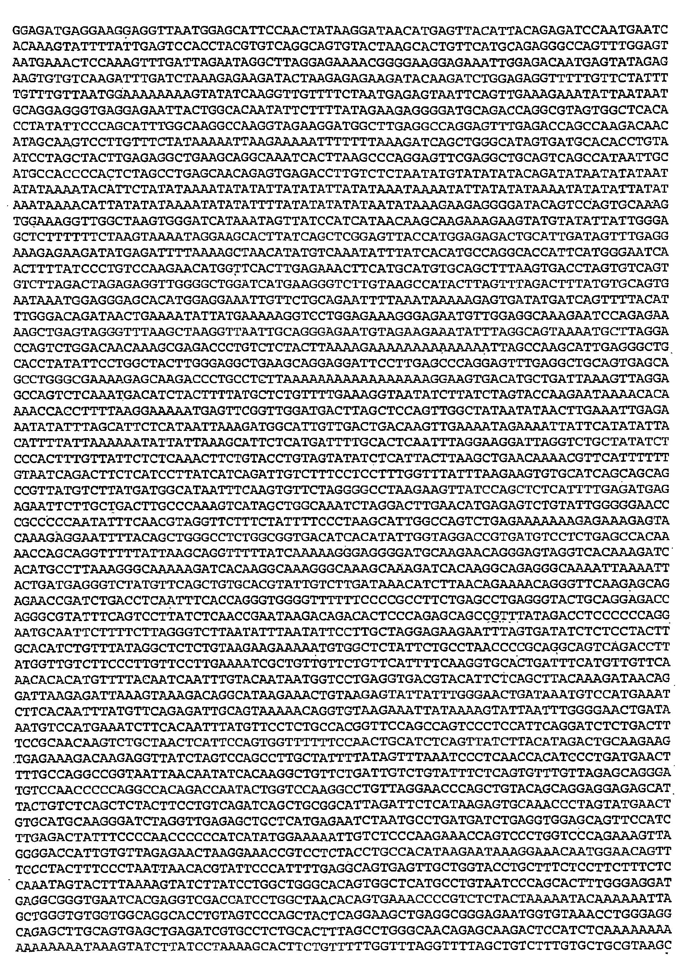

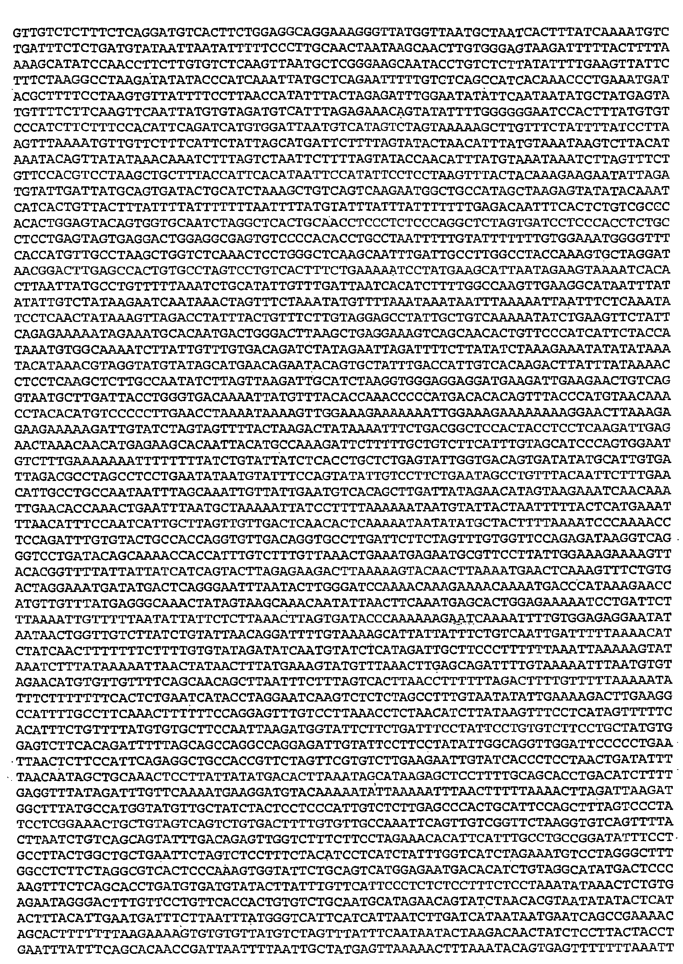

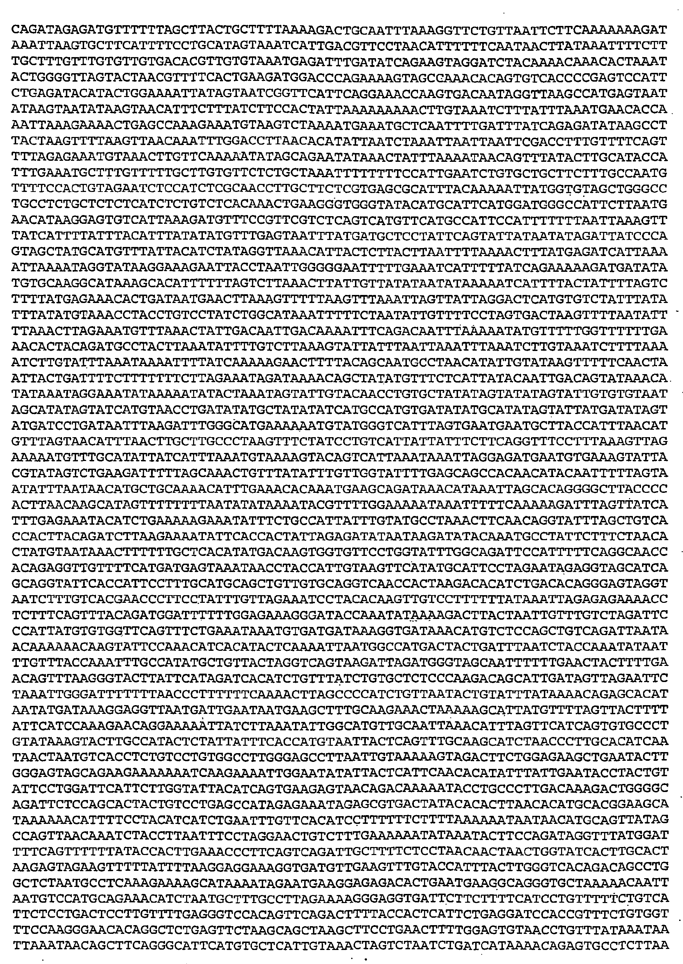

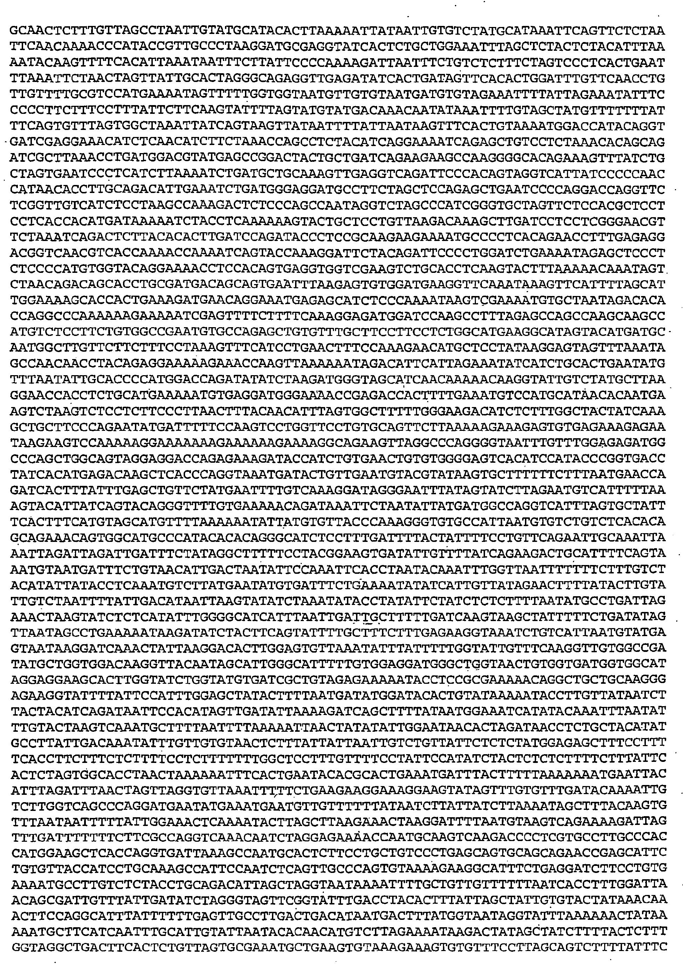

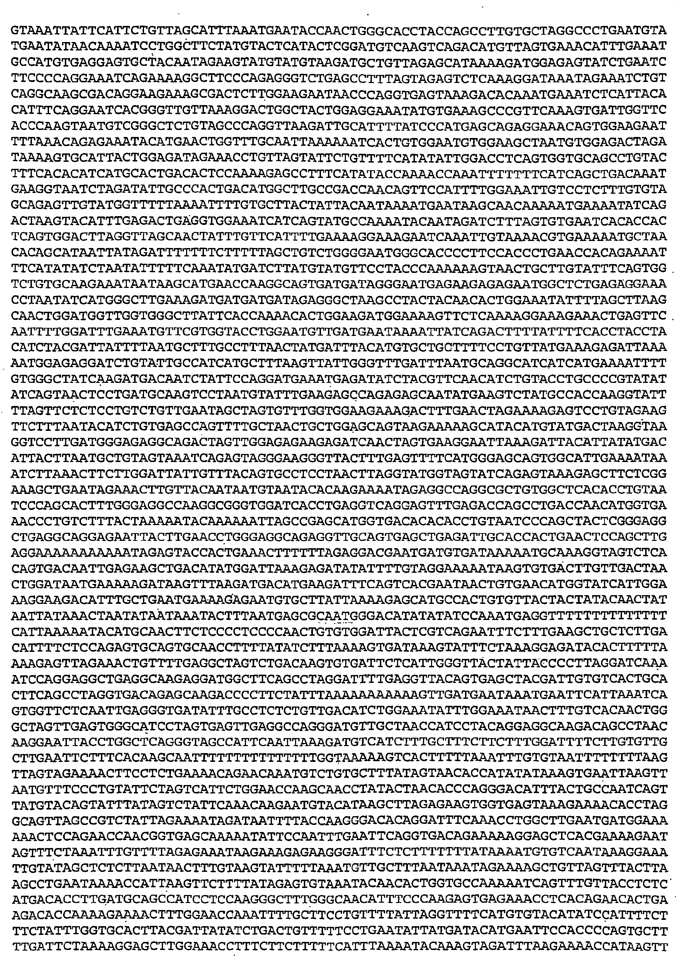

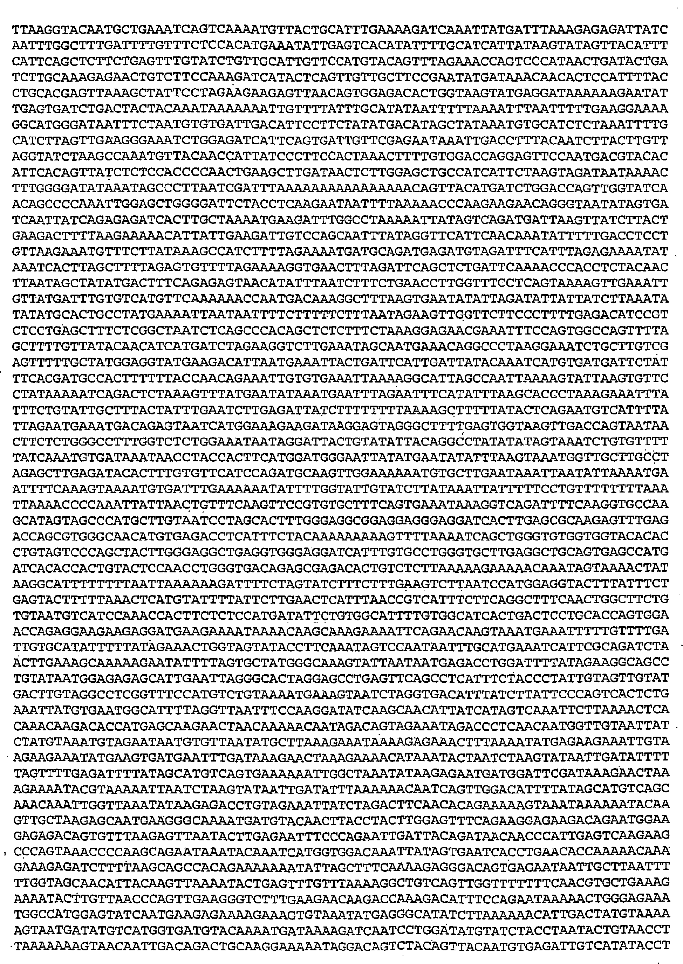

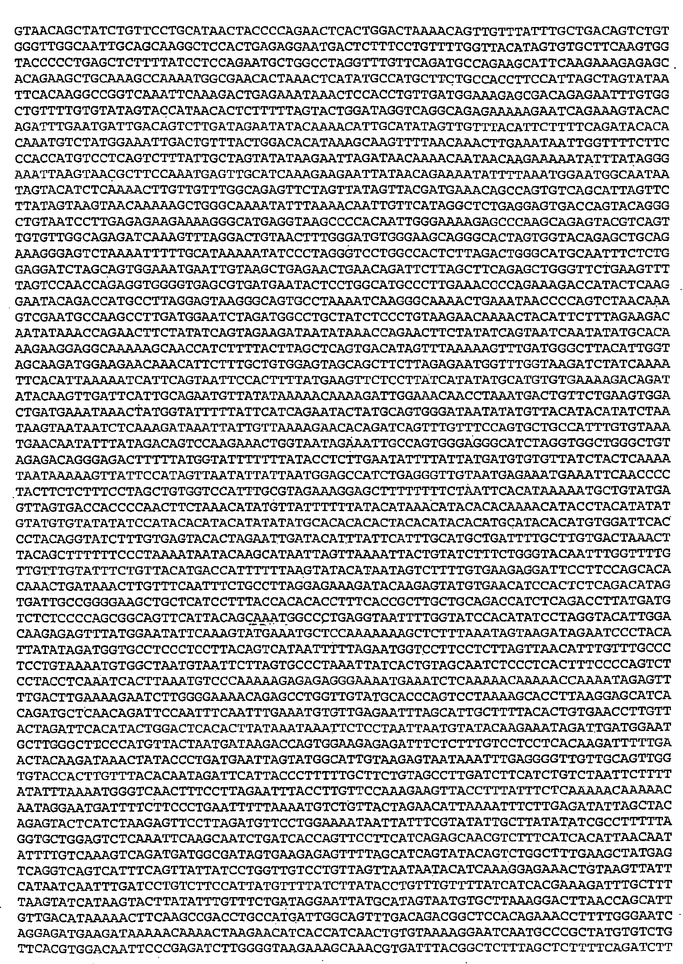

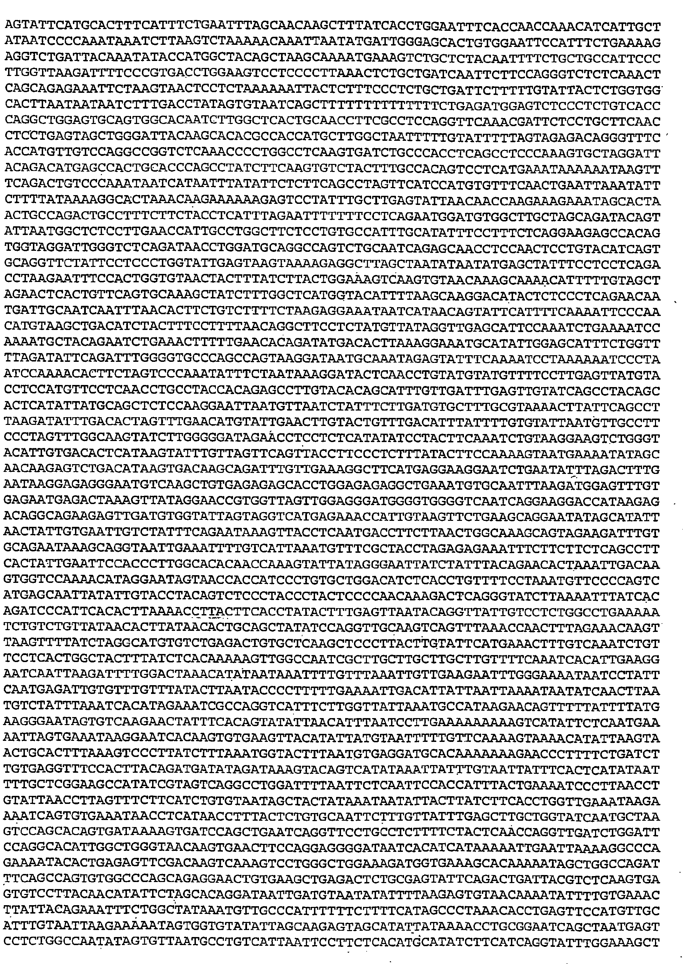

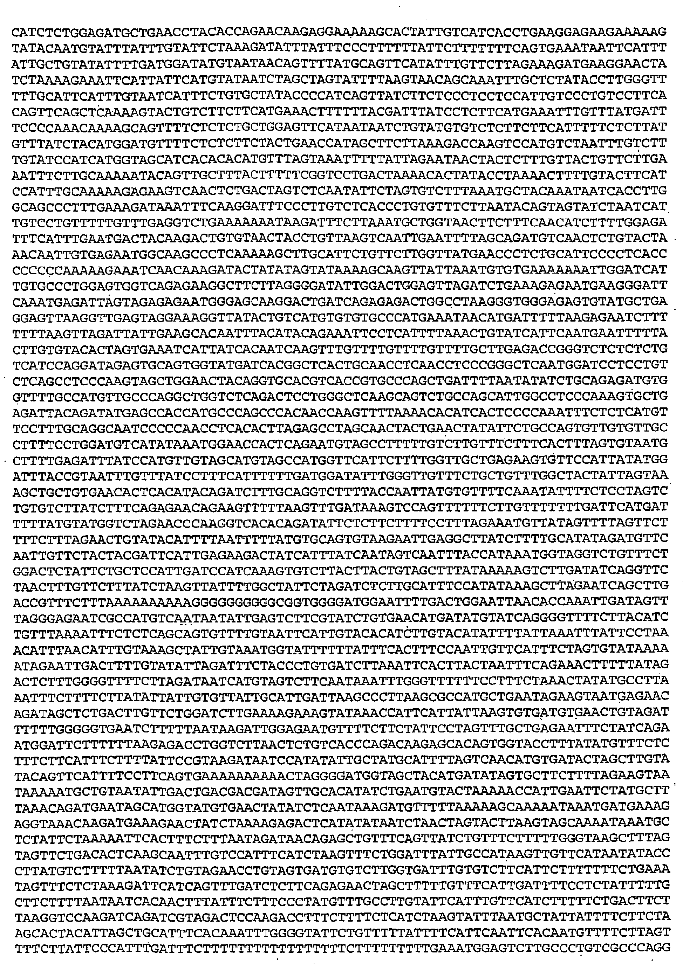

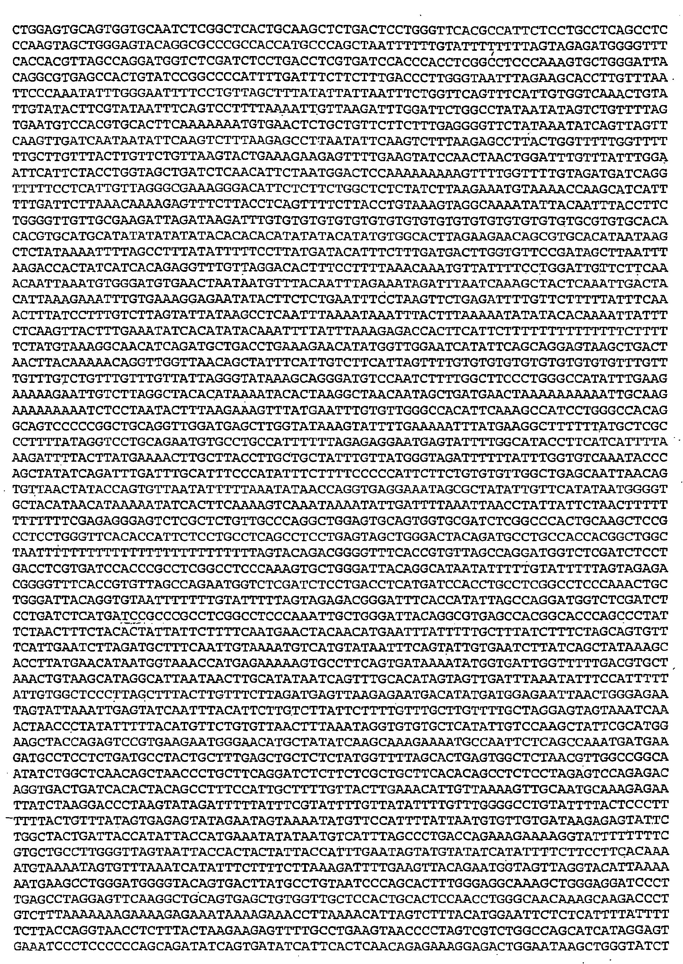

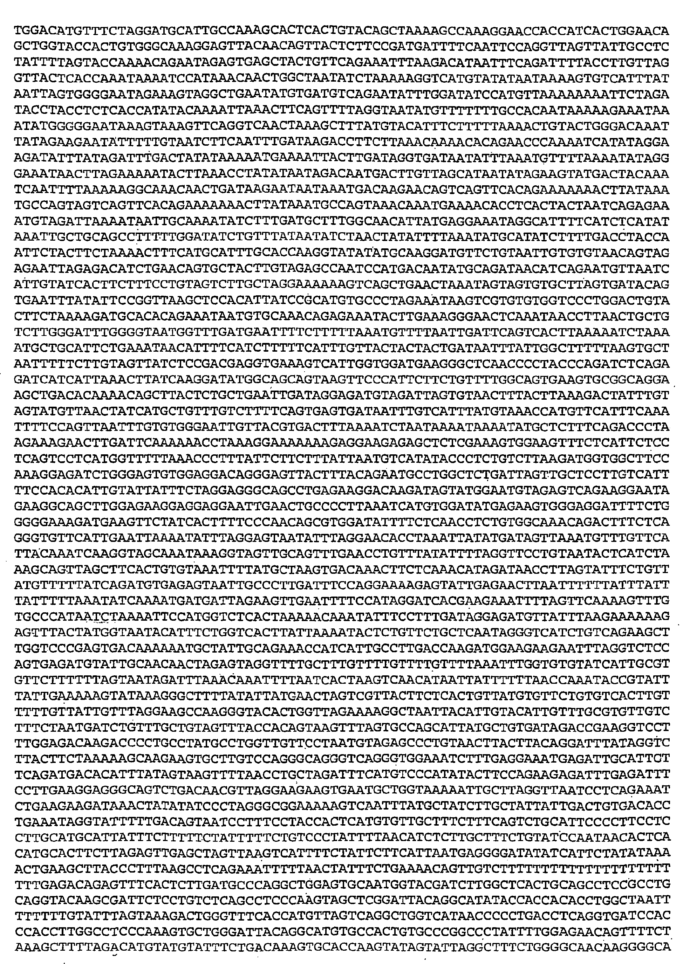

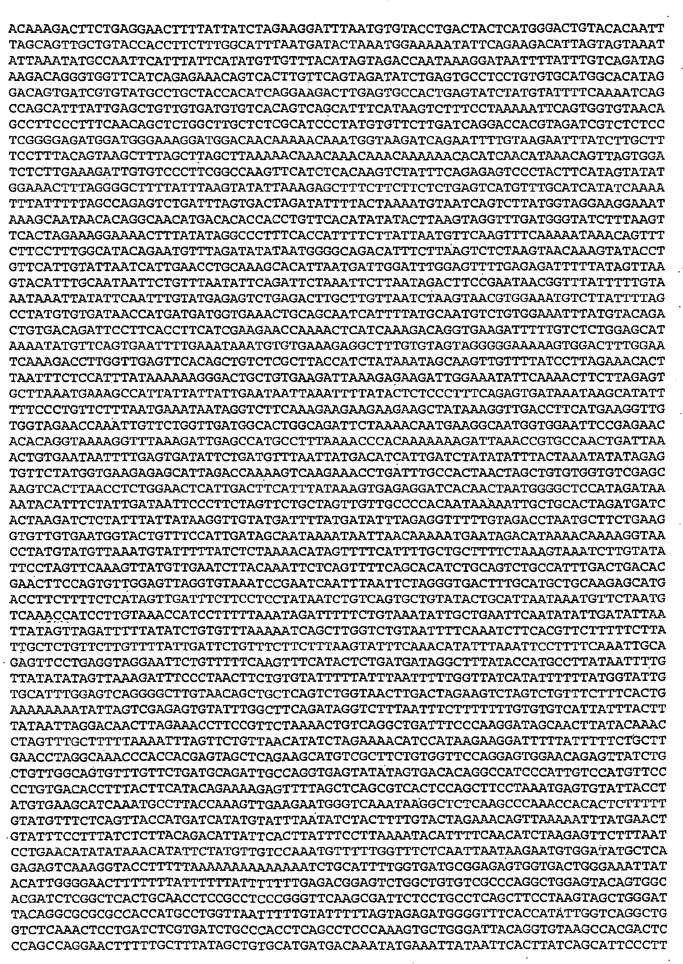

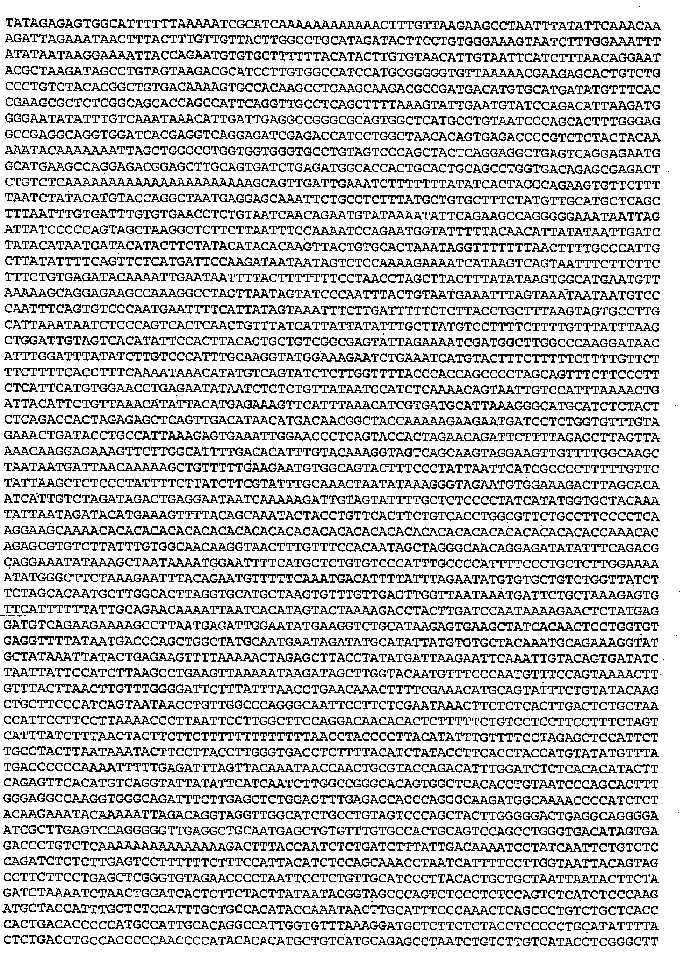

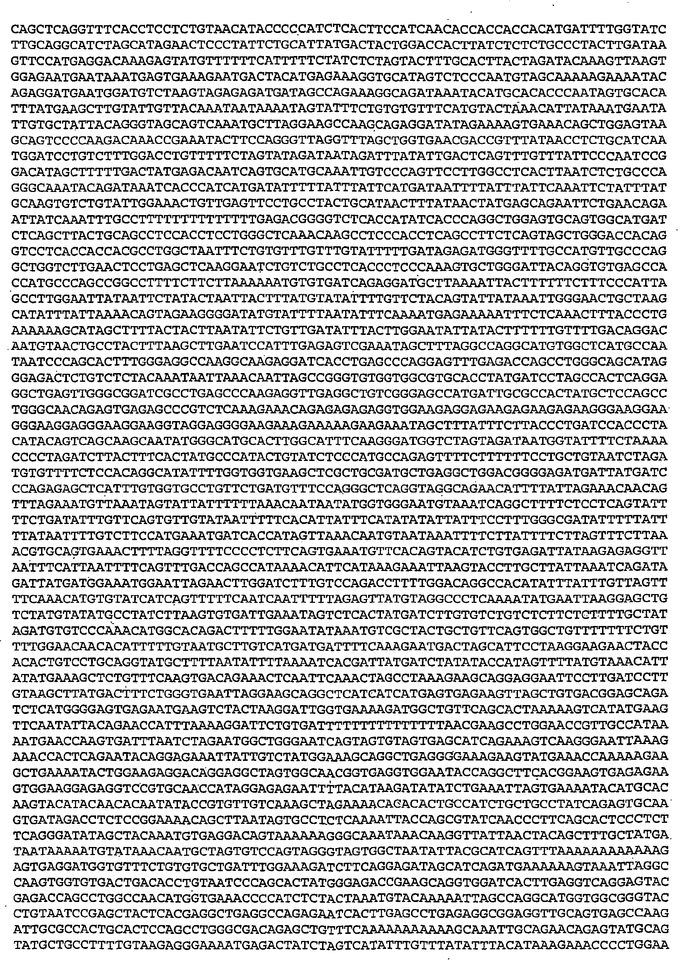

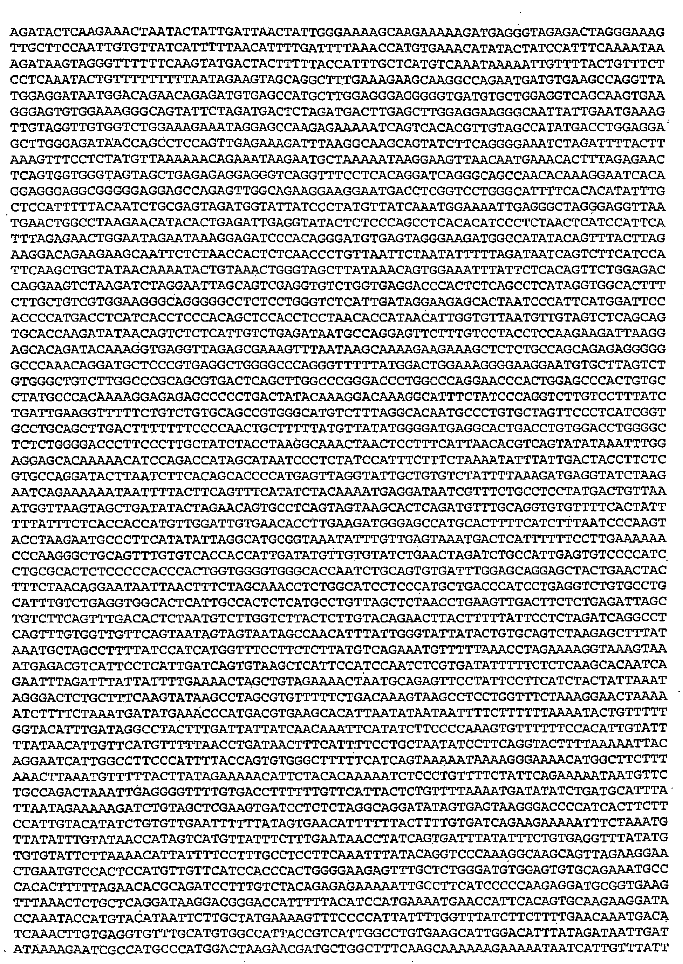

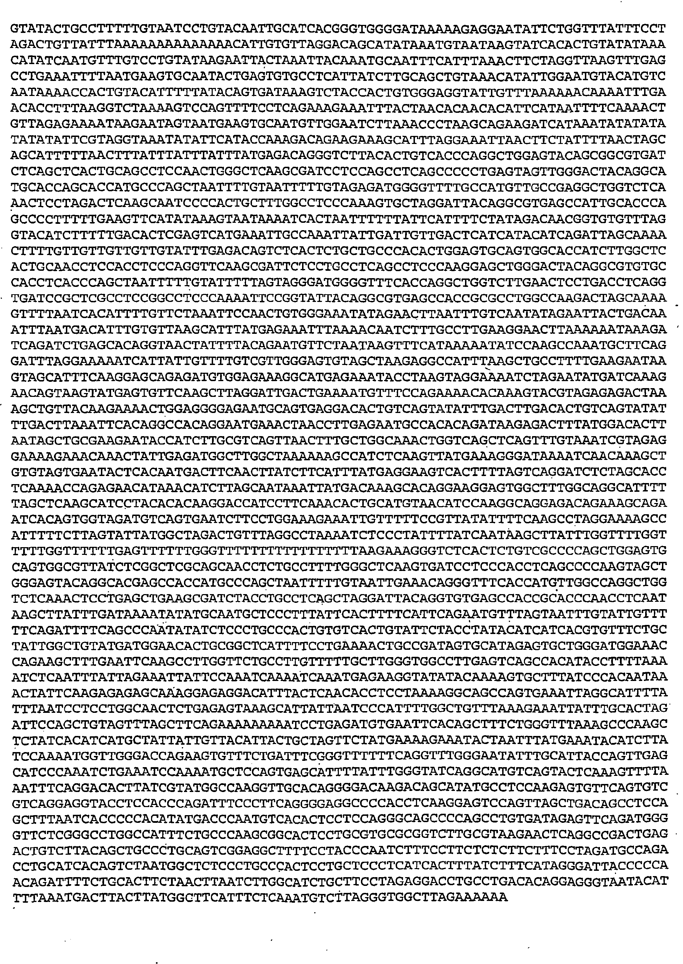

Figure 7: Protein, genomic and coding nucleotide sequence of human PAM according to NCBI accession numbers AAC39928 (protein sequence; SEQ ID No.1), AF075587 (coding sequence; SEQ ID No.2). Human PAM is located on Chromosome 13q22; its genomic sequence is publicly available under NT_024524.11 (Start: position 24679861; Stop: position 24962245; SEQ ID No 3); Figure 7C shows the contiguous sequence from position 24679861 to position 24962245.

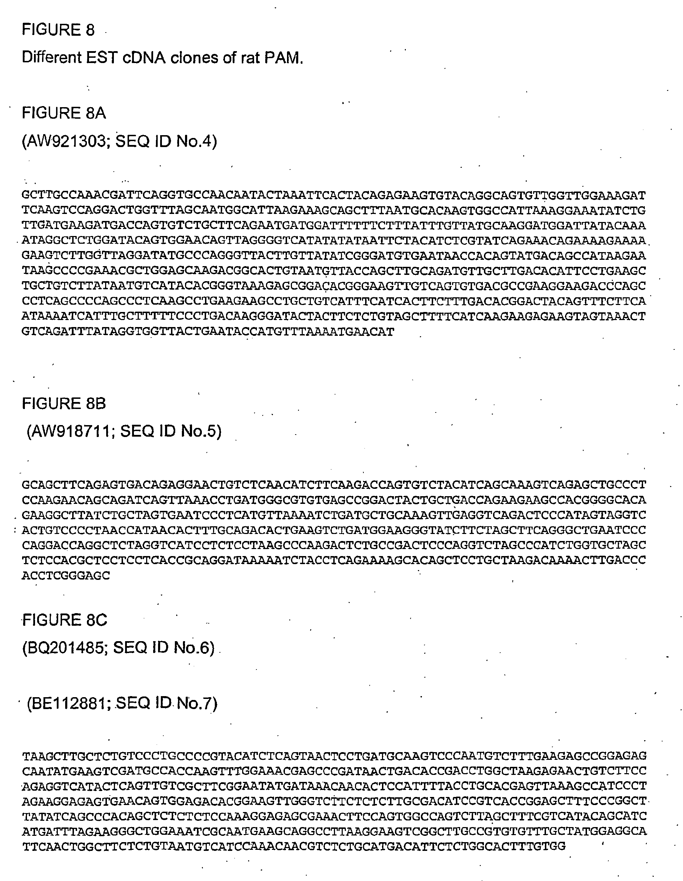

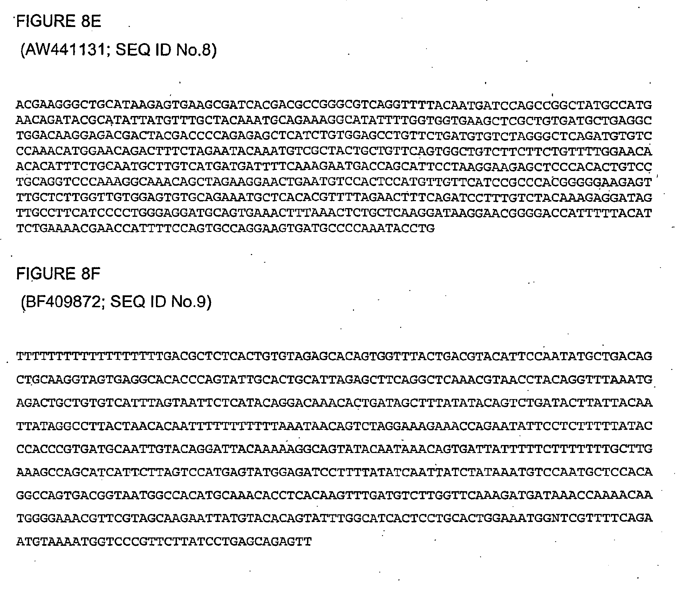

Figure 8: EST-clone coding sequences for rat PAM:
Figure 8A: AW921303 (corresponds to bp 960-1394 of hs cDNA; SEQ ID No. 4)
Figure 8B: AW918711 (corresponds to bp 8188-8632 of hs cDNA; SEQ ID No. 5)
Figure 8C: BQ201485 (corresponds to bp 8966-9633 of hs cDNA; SEQ ID No. 6)
Figure 8D: BE112881 (corresponds to bp 10311-10830 of hs cDNA; SEQ Id No. 7)
Figure 8E: AW441131 (corresponds to bp 13569-14152 of hs cDNA; SEQ ID No. 8)
Figure 8F: BF409872 (corresponds to bp 13569-14807 of hs cDNA). (SEQ ID No. 9)

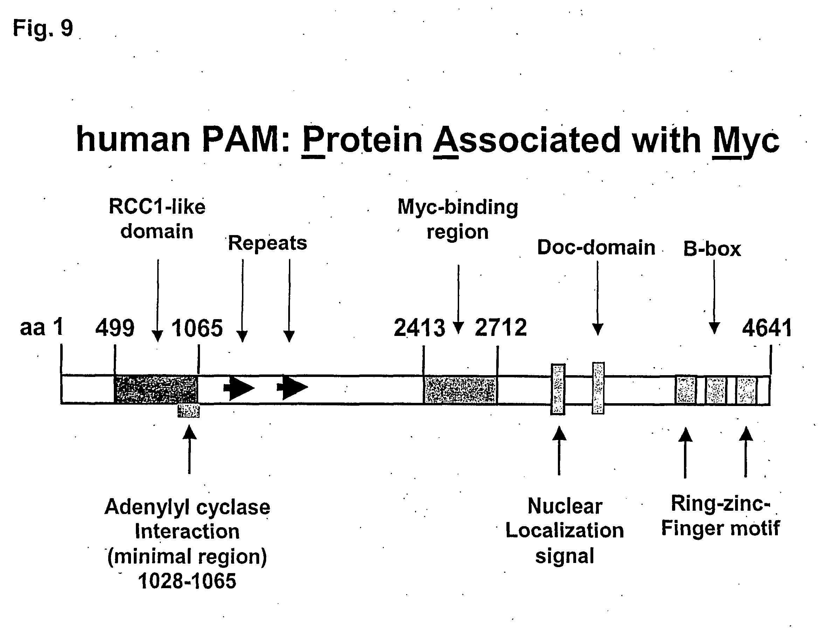

Figure 9: Overview of domain structure of human PAM according to Guo et al., 1988 / Grossberger et al., JBC 1999 and Scholich et al., JBC 2001)

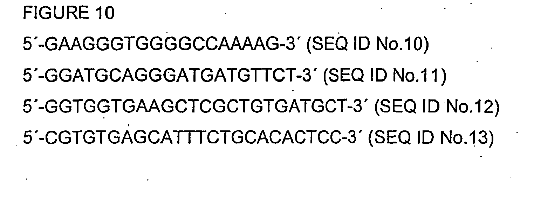

Figure 10: PCR Primers for rat PAM RT PCR.

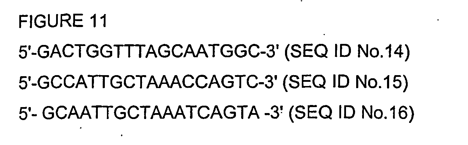

Figure 11: Antisense Oligodesoxynucleotides for inhibiting rat PAM expression and control oligonucleotide.

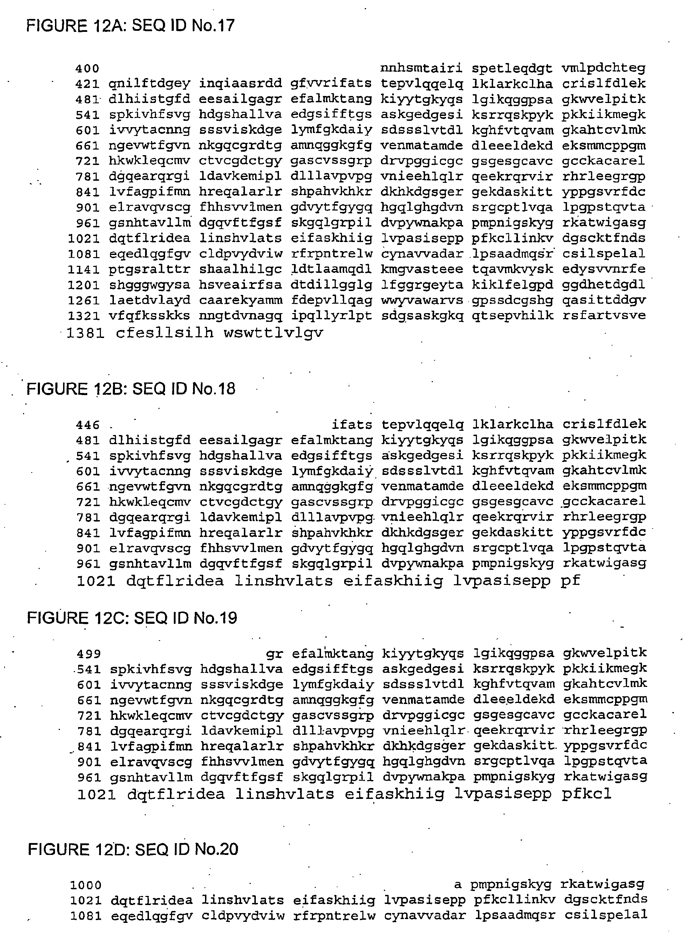

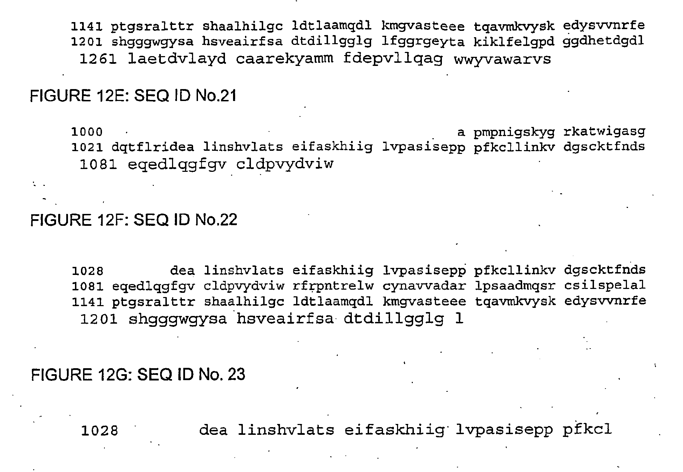

Figure 12: Different PAM hs polypeptides for the use in the context of present invention. The polypeptides are fragments derived from the polypeptide according to SEQ ID No.2.

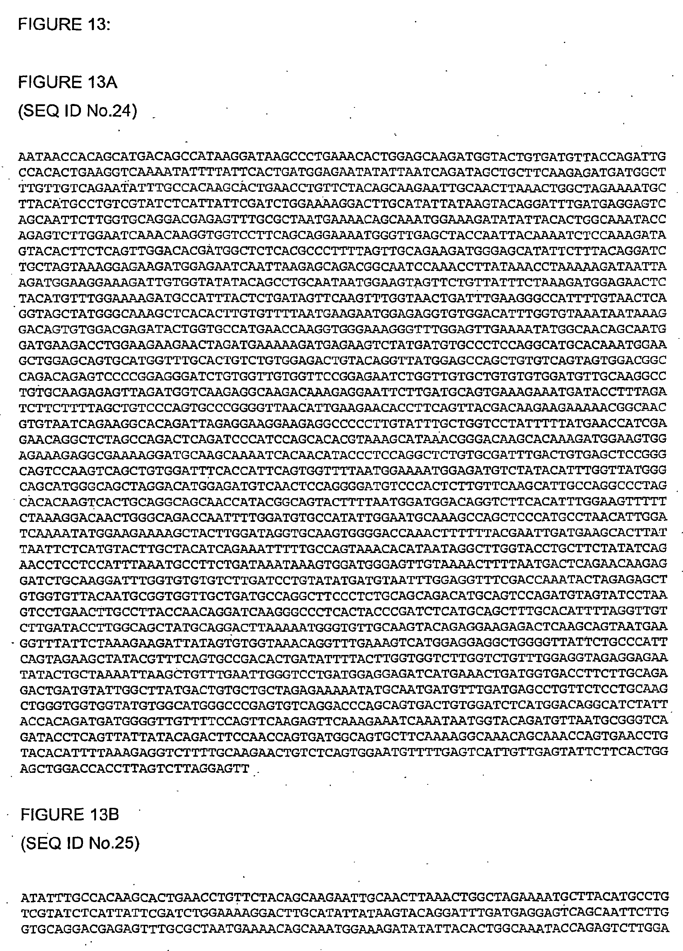

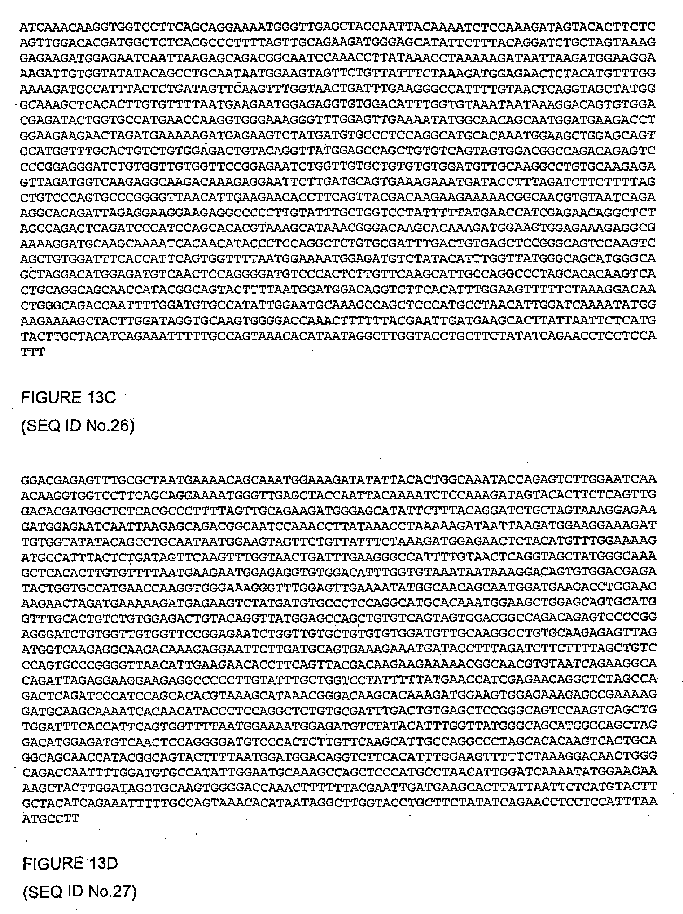

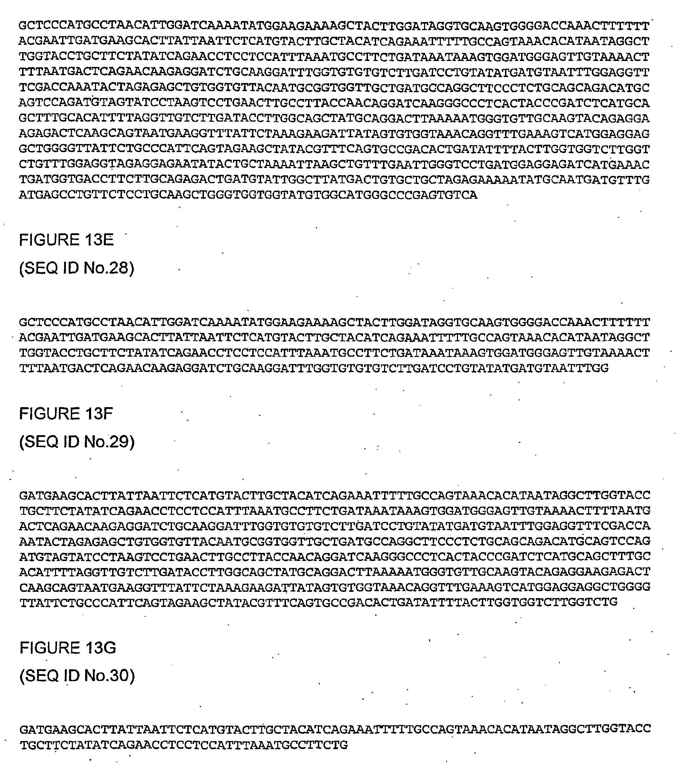

Figure 13: Different PAM hs polynucleotides for the use in the context of present invention.
Figure 13A: cDNA sequence coding for the protein fragment according to SEQ ID No.17 comprising nucleotide positions 1317 to 4366 of hs Pam cDNA (SEQ ID No. 24);
Figure 13B: cDNA sequence coding for the protein fragment according to SEQ ID No.18 comprising nucleotide positions 1482 to 3332 of hs Pam cDNA (SEQ ID No. 25);
Figure 13C: cDNA sequence coding for the protein fragment according to SEQ ID No.19 comprising nucleotide positions 1641 to 3341 of hs Pam cDNA (SEQ ID No. 26);
Figure 13D: cDNA sequence coding for the protein fragment according to SEQ ID No.20 comprising nucleotide positions 3142 to 4046 of hs Pam cDNA (SEQ ID No. 27);
Figure 13E: cDNA sequence coding for the protein fragment according to SEQ ID No.21 comprising nucleotide positions 3142 to 3446 of hs Pam cDNA (SEQ ID No. 28);
Figure 13F: cDNA sequence coding for the protein fragment according to SEQ ID No.22 comprising nucleotide positions 3228 to 3839 of hs Pam cDNA (SEQ ID No. 29);
Figure 13G: cDNA sequence coding for the protein fragment according to SEQ ID No.23 comprising nucleotide positions 3228 to 3341 of hs Pam cDNA (SEQ ID No. 30);

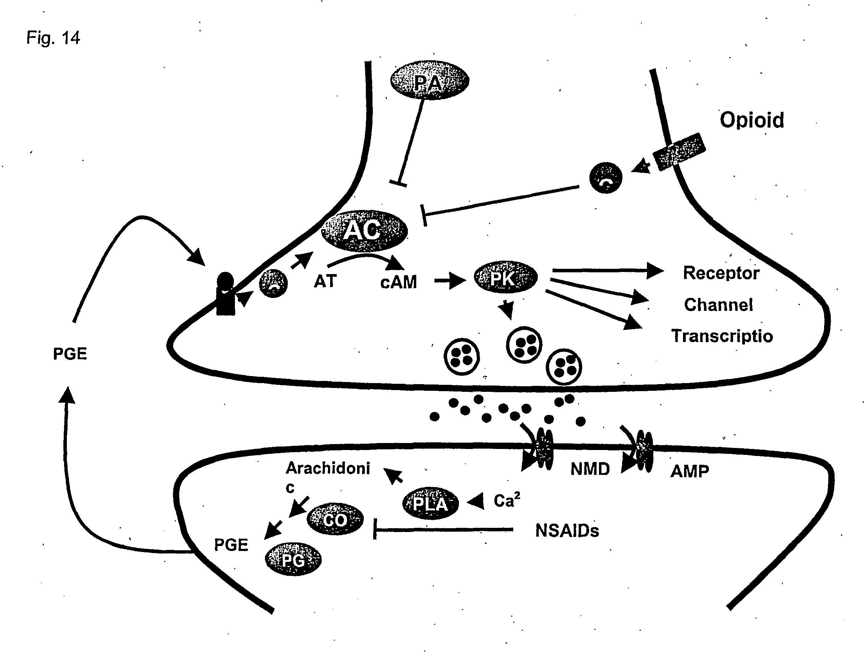

Figure 14: Overview of the postulated PAM signaling pathway in neurons of the spiral cord and the DRGs according to the above findings.

### Table 1

Basal AC activity is elevated in spinal cord lysates of antisense treated rats. Spinal cords lysates (20 µg) were assayed for AC activity in the absence or presence of 80 nM Gαs or 100 µM forskolin as described above. The mean AC activity ± SEM of spinal cord lysates from at least three rats per group, each measured twice in triplicates, is shown (ns = not significant).

**Table**

| Condition | AC activity | | |
|---|---|---|---|
| | (pmol/min/mg | | |
| basal | | | |
| sense | | 102.7+6.6 | |
| antisense | 1 | 24.0 ± 6.2 | (p≤0.01) |

| Gαs | | | |
|---|---|---|---|
| sense | | 398.8 ± 16.9 | |
| antisense | | 432.6 ± 17.2 | (ns) |

| Forskolin | | | |
|---|---|---|---|
| sense | | 283.9 ± 25.7 | |
| antisense | | 316.2 ± 24.3 | (ns) |

## Claims

1. The use of PAM or functional fragments thereof for the preparation of pharmaceutical compounds.

2. The use of PAM or functional fragments thereof for identifying compounds that enhance PAM activity, thus lessening or abolishing pain.

3. The use according to claim 1, wherein the pharmaceutical compounds are compounds for the prevention or treatment of pain.

4. A method of screening pharmaceuticals useful for treating and/or preventing pain, comprising the steps
a. Providing two samples
b. contacting one sample containing PAM or a functional fragment thereof with a compound,
c. determining the PAM activity in the presence of compound,
d. determining the PAM activity in the absence of compound, and
e. comparing the PAM activity according to c) with that according to d),
wherein an enhanced PAM activity according to c) identifies a compound as having the ability to treat and/or prevent pain.

5. The use according to one of the claims 1, 2 or 3 or the method according to claim 4, wherein PAM is human PAM.

6. The use according to one of the claims 1, 2 or 3 or the method according to claim 4, wherein PAM is an isolated polypeptide or polynucleotide.

7. The use or method according to claim 6, wherein PAM is a polypeptide.

8. The use or method according to claim 6 or 7, wherein PAM is a polypeptide or functional fragment thereof that comprises or consists of the sequence according to SEQ ID No 2 or a fragment thereof or is encoded by a polynucleotide comprising or consisting of the sequence according to SEQ ID No 1 or a fragment thereof.

9. The use or method according to claim 6, wherein PAM is a polynucleotide.

10. The use or method according to claim 6, wherein PAM is a polynucleotide comprising or consisting of the sequence or a part of the sequence coding for a functional fragment of PAM according to SEQ ID No 1 or a polynucleotide comprising or consisting of a sequence that is able to hybridize with said polynucleotide under stringent conditions.

11. The use or method according to one of the above claims, wherein the functional fragments thereof are capable of inhibiting adenylyl cyclase activity.

12. The use or method according to claim 11, wherein the adenylyl cyclase is adenylyl cyclase type I, V or VI.

13. The use or method according to one of the above claims, wherein the functional fragments comprise or consist of amino acids 400 to 1400, preferably 446 to 1062, 499 to 1065 or 1028 to 1231, more preferably 1000 to 1300 and even more preferably 1000 to 1100 and even more preferably 1028 to 1065 of human PAM, preferably of human PAM according to SEQ ID No. 2.

14. The use or method according to claim 11, wherein the fragments comprise or consist of one of the polypeptides according to SEQ ID No. 17 to 23.

15. The method according to claim 4, wherein a cell expressing, preferably expressing recombinant PAM is used.

16. The method according to claim 4, wherein a modified cell, having a lower PAM activity as compared to its unmodified state, is used.

17. The method according to claim 16, wherein a PAM knock-out cell is used.

18. The method according to one of the claims 4, 15 or 16, wherein the PAM activity is determined directly.

19. The method according to claim 18, wherein the determination of PAM activity concerns its ability to interact with and preferably inactivate adenylate cyclase.

20. The method according to one of the claims 4, 15 or 16, wherein the PAM activity is determined indirectly.

## Patentansprüche

1. Verwendung von PAM oder funktionellen Fragmenten davon zur Herstellung pharmazeutischer Verbindungen.

2. Verwendung von PAM oder funktionellen Fragmenten davon zur Identifizierung von Verbindungen, die die PAM-Aktivität verstärken, womit Schmerzen gelindert oder beseitigt werden.

3. Verwendung nach Anspruch 1, wobei es sich bei den pharmazeutischen Verbindungen um Verbindungen zur Schmerzvorbeugung oder -behandlung handelt.

4. Verfahren zum Screening von zur Schmerzbehandlung und/oder -vorbeugung geeigneten Pharmazeutika, das die folgenden Schritte umfasst:
a. Bereitstellen zweier Proben,
b. Inkontaktbringen einer PAM oder ein funktionelles Fragment davon enthaltenden Probe mit einer Verbindung,
c. Bestimmen der PAM-Aktivität in Gegenwart von Verbindung,
d. Bestimmen der PAM-Aktivität in Abwesenheit von Verbindung und
e. Vergleichen der PAM-Aktivität gemäß c) mit derjenigen gemäß d),
wobei eine verstärkte PAM-Aktivität gemäß c) eine Verbindung identifiziert, die die Fähigkeit zur Schmerzbehandlung und/oder -vorbeugung besitzt.

5. Verwendung nach einem der Ansprüche 1, 2 oder 3 oder Verfahren nach Anspruch 4, wobei es sich bei PAM um menschliches PAM handelt.

6. Verwendung nach einem der Ansprüche 1, 2 oder 3 oder Verfahren nach Anspruch 4, wobei es sich bei PAM um ein isoliertes Polypeptid oder Polynukleotid handelt.

7. Verwendung oder Verfahren nach Anspruch 6, wobei es sich bei PAM um ein Polypeptid handelt.

8. Verwendung oder Verfahren nach Anspruch 6 oder 7, wobei es sich bei PAM um ein Polypeptid oder ein funktionelles Fragment davon handelt, das die Sequenz gemäß SEQ ID Nr. 2 oder ein Fragment davon umfasst bzw. daraus besteht oder durch ein Polynukleotid codiert wird, das die Sequenz gemäß SEQ ID Nr. 1 oder ein Fragment davon umfasst bzw. daraus besteht.

9. Verwendung oder Verfahren nach Anspruch 6, wobei es sich bei PAM um ein Polynukleotid handelt.

10. Verwendung oder Verfahren nach Anspruch 6, wobei es sich bei PAM um ein Polynukleotid, das die für ein funktionelles Fragment von PAM codierende Sequenz gemäß SEQ ID Nr. 1 oder einen Teil dieser Sequenz umfasst bzw. daraus besteht, oder ein Polynukleotid, das eine Sequenz, die mit dem Polynukleotid unter stringenten Bedingungen hybridisieren kann, umfasst bzw. daraus besteht, handelt.

11. Verwendung oder Verfahren nach einem der obigen Ansprüche, wobei die funktionellen Fragmente davon in der Lage sind, Adenylylcyclase-Aktivität zu hemmen.

12. Verwendung oder Verfahren nach Anspruch 11, wobei es sich bei der Adenylylcyclase um Adenylylcyclase Typ I, V oder VI handelt.

13. Verwendung oder Verfahren nach einem der obigen Ansprüche, wobei die funktionellen Fragmente Aminosäuren 400 bis 1400, vorzugsweise 446 bis 1062, 499 bis 1065 oder 1028 bis 1231, stärker bevorzugt 1000 bis 1300 und noch stärker bevorzugt 1000 bis 1100 und noch stärker bevorzugt 1028 bis 1065, von menschlichem PAM, vorzugsweise von menschlichem PAM gemäß SEQ ID Nr. 2, umfassen bzw. daraus bestehen.

14. Verwendung oder Verfahren nach Anspruch 11, wobei die Fragmente eines der Polypeptide gemäß SEQ ID Nr. 17 bis 23 umfassen bzw. daraus bestehen.

15. Verfahren nach Anspruch 4, wobei eine exprimierende, vorzugsweise eine rekombinantes PAM exprimierende Zelle verwendet wird.

16. Verfahren nach Anspruch 4, wobei eine modifizierte Zelle, die eine geringere PAM-Aktivität verglichen mit ihrem nichtmodifizierten Zustand aufweist, verwendet wird.

17. Verfahren nach Anspruch 16, wobei eine PAM-Knockout-Zelle verwendet wird.

18. Verfahren nach einem der Ansprüche 4, 15 oder 16, wobei die PAM-Aktivität direkt bestimmt wird.

19. Verfahren nach Anspruch 18, wobei die Bestimmung der PAM-Aktivität dessen Fähigkeit zur Wechselwirkung mit und vorzugsweise Inaktivierung von Adenylylcyclase betrifft.

20. Verfahren nach einem der Ansprüche 4, 15 oder 16, wobei die PAM-Aktivität indirekt bestimmt wird.

## Revendications

1. Utilisation de PAM ou de fragments fonctionnels de celui-ci pour la préparation de composés pharmaceutiques.

2. Utilisation de PAM ou de fragments fonctionnels de celui-ci pour identifier des composés qui augmentent l'activité PAM, de manière à atténuer ou éliminer une douleur.

3. Utilisation selon la revendication 1, **caractérisée en ce que** les composés pharmaceutiques sont des composés pour la prévention ou le traitement de la douleur.

4. Procédé de criblage d'agents pharmaceutiques utiles pour traiter et/ou prévenir la douleur, comprenant les étapes consistant à
a. produire deux échantillons
b. mettre en contact un échantillon contenant PAM ou un fragment fonctionnel de celui-ci avec un composé,
c. déterminer l'activité PAM en présence de composé,
d. déterminer l'activité PAM en l'absence de composé, et
c. comparer l'activité PAM selon c) à celle selon d),
où une activité PAM augmentée selon c) identifie un composé ayant la capacité à traiter et/ou prévenir une douleur.

5. Utilisation selon l'une des revendications 1, 2 ou 3 ou procédé selon la revendication 4, où PAM est PAM humain.

6. Utilisation selon l'une des revendications 1, 2 ou 3 ou procédé selon la revendication 4, où PAM est un polypeptide ou polynucléotide isolé.

7. Utilisation ou procédé selon la revendication 6, **caractérisée en ce que** PAM est un polypeptide.

8. Utilisation ou procédé selon la revendication 6 ou 7, **caractérisés en ce que** PAM est un polypeptide ou fragment fonctionnel de celui-ci qui comprend ou est constitué de la séquence SEQ ID N° 2 ou un fragment de celle-ci ou est codé par un polynucléotide comprenant ou constitué de la séquence selon SEQ ID N° 1 ou un fragment de celle-ci.

9. Utilisation ou procédé selon la revendication 6, **caractérisés en ce que** PAM est un polynucléotide.

10. Utilisation ou procédé selon la revendication 6, **caractérisés en ce que** PAM est un polynucléotide comprenant ou constitué de la séquence ou une partie de la séquence codant pour un fragment fonctionnel de PAM selon SEQ ID N° 1 ou un polynucléotide comprenant ou constitué d'une séquence qui est capable de s'hybrider avec ledit polynucléotide dans des conditions stringentes.

11. Utilisation ou procédé selon l'une des revendications ci-dessus, **caractérisés en ce que** les fragments fonctionnels de ceux-ci sont capables d'inhiber l'activité adénylyle cyclase.

12. Utilisation ou procédé selon la revendication 11, **caractérisés en ce que** l'adénylyle cyclase est l'adénylyle cyclase de type I, V ou VI.

13. Utilisation ou procédé selon l'une des revendications ci-dessus, **caractérisés en ce que** les fragments fonctionnels comprennent ou sont constitués des acides aminés 400 à 1400, de préférence 446 à 1062 ; 499 à 1065 ou 1028 à 1231, plus préférablement 1000 à 1300 et encore plus préférablement 1000 à 1100 et encore plus préférablement 1028 à 1065 de PAM humain, de préférence de PAM humain selon SEQ ID N° 2.

14. Utilisation ou procédé selon la revendication 11, **caractérisés en ce que** les fragments comprennent ou sont constitués d'un des polypeptides selon SEQ ID N° 17 à 23.

15. Procédé selon la revendication 4, **caractérisé en ce qu'**une cellule exprimant un PAM, de préférence exprimant un PAM recombinant est utilisée.

16. Procédé selon la revendication 4, **caractérisé en ce qu'**une cellule modifiée, ayant une activité PAM inférieure par rapport à son état non modifié, est utilisée.

17. Procédé selon la revendication 16, **caractérisé en ce qu'**une cellule PAM knock-out est utilisée.

18. Procédé selon l'une des revendications 4, 15 ou 16, **caractérisé en ce que** l'activité PAM est déterminée directement.

19. Procédé selon la revendication 18, **caractérisé en ce que** la détermination d'activité PAM concerne sa capacité à interagir avec et de préférence inactiver l'adénylate cyclase.

20. Procédé selon l'une des revendications 4, 15 ou 16, **caractérisé en ce que** l'activité PAM est déterminée indirectement.
